# EUROPEAN PATENT APPLICATION

(11) **EP 4 339 204 A1**
(43) Date of publication of application: **20.03.2024**
(21) Application number: 22798955.5
(22) Date of filing: 06.05.2022
(51) Int. Cl.: C07K 7/54, C07K 2/00

(54) **BINDING MOLECULE EXHIBITING SELECTIVE BINDING FOR RAS(G12D) MUTANT**

(30) Priority: 07.05.2021 WO PCT/JP2021/017466
(71) Applicant: CHUGAI SEIYAKU KABUSHIKI KAISHA, Tokyo 115-8543 (JP)
(72) Inventor: HAYASHI, Ryuji, Kamakura-shi, Kanagawa 247-8530 (JP); HASHIMOTO, Satoshi, Gotemba-shi, Shizuoka 412-8513 (JP); MINAMI, Saki, Gotemba-shi, Shizuoka 412-8513 (JP)
(74) Representative: Vossius & Partner Patentanwälte Rechtsanwälte mbB
(86) International application number: PCT/JP2022/019541
(87) International publication number: WO 2022/234851

(57) **Abstract**

It was found that a binding molecule having features (i) of directly interacting with Asp 12 of mutant RAS (G12D), and (ii) of interacting with a Switch 2 region of the mutant RAS (G12D), and having the Switch 2 region of the mutant RAS (G12D) intramolecularly interacting with Asp 12 of the mutant RAS (G12D) has high binding selectivity for the mutant RAS (G12D). Such a molecule can be a promising candidate compound for treating and/or preventing a disease related to expression of the mutant RAS (G12D).

## Description

### [Technical Field]

The present disclosure relates to a binding molecule exhibiting selective binding to mutant RAS (G12D).

### [Background Art]

RAS is a protein belonging to the small GTPase family, and KRAS, NRAS, and HRAS are known. RAS is prescribed to be in an inactive state or an active state depending on a binding state to GDP or GTP, is activated through an exchange reaction from GDP to GTP by GEF (guanine nucleotide exchange factor), and is inactivated through a hydrolysis reaction of GTP by a GAP (GTPase-activating protein) (Non Patent Literature 1). Activated RAS activates various downstream signals such as MAPK pathway, PI3K/Akt pathway, and RAL pathway to induce growth, survival, and differentiation of cells, and thus, constitutive activation of RAS plays a significant role in occurrence and progression of cancer. It is known that RAS-RAF-MEK-ERK pathway is activated in cancer by activation of RAS upstream signal, RAS constitutive activation, and/or RAS active form mutation (Non Patent Literature 2). Such RAS active form mutations are found in a large number of types of cancers. G12, G13, G61 are known as hot spots of RAS mutation, and mutation highly frequently occurs in G12 in KRAS and in Q61 in NRAS. It is also known that these mutations relate to prognosis of patients (Non Patent Literature 3).

In particular, selective inhibitors against KRAS G12 mutation are clinically variable. These compounds are inhibitors highly selective for and covalently binding to G12C. As another feature, selectivity for G12C is higher than that for wild type KRAS, which probably increases safety with the medicinal effect retained.

Representative KRAS G12 mutations are G12D, G12V, and G12C, and among these, the number of cancer patients having KRAS G12D mutation is the largest, and hence there is a demand for a therapeutic agent for cancer with G12D mutation.

For example, a low molecular compound has been disclosed as an inhibitor against KRAS G12D (Patent Literature 2), and some cyclic peptide compounds have been disclosed as compounds exhibiting selective binding to KRAS G12D (Patent Literature 1, and Non Patent Literatures 4 and 5).

### [Citation List]

### [Patent Literature]

[Patent Literature 1]
   US20210032293A
[Patent Literature 2]
   WO2021041671

### [Non Patent Literature]

[Non Patent Literature 1]
   Nat. Rev. Drug Discov. 2014 Nov; 13(11):828-851.
[Non Patent Literature 2]
   Nat. Rev. Drug Discov. 2014 Dec;13(12):928-942.
[Non Patent Literature 3]
   Nat. Rev. Drug Discov. 2016 Nov; 15(11):771-785.
[Non Patent Literature 4]
   ACS Med. Chem. Lett. 2017, 8, 732-736
[Non Patent Literature 5]
   ACS Cent. Sci. 2020, 6, 1753-1761

### [Summary of Invention]

### [Technical Problem]

Patent Literature 1, Non Patent Literature 4, and Non Patent Literature 5 disclose peptide compounds exhibiting prescribed selective binding to KRAS G12D. In order to use these peptide compounds as active ingredients of effective drugs, it is necessary to find more excellent selective binding. There has not been found, however, systematic mechanism for finding which binding form should be achieved in mutant RAS (G12D) to improve selective binding.

### [Solution to Problem]

Under these circumstances, the present inventors made earnest studies, resulting in finding surprisingly that binding selectivity of a compound for mutant RAS (G12D) is remarkably improved by satisfying the following conditions:
1. the compound interacts with Asp12 of mutant RAS (G12D); and
2. the compound interacts with the Switch 2 region of the mutant RAS (G12D), and the Switch 2 region of the mutant RAS (G12D) and Asp12 of the mutant RAS (G12D) intermolecularly interact with each other.

The present invention is based on these findings, and encompasses the following:
[1] A binding molecule having the following features (1) and (2), and having binding selectivity for mutant RAS (G12D) 5 times or more over wild type RAS:
   (1) the binding molecule interacts with Asp12 of the mutant RAS (G12D); and
   (2) the binding molecule interacts with a Switch 2 region of the mutant RAS (G12D), and an amino acid residue present in the Switch 2 region intermolecularly interacts with Asp12 in the mutant RAS (G12D).
[2] The binding molecule according to [1], wherein the binding molecule interacts with an α3 helix region of the mutant RAS (G12D).
[3] The binding molecule according to [1] or [2], wherein when the amino acid residue present in the Switch 2 region is intermolecularly interacting with Asp12, a distance between an α carbon atom of Asp69 present in the Switch 2 region and an α carbon atom of Gln99 present in the α3 helix region is 15.0 angstroms (Å) or less in the mutant RAS (G12D).
[4] The binding molecule according to any one of [1] to [3], wherein the binding selectivity is 10 times or more.
[5] The binding molecule according to any one of [1] to [4], wherein a dissociation constant (K_{D}) for the mutant RAS (G12D) is 10 nM or less.
[6] The binding molecule according to any one of [1] to [5], wherein the mutant RAS (G12D) is mutant KRAS (G12D).
[7] The binding molecule according to any one of [1] to [6], wherein the mutant RAS (G12D) is in GDP form.
[8] The binding molecule according to any one of [1] to [7], wherein the Switch 2 region contains Ala59 to Glu76.
[9] The binding molecule according to any one of [1] to [8], wherein the Switch 2 region has an amino acid sequence of SEQ ID NO: 6.
[10] The binding molecule according to any one of [1] to [9], wherein the amino acid residue that is present in the Switch 2 region and interacts with the Asp12 is any one or more selected from the group consisting of Ala59, Gly60, Gln61, and Glu62.
[11] The binding molecule according to [10], wherein a sum of interaction energies between all atoms contained in a main chain of an amino acid residue, out of the Ala59, Gly60, Gln61, and Glu62, having one or more non-hydrogen atoms and present within a distance of 4.5 angstroms (Å) or less from either of two oxygen atoms of a carboxy group of a side chain of the Asp12, and all atoms of Asp12 is -0.5 kcal/mol or less.
[12] The binding molecule according to [10], wherein a sum of interaction energies between all atoms contained in an amino acid residue, out of the Ala59, Gly60, Gln61, and Glu62, having one or more non-hydrogen atoms and present within a distance of 4.5 angstroms (Å) or less from either of two oxygen atoms of a carboxy group of a side chain of the Asp12, and all atoms of Asp12 is -0.5 kcal/mol or less.
[13] The binding molecule according to any one of [1] to [12], wherein the binding molecule interacts with any one or more amino acid residues selected from the group consisting of Gln61 to Met72 of the Switch 2 region.
[14] The binding molecule according to any one of [1] to [12], wherein the binding molecule interacts with any one or more amino acid residues selected from the group consisting of Gln61 to Arg73 of the Switch 2 region.
[15] The binding molecule according to [13], wherein Gln61 to Met72 of the Switch 2 region are represented by an amino acid sequence of SEQ ID NO: 7.
[16] The binding molecule according to [14], wherein Gln61 to Arg73 of the Switch 2 region are represented by an amino acid sequence of SEQ ID NO: 10.
[17] The binding molecule according to any one of [1] to [12], [14], and [16], wherein the binding molecule interacts with any one or more amino acid residues selected from the group consisting of Gln61, Glu62, Arg68, Asp69, Met72, and Arg73 of the Switch 2 region.
[18] The binding molecule according to any one of [1] to [13] and [15], wherein the binding molecule interacts with any one or more amino acid residues selected from the group consisting of Gln61, Glu62, Arg68, Asp69, and Met72 of the Switch 2 region.
[19] The binding molecule according to any one of [1] to [18], wherein the binding molecule interacts with Gln61 of the Switch 2 region.
[20] The binding molecule according to any one of [1] to [19], comprising an amino acid residue that interacts with Gln61, wherein a sum of interaction energies between all atoms of the amino acid residue that interacts with the Gln61 and all atoms of the Gln61 is -10 kcal/mol or less.
[21] The binding molecule according to any one of [2] to [20], wherein the α3 helix region of the mutant RAS (G12D) contains Thr87 to Lys104.
[22] The binding molecule according to any one of [2] to [21], wherein the α3 helix region of the mutant RAS (G12D) has an amino acid sequence of SEQ ID NO: 8.
[23] The binding molecule according to any one of [2] to [22], wherein the binding molecule interacts with any one or more amino acid residues selected from the group consisting of His95 to Val103 of the α3 helix region of the mutant RAS (G12D).
[24] The binding molecule according to [23], wherein His95 to Val103 of the α3 helix region of the mutant RAS (G12D) are represented by an amino acid sequence of SEQ ID NO: 9.
[25] The binding molecule according to any one of [2] to [24], wherein the binding molecule interacts with any one or more amino acid residues selected from the group consisting of His95, Tyr96, Gln99, Arg102, and Val103 of the α3 helix region of the mutant RAS (G12D).
[26] The binding molecule according to any one of [2] to [25], wherein the binding molecule interacts with Gln99 of the α3 helix region of the mutant RAS (G12D).
[27] The binding molecule according to any one of [1] to [26], comprising one or more α amino acid residues that interacts with the Switch 2 region.
[28] The binding molecule according to any one of [2] to [27], comprising one or more α amino acid residues that interacts with the α3 helix region.
[29] The binding molecule according to any one of [1] to [28], comprising one or more α amino acid residues that interacts with the Asp12 amino acid.
[30] The binding molecule according to any one of [1] to [29], comprising a structure (1) of *-(linear or branched C₁-C₈ alkylene)-X, or *-(linear or branched C₁-C₈ alkylene)-(C₆-C₁₀ arylene)-X, wherein X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4- to 8-membered heterocycle containing 1 to 3 hetero atoms selected from the group consisting of N, O, and S), C₁-C₃ alkyl, C₁-C₃ haloalkyl, -C=CH, or optionally substituted C₃-C₈ cycloalkyl, * means a binding point, and if the binding molecule comprises *-(linear or branched Ci-Cs alkylene)-X, X is not C₁-C₃ alkyl.
[31] The binding molecule according to any one of [1] to [30], comprising a structure (2) of *-(linear or branched C₁-C₈ alkylene)-Y, wherein Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and * means a binding point.
[32] The binding molecule according to any one of [1] to [31], comprising the following structure (3): 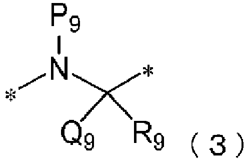
   wherein R₉ is C₁-C₆ alkyl, optionally substituted C₁-C₆ alkoxy C₁-C₆ alkyl, or optionally substituted C₆-C₁₀ aryl, or
   R₉ forms a 4- to 7-membered saturated heterocycle together with carbon atoms to which P₉ and R₉ are bound, and a nitrogen atom to which P₉ is bound,
   P₉ is hydrogen or C₁-C₃ alkyl unless R₉ and P₉ form a 4- to 7-membered saturated heterocycle,
   Q₉ is hydrogen or C₁-C₆ alkyl, and
   * means a binding point.
[33] The binding molecule according to [31] or [32], wherein an atom farthest from the binding point of the structure (1) among atoms contained in X of the structure (1), and an atom farthest from the binding point of the structure (2) among atoms contained in Y of the structure (2) are bound to each other via 20 or less atoms.
[34] The binding molecule according to [32] or [33], wherein an atom farthest from the binding point of the structure (1) among atoms contained in X of the structure (1), and an atom farthest from a carbon atom binding to Q₉ and R₉ in the structure (3) among atoms contained in R₉ of the structure (3) are bound to each other via 25 or less atoms unless R₉ and P₉ form a 4- to 7-membered saturated heterocycle.
[35] The binding molecule according to any one of [1] to [34], wherein a molecular weight of the binding molecule is 5,000 or less.
[36] The binding molecule according to any one of [1] to [35], wherein CLogP of the binding molecule is 25 or less.
[37] The binding molecule according to any one of [1] to [36] that is a peptide compound.
[38] The binding molecule according to any one of [1] to [37] that is a cyclic peptide compound.
[39] The binding molecule according to any one of [1] to [38] that is a peptide compound having 5 to 30 amino acid residues.
[40] The binding molecule according to any one of [1] to [39] that is a peptide compound containing one or more non-natural amino acid residues.
[41] The binding molecule according to any one of [1] to [40] that is a peptide compound containing one or more N-substituted amino acid residues.
[42] The binding molecule according to any one of [1] to [41], comprising the following structure: 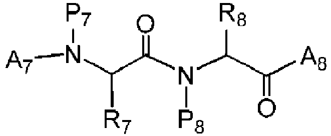
   wherein A₇ and As independently are an arbitrary amino acid residue, or an arbitrary peptide residue, A₇ and A₈ being optionally linked to each other,
   R₇ is -(linear or branched C₁-C₈ alkylene)-X, or -(linear or branched C₁-C₈ alkylene)-(C₆-C₁₀ arylene)-X,
   X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4-to 8-membered heterocycle containing 1 to 3 hetero atoms selected from the group consisting of N, O, and S), C₁-C₃ alkyl, C₁-C₃ haloalkyl, -C=CH, or optionally substituted C₃-C₈ cycloalkyl, and if R₇ is -(linear or branched C₁-C₈ alkylene)-X, X is not C₁-C₃ alkyl,
   P₇ is hydrogen or C₁-C₃ alkyl,
   R₈ is -(linear or branched C₁-C₈ alkylene)-Y,
   Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and
   P₈ is hydrogen or C₁-C₃ alkyl.
[43] The binding molecule according to any one of [1] to [42], comprising the following structure: 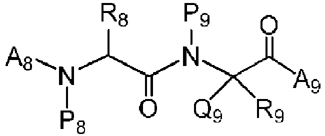
   wherein A₈ and A₉ independently are an arbitrary amino acid residue, or an arbitrary peptide residue, A₈ and A₉ being optionally linked to each other,
   R₈ is -(linear or branched C₁-C₈ alkylene)-Y,
   Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl,
   P₈ is hydrogen or C₁-C₃ alkyl,
   R₉ is C₁-C₆ alkyl, optionally substituted C₁-C₆ alkoxy C₁-C₆ alkyl, or optionally substituted C₆-C₁₀ aryl, or
   R₉ forms a 4- to 7-membered saturated heterocycle together with carbon atoms to which P₉ and R₉ are bound, and a nitrogen atom to which P₉ is bound,
   P₉ is hydrogen or C₁-C₃ alkyl unless R₉ and P₉ form a 4- to 7-membered saturated heterocycle, and
   Q₉ is hydrogen or C₁-C₆ alkyl.
[44] The binding molecule according to any one of [1] to [43], comprising the following structure: 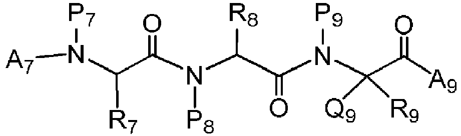
   wherein A₇ and A₉ independently are an arbitrary amino acid residue, or an arbitrary peptide residue, A₇ and A₉ being optionally linked to each other,
   R₇ is -(linear or branched C₁-C₈ alkylene)-X, or -(linear or branched C₁-C₈ alkylene)-(C₆-C₁₀ arylene)-X,
   X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4-to 8-membered heterocycle containing 1 to 3 hetero atoms selected from the group consisting of N, O, and S), C₁-C₃ alkyl, C₁-C₃ haloalkyl, -C=CH, or optionally substituted C₃-C₈ cycloalkyl, and if R₇ is -(linear or branched C₁-C₈ alkylene)-X, X is not C₁-C₃ alkyl,
   P₇ is hydrogen or C₁-C₃ alkyl,
   R₈ is -(linear or branched C₁-C₈ alkylene)-Y,
   Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl,
   P₈ is hydrogen or C₁-C₃ alkyl,
   R₉ is C₁-C₆ alkyl, optionally substituted C₁-C₆ alkoxy C₁-C₆ alkyl, or optionally substituted C₆-C₁₀ aryl, or
   R₉ forms a 4- to 7-membered saturated heterocycle together with carbon atoms to which P₉ and R₉ are bound, and a nitrogen atom to which P₉ is bound,
   P₉ is hydrogen or C₁-C₃ alkyl unless R₉ and P₉ form a 4- to 7-membered saturated heterocycle, and
   Q₉ is hydrogen or C₁-C₆ alkyl.
[45] The binding molecule according to [43] or [44], wherein an atom farthest from an α carbon atom to which R₇ is bound among atoms contained in X, and an atom farthest from an α carbon atom to which R₉ is bound among atoms contained in R₉ are bound to each other via 25 or less atoms unless R₉ and P₉ form a 4- to 7-membered saturated heterocycle.
[46] The binding molecule according to any one of [2] to [45], wherein interaction with the Switch 2 region, interaction with the Asp12, and/or interaction with the α3 helix region are not a covalent bond.
[47] The binding molecule according to any one of [2] to [46], wherein interaction with the Switch 2 region, interaction with the Asp12, and/or interaction with the α3 helix are each independently selected from the group consisting of electrostatic interaction, van der Waals interaction, and interaction through a hydrogen bond.
[48] The binding molecule according to any one of [2] to [47], wherein interaction with the Switch 2 region, interaction with the Asp12, and/or interaction with the α3 helix region are interactions not via another molecule.
[49] The binding molecule according to any one of [1] to [48], wherein interaction with the Asp12 is an interaction not via another molecule.
[50] The binding molecule according to any one of [1] to [49], wherein the amino acid residue present in the Switch 2 region and the Asp12 intermolecularly interacts with each other not via another molecule in the mutant RAS (G12D).
[51] The binding molecule according to any one of [1] to [50], wherein interaction with the Switch 2 region is an interaction not via another molecule.
[52] The binding molecule according to any one of [1] to [51], wherein the binding molecule is not a compound selected from the group consisting of the following (1) to (149):
   (1) (6S,9S,13S,16S,22S,25S,31S,34S)-6-[(2S)-butan-2-yl]-25-[(3-iodophenyl)methyl]-22-[(4-methoxyphenyl)methyl]-4,10,14,19,19,20,29,32-octamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (2) (3S,6S,9S,12S,18S,27S,30S,34S)-6-benzyl-27-[(2S)-butan-2-yl]-9-butyl-12-[(3-iodophenyl)methyl]-18-[(4-methoxyphenyl)methyl]-1,6,7,16,19,22,25,31-octamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (3) (3S,6S,9S,13S,16S,22S,25S,31S,34S)-6-[(2S)-butan-2-yl]-31-(cyclohexylmethyl)-25-[(3-iodophenyl)methyl]-22-[(4-methoxyphenyl)methyl]-3,4,10,14,19,19,20,29,32-nonamethyl-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (4) (3S,6S,9S,13S,16S,22S,25S,31S,34S)-6-[(2S)-butan-2-yl]-25-[(3-iodophenyl)methyl]-22-[(4-methoxyphenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (5) (3S,9S,12S,18S,27S,30S,34S)-27-[(2S)-butan-2-yl]-12-[(3-iodophenyl)methyl]-9-[(2-methoxyphenyl)methyl]-1,6,6,7,16,19,22,25,31-nonamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (6) (3S,9S,12S,18S,27S,30S,34S)-27-[(2S)-butan-2-yl]-18-(cyclohexylmethyl)-12-[(3-iodophenyl)methyl]-9-[(4-methoxyphenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (7) (3S,6S,9S,12S,18S,27S,30S,34S)-27-[(2S)-butan-2-yl]-9-butyl-18-[(4-chlorophenyl)methyl]-6-[(3-fluorophenyl)methyl]-12-[(3-iodophenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (8) (3S,9S,12S,18S,27S,30S,34S)-27-[(2S)-butan-2-yl]-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (9) (3S,6S,9S,13S,16S,22S,25S,31S,34S)-6-[(2S)-butan-2-yl]-22-butyl-9-ethyl-25-[(5-iodo-2-methylphenyl)methyl]-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (10) (3S,9S,12S,18S,27S,30S,34S)-27-[(2S)-butan-2-yl]-18-(cyclohexylmethyl)-12-[(3-iodophenyl)methyl]-1,6,6,7,16,19,22,25,31-nonamethyl-9-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (11) (3S,6S,9S,13S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-22-(3-methylbut-2-enyl)-9,16-bis(2-methylpropyl)-13 -(piperidine-1 -carbonyl)-1,4,7,10,14,17,20,23,26,29,32-undecazabicyclo[32.2.0]hexatriacontane-2, 5,8,11,15,18,21,24,27,30,33-undecone,
   (12) (6S,9S,13S,16S,22S,25S,31S,34S)-6-[(2S)-butan-2-yl]-25-[(3-iodophenyl)methyl]-4,10,14,19,19,20,29,32-octamethyl-9,16-bis(2-methylpropyl)-13-(piperidine-1-carbonyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
   (13) (3S,9S,12S,18S,27S,30S,34S)-12-[(3-bromophenyl)methyl]-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (14) (3S,6S,9S,12S,18S,27S,30S,34S)-27-[(2S)-butan-2-yl]-9-butyl-12-[(3-iodophenyl)methyl]-1,6,7,16,19,22,25,31-octamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (15) (3 S,6S,9S,13 S,16S,22S,25S,31S,34S)-6-[(2S)-butan-2-yl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-31-[[4-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
   (16) (3S,9S,12S,18S,27S,30S,34S)-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-18-[[4-(trifluoromethoxy)phenyl]methyl]-1,4,7,10,13,16,19,22,25,28,31-undecazacyclotetratriacontane-2,5,8,11,14,17,20,23,26,29,32-undecone,
   (17) (3S,9S,12S,18S,27S,30S,34S)-27-[(2S)-butan-2-yl]-18-(cyclohexylmethyl)-9-[(4-fluorophenyl)methyl]-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (18) (3 S,6S,9S,13 S,16S,22S,25S,31S,34S)-25-[(3-bromophenyl)methyl]-6-[(2S)-butan-2-yl]-22-butyl-31-[(4-chlorophenyl)methyl]-3,4,10,14,19,19,20,29,32-nonamethyl-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (19) (3S,9S,12S,18S,27S,30S,34S)-12-benzyl-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (20) (3S,6S,9S,13S,16S,22S,25S,31S,34S)-25-[(5-bromo-2-methylphenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (21) (3S,9S,12S,18S,27S,30S,34S)-27-[(2S)-butan-2-yl]-9-butyl-18-[(4-chlorophenyl)methyl]-12-[(3-ethynylphenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (22) (3S,6S,9S,13S,16S,22S,25S,31S,34S)-31-benzyl-6-[(2S)-butan-2-yl]-25-[(3-iodophenyl)methyl]-3,4,10,14,19,19,20,29,32-nonamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (23) (3S,9S,12S,18S,27S,30S,34S)-27-[(2S)-butan-2-yl]-9-butyl-18-[(4-chlorophenyl)methyl]-12-[(5-chlorothiophen-2-yl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (24) (3S,9S,12S,18S,27S,30S,34S)-12-[(5-bromothiophen-2-yl)methyl]-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (25) (3S,9S,12S,18S,27S,30S,34S)-27-[(2S)-butan-2-yl]-9-butyl-18-[(4-chlorophenyl)methyl]-12-[(4-fluorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (26) (3S,6S,12S,15S,20S,23S,26S,30S,33S,36R)-23-[(2S)-butan-2-yl]-3-butyl-6-[(3-iodophenyl)methyl]-10,13,20,21,27,31-hexamethyl-12-[(4-methylphenyl)methyl]-26,33-bis(2-methylpropyl)-3 0-(piperidine-1 -carbonyl)-1,4,7,10,13,18,21,24,27,31,34-undecazatricyclo[34.3.0.015,18]nonatriacontane-2,5,8,11,14,19,22,25,28,32,35-undecone,
   (27) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,12,16,19,22,25,31-decamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (28) (3S,6S,9S,13S,16S,22S,25S,31S,34S)-6-[(2S)-butan-2-yl]-31-(cyclopentylmethyl)-25-[(3-iodophenyl)methyl]-3,4,10,14,19,19,20,29,32-nonamethyl-22-(2-methylprop-2-enyl)-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (29) (3S,9S,12S,18S,27S,30S,34S)-27-[(2S)-butan-2-yl]-9-butyl-18-(cyclohexylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (30) (3S,6S,9S,13S,16S,22S,25S,31S,34S)-6-[(2S)-butan-2-yl]-22-butyl-31-(cyclobutylmethyl)-25-[(3-iodophenyl)methyl]-3,4,10,14,19,19,20,29,32-nonamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (31) (6S,9S,13S,16S,22S,25S,31S,34S)-6-[(2S)-butan-2-yl]-22-butyl-31-(cyclohexylmethyl)-25-[(3-iodophenyl)methyl]-4,10,14,19,19,20,29,32-octamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (32) (3S,6S,9S,13S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-22-(2-methylprop-2-enyl)-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (33) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-9-(2-methylprop-2-enyl)-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (34) (3S,9S,12S,18S,27S,30S,34R)-27-[(2S)-butan-2-yl]-18-[(4-chlorophenyl)methyl]-12-[(3-iodophenyl)methyl]-1,6,6,7,16,19,22,25,31,34-decamethyl-9-(3-methylbut-2-enyl)-3,30-bis(2-methylpropyl)-1,4,7,10,13,16,19,22,25,28,31-undecazacyclotetratriacontane-2,5,8,11,14,17,20,23,26,29,32-undecone,
   (35) (6S,9S,13R,16S,22S,25S,31S,34S)-6-[(2S)-butan-2-yl]-31-[(4-chlorophenyl)methyl]-25-[(3-iodophenyl)methyl]-4,10,13,14,19,19,20,29,32-nonamethyl-22-(3-methylbut-2-enyl)-9,16-bis(2-methylpropyl)-1,4,7,10,14,17,20,23,26,29,32-undecazabicyclo[32.2.0]hexatriacontane-2,5,8,11,15,18,21,24,27,30,33-undecone,
   (36) (6S,9S,13S,16S,22S,25S,31S,34S)-6-[(2S)-butan-2-yl]-31-[(4-chlorophenyl)methyl]-25-[(3-iodophenyl)methyl]-4,10,14,19,19,20,29,32-octamethyl-22-(2-methylprop-2-enyl)-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (37) (3S,9S,12S,18S,27S,30S,34S)-27-[(2S)-butan-2-yl]-9-butyl-12-[(3-iodophenyl)methyl]-1,6,6,7,16,19,22,25,31-nonamethyl-3-(3-methylbutyl)-30-(2-methylpropyl)-34-(piperidine-1-carbonyl)-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
   (38) (3S,9S,12S,18S,27S,30S,34R)-27-[(2S)-butan-2-yl]-12-[(3-iodophenyl)methyl]-1,6,6,7,16,19,22,25,31,34-decamethyl-3,30-bis(2-methylpropyl)-9-prop-2-inyl-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
   (39) (6S,9S,13R,16S,22S,25S,31S,34S)-6-[(2S)-butan-2-yl]-25-[(3-iodophenyl)methyl]-4,10,13,14,19,19,20,29,32-nonamethyl-9,16-bis(2-methylpropyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
   (40) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
   (41) (3S,6S,9S,13S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (42) (3S,9S,12S,18S,27S,30S,34S)-27-[(2S)-butan-2-yl]-9-butyl-18-[(4-chlorophenyl)methyl]-3-(cyclobutylmethyl)-12-[(3-iodophenyl)methyl]-1,6,6,7,16,19,22,25,31-nonamethyl-30-(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (43) (3S,9S,12S,18S,27S,30S,34S)-27-[(2S)-butan-2-yl]-9-butyl-18-(cyclopentylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (44) (3S,9S,12S,18S,27S,30S,34S)-27-[(2S)-butan-2-yl]-3,9-dibutyl-18-[(4-chlorophenyl)methyl]-12-[(3-iodophenyl)methyl]-1,6,6,7,16,19,22,25,31-nonamethyl-30-(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (45) (1S,4S,7S,12S,15S,21S,24S,30S,33S)-4-[(2S)-butan-2-yl]-24-butyl-15-(cyclopentylmethyl)-21-[(3-iodophenyl)methyl]-6,7,14,17,26,27,27,32-octamethyl-30-(2-methylpropyl)-33-(piperidine-1-carbonyl)-3,6,9,14,17,20,23,26,29,32,36-undecazatricyclo[34.4.0.09,12]tetracontane-2,5,8,13,16,19,22,25,28,31,35-undecone,
   (46) (3S,6S,9S,13S,16S,22S,25S,31S,34S)-6-[(2S)-butan-2-yl]-22-butyl-31-(cyclopentylmethyl)-25-[(3-iodophenyl)methyl]-3,4,10,14,19,19,20,29,32-nonamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (47) (3S,9S,12S,18S,27S,30S,34S)-27-[(2S)-butan-2-yl]-9-butyl-18-[(4-chlorophenyl)methyl]-19-ethyl-12-[(3-iodophenyl)methyl]-1,6,6,7,16,22,25,31-octamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (48) (3S,9S,12S,18S,27S,30S,34S)-27-[(2S)-butan-2-yl]-9-butyl-18-[(4-chlorophenyl)methyl]-12-[(3,5-dichlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (49) (3S,9S,12S,18S,27S,30S,34S)-27-[(2S)-butan-2-yl]-9-butyl-12-[(3-chloro-5-fluorophenyl)methyl]-18-[(4-chlorophenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (50) (3S,9S,12S,18S,27S,30S,34S)-27-[(2S)-butan-2-yl]-9-butyl-12-[(3-chloro-4-fluorophenyl)methyl]-18-[(4-chlorophenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (51) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,9,30-tris(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (52) (3S,9S,12S,18S,27S,30S,34S)-27-[(2S)-butan-2-yl]-18-[(4-chlorophenyl)methyl]-9-ethyl-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (53) (3S,9S,12S,18S,27S,30S,34S)-3-benzyl-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-34-(pyrrolidine-1 -carbonyl)-1,4,7,10,13,16,19,22,25,28,31 - undecazacyclotetratriacontane-2,5,8,11,14,17,20,23,26,29,32-undecone,
   (54) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-3-propyl-34-(pyrrolidine-1-carbonyl)-1,4,7,10,13,16,19,22,25,28,31-undecazacyclotetratriacontane-2,5,8,11,14,17,20,23,26,29,32-undecone,
   (55) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-3-(2-phenylethyl)-34-(pyrrolidine-1-carbonyl)-1,4,7,10,13,16,19,22,25,28,31-undecazacyclotetratriacontane-2,5,8,11,14,17,20,23,26,29,32-undecone,
   (56) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,30-bis(2-methylpropyl)-9-prop-2-enyl-34-(pyrrolidine-1 -carbonyl)-1,4,7,10,13,16,19,22,25,28,31 -undecazacyclotetratriacontane-2,5,8,11,14,17,20,23,26,29,32-undecone,
   (57) (3S,9S,12S,18S,27S,30S,34S)-27-[(2S)-butan-2-yl]-9-butyl-12-[(3-chlorophenyl)methyl]-18-[(4-chlorophenyl)methyl]-1,6,6,7,16,19,22,25,31-nonamethyl-3,30-bis(2-methylpropyl)-34-(pyrrolidine-1 -carbonyl)-1,4,7,10,13,16,19,22,25,28,31 -undecazacyclotetratriacontane-2,5,8,11,14,17,20,23,26,29,32-undecone,
   (58) (3S,9S,12S,18S,27S,30S,34S)-27-[(2S)-butan-2-yl]-9-butyl-18-[(4-chlorophenyl)methyl]-12-[(3-methoxyphenyl)methyl]-1,6,6,7,16,19,22,25,31-nonamethyl-3,30-bis(2-methylpropyl)-34-(pyrrolidine-1-carbonyl)-1,4,7,10,13,16,19,22,25,28,31-undecazacyclotetratriacontane-2,5,8,11,14,17,20,23,26,29,32-undecone,
   (59) (3S,6S,9S,12S,18S,27S,30S,34S)-6,9-dibenzyl-27-[(2S)-butan-2-yl]-18-[(4-chlorophenyl)methyl]-4,7,16,19,22,25,30,31-octamethyl-12-[(3-methylphenyl)methyl]-3-propan-2-yl-34-(pyrrolidine-1 -carbonyl)-1,4,7,10,13,16,19,22,25,28,31 -undecazacyclotetratriacontane-2,5,8,11,14,17,20,23,26,29,32-undecone,
   (60) (3S,6S,9S,12S,18S,27S,30S,34S)-6,9-dibenzyl-27-[(2S)-butan-2-yl]-18-[(4-chlorophenyl)methyl]-12-[(3-fluorophenyl)methyl]-4,7,16,19,22,25,30,31-octamethyl-3-propan-2-yl-34-(pyrrolidine-1 -carbonyl)-1,4,7,10,13,16,19,22,25,28,31 -undecazacyclotetratriacontane-2,5,8,11,14,17,20,23,26,29,32-undecone,
   (61) (3S,6S,9S,12S,18S,27S,30S,34S)-6,9-dibenzyl-27-[(2S)-butan-2-yl]-18-[(4-chlorophenyl)methyl]-12-[(3,5-difluorophenyl)methyl]-4,7,16,19,22,25,30,31-octamethyl-3-propan-2-yl-34-(pyrrolidine-1-carbonyl)-1,4,7,10,13,16,19,22,25,28,31-undecazacyclotetratriacontane-2,5,8,11, 14,17,20,23,26,29,32-undecone,
   (62) (3S,6S,9S,12S,18S,27S,30S,34S)-6,9-dibenzyl-27-[(2S)-butan-2-yl]-12-[(3-chlorophenyl)methyl]-18-[(4-chlorophenyl)methyl]-25-ethyl-4,7,16,19,22,30,31-heptamethyl-3-propan-2-yl-34-(pyrrolidine-1-carbonyl)-1,4,7,10,13,16,19,22,25,28,31-undecazacyclotetratriacontane-2,5,8,11, 14,17,20,23,26,29,32-undecone,
   (63) (3S,6S,9S,12S,18S,27S,30S,34S)-6,9-dibenzyl-27-[(2S)-butan-2-yl]-12-[(3-chlorophenyl)methyl]-18-[(4-chlorophenyl)methyl]-16-ethyl-4,7,19,22,25,30,31-heptamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
   (64) (3S,9S,18S,21S,25S,28S,31S,34S,36R)-18-[(2S)-butan-2-yl]-3-[(3-chlorophenyl)methyl]-9-[(4-chlorophenyl)methyl]-3 6-ethoxy-7,10,13,16,21,22,29,32-octamethyl-31 -(3 - methylbutoxymethyl)-28-(2-methylpropyl)-25-(pyrrolidine-1-carbonyl)-1,4,7,10,13,16,19,22,26,29,32-undecazabicyclo[32.3.0]heptatriacontane-2,5,8,11,14,17,20,23,27,30,33-undecone,
   (65) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,16,19,22,25,30,31-octamethyl-3,9-bis(2-methylpropyl)-34-(pyrrolidine-1 -carbonyl)-1,4,7,10,13,16,19,22,25,28,31 - undecazacyclotetratriacontane-2,5,8,11,14,17,20,23,26,29,32-undecone,
   (66) (3S,6S,9S,12S,18S,27S,30S,34S)-6-benzyl-27-[(2S)-butan-2-yl]-12-[(3-chloro-5-fluorophenyl)methyl]-18-[(4-chlorophenyl)methyl]-4,7,16,19,22,25,30,31-octamethyl-3,9-bis(2-methylpropyl)-34-(pyrrolidine-1 -carbonyl)-1,4,7,10,13,16,19,22,25,28,31 - undecazacyclotetratriacontane-2,5,8,11,14,17,20,23,26,29,32-undecone,
   (67) (3S,6S,9S,12S,18S,27S,30S,34S)-6-benzyl-27-[(2S)-butan-2-yl]-18-[(4-chlorophenyl)methyl]-12-[(3-iodophenyl)methyl]-4,7,16,19,22,25,30,31-octamethyl-3,9-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (68) (3S,6S,9S,12S,18S,27S,30S,34R)-6-benzyl-27-[(2S)-butan-2-yl]-18-[(4-chlorophenyl)methyl]-12-[(3-iodophenyl)methyl]-4,7,16,19,22,25,30,31,34-nonamethyl-3,9-bis(2-methylpropyl)-1,4,7,10,13,16,19,22,25,28,31-undecazacyclotetratriacontane-2,5,8,11,14,17,20,23,26,29,32-undecone,
   (69) 3-[[(3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-2, 5, 8,11,14,17,20,23,26,29,32-undecaoxo-34-(pyrrolidine-1-carbonyl)-1,4,7,10,13,16,19,22,25,28,31-undecazacyclotetratriacont-12-yl]methyl]benzonitrile,
   (70) (3S,6S,9S,12S,18S,27S,30S,34S)-6-benzyl-27-[(2S)-butan-2-yl]-18-[(4-chlorophenyl)methyl]-12-[(3-iodophenyl)methyl]-4,7,16,19,22,25,30,31-octamethyl-3,9-bis(2-methylpropyl) -3 4-(pyrroli dine-1-carbonyl)-1,4,7,10,13,16,19,22,25,28,3 1-undecazacyclotetratriacontane-2,5,8,11,14,17,20,23,26,29,32-undecone,
   (71) (3S,9S,18S,21S,25S,28S,31S,34S,36R)-31-benzyl-18-[(2S)-butan-2-yl]-9-[(4-chlorophenyl)methyl]-36-ethoxy-3-[(3-iodophenyl)methyl]-7,10,13,16,21,22,29,32-octamethyl-28-(2-methylpropyl)-25-(pyrrolidine-1 -carbonyl)-1,4,7,10,13,16,19,22,26,29,32-undecazabicyclo[32.3.0]heptatriacontane-2,5,8,11,14,17,20,23,27,30,33-undecone,
   (72) (3S,6S,9S,12S,18S,27S,30S,34S)-6-benzyl-27-[(2S)-butan-2-yl]-12-[(3-chlorophenyl)methyl]-18-[(4-chlorophenyl)methyl]-7,16,19,22,25,30,31-heptamethyl-3,9-bis(2-methylpropyl)-34-(pyrrolidine-1 -carbonyl)-1,4,7,10,13,16,19,22,25,28,31 - undecazacyclotetratriacontane-2,5,8,11,14,17,20,23,26,29,32-undecone,
   (73) 3-[[(3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-2,5,8,11,14,17,20,23,26,29,32-undecaoxo-34-(piperidine-1-carb onyl)-1,4,7,10,13,16,19,22,25,28,31-undecazacyclotetratriacont-12-yl]methyl]benzonitrile,
   (74) (3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-34-(piperidine-1-carbonyl)-12-[[3-(trifluoromethoxy)phenyl]methyl]-1,4,7,10,13,16,19,22,25,28,31-undecazacyclotetratriacontane-2,5,8,11,14,17,20,23,26,29,32-undecone,
   (75) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,16,19,22,25,30,31-octamethyl-9-[(3-methylphenyl)methyl]-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
   (76) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,9,16,19,22,25,30,31-nonamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
   (77) (3S,9S,12S,18S,27S,30S,34S)-27-[(2S)-butan-2-yl]-9-butyl-18-[(4-chlorophenyl)methyl]-12-[(2-fluoro-5-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (78) (3S,9S,12S,18S,27S,30S,34S)-27-[(2S)-butan-2-yl]-9-butyl-12-[(2-chloro-5-iodophenyl)methyl]-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (79) (1S,10S,13S,17S,20S,26S,29S)-10-[(2S)-butan-2-yl]-29-[(3-iodophenyl)methyl]-2,5,8,14,18,23,23,24,3 3-nonamethyl-13,20-bis(2-methylpropyl)-17-(piperi dine-1 -carbonyl)-2,5,8,11,14,18,21,24,27,30,33-undecazabicyclo[24.8.6]tetracontane-3,6,9,12,15,19,22,25,28,31,34-undecone,
   (80) (6S,9S,13S,16S,22S,25S,31S,34S)-6-[(2S)-butan-2-yl]-22-butyl-9-hexyl-25-[(3-iodophenyl)methyl]-31-[(4-methoxyphenyl)methyl]-4,10,14,19,19,20,29,32-octamethyl-16-(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (81) (6S,9S,13S,16S,22S,25S,31S,34S)-6-[(2S)-butan-2-yl]-25-[(3-iodophenyl)methyl]-22-[(2-methoxyphenyl)methyl]-4,10,14,19,19,20,29,32-octamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (82) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-22-(phenoxymethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (83) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-22-(2-phenylethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (84) (3S,9S,12S,18S,27S,30S,34S)-27-[(2S)-butan-2-yl]-18-(cyclohexylmethyl)-12-[(3-iodophenyl)methyl]-1,6,6,7,16,19,22,25,31-nonamethyl-3,30-bis(2-methylpropyl)-34-(piperidine-1 -carbonyl)-9-[[4-(trifluoromethyl)phenyl]methyl]-1,4,7,10,13,16,19,22,25,28,31 - undecazacyclotetratriacontane-2,5,8,11,14,17,20,23,26,29,32-undecone,
   (85) (3S,9S,12S,18S,27S,30S,34S)-27-[(2S)-butan-2-yl]-9-[(4-chlorophenyl)methyl]-18-(cyclohexylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (86) (3S,6S,9S,13S,16S,22S,25S,31S,34S)-6-[(2S)-butan-2-yl]-31-[(4-fluorophenyl)methyl]-25-[(3-iodophenyl)methyl]-3,4,10,14,19,19,20,29,32-nonamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (87) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-13-(piperidine-1-carbonyl)-22-[[2-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
   (88) (6S,9S,13S,16S,22S,25S,31S,34S)-6-[(2S)-butan-2-yl]-22-[(2-chlorophenyl)methyl]-25-[(3-iodophenyl)methyl]-4,10,14,19,19,20,29,32-octamethyl-31-[(4-methylphenyl)methyl]-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (89) (6S,9S,13S,16S,22S,25S,31S,34S)-6-[(2S)-butan-2-yl]-22-[(2-fluorophenyl)methyl]-25-[(3-iodophenyl)methyl]-4,10,14,19,19,20,29,32-octamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (90) (6S,9S,13S,16S,22S,25S,31S,34S)-6-[(2S)-butan-2-yl]-25-[(3-iodophenyl)methyl]-4,10,14,19,19,20,29,32-octamethyl-22-[(2-methylphenyl)methyl]-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (91) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-22-(2-phenylethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (92) (6S,9S,13S,16S,22S,25S,31S,34S)-6-[(2S)-butan-2-yl]-31-(cyclohexylmethyl)-25-[(3-iodophenyl)methyl]-4,10,14,19,19,20,29,32-octamethyl-9,16-bis(2-methylpropyl)-22-(2-phenylethyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (93) (3S,9S,12S,18S,27S,30S,34S)-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
   (94) (3S,9S,12S,18S,27S,30S,34S)-27-[(2S)-butan-2-yl]-12-[(3-iodophenyl)methyl]-18-[(4-methoxyphenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
   (95) (3S,9S,12S,18S,27S,30S,34S)-18-benzyl-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
   (96) (3S,9S,12S,18S,27S,30S,34S)-27-[(2S)-butan-2-yl]-18-[(4-fluorophenyl)methyl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
   (97) (6S,9S,13S,16S,22S,25S,31S,34S)-6-[(2S)-butan-2-yl]-25-[(3-iodophenyl)methyl]-4,10,14,19,19,20,29,32-octamethyl-9,16-bis(2-methylpropyl)-13-(piperidine-1-carbonyl)-22-prop-2-inyl-31-[[4-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
   (98) (6S,9S,13S,16S,22S,25S,31S,34S)-6-[(2S)-butan-2-yl]-25-[(3-iodophenyl)methyl]-31-[(4-methoxyphenyl)methyl]-4,10,14,19,19,20,29,32-octamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (99) (6S,9S,13S,16S,22S,25S,31S,34S)-6-[(2S)-butan-2-yl]-31-[(4-fluorophenyl)methyl]-25-[(3-iodophenyl)methyl]-4,10,14,19,19,20,29,32-octamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (100) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-9-(2-phenylethyl)-34-(piperidine-1 -carbonyl)-1,4,7,10,13,16,19,22,25,28,31 - undecazacyclotetratriacontane-2,5,8,11,14,17,20,23,26,29,32-undecone,
   (101) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1 -carbonyl)-9-[[2-(trifluoromethyl)phenyl]methyl]-1,4,7,10,13,16,19,22,25,28,31 - undecazacyclotetratriacontane-2,5,8,11,14,17,20,23,26,29,32-undecone,
   (102) (3S,9S,12S,18S,27S,30S,34S)-27-[(2S)-butan-2-yl]-9-[(2-chlorophenyl)methyl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (103) (3S,9S,12S,18S,27S,30S,34S)-27-[(2S)-butan-2-yl]-9-[(2-fluorophenyl)methyl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (104) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
   (105) (3S,6S,9S,13S,16S,22S,25S,31S,34S)-6-[(2S)-butan-2-yl]-25-[(3-iodophenyl)methyl]-31-[(4-methoxyphenyl)methyl]-3,4,10,14,19,19,20,29,32-nonamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
   (106) (3S,9S,12S,18S,27S,30S,34S)-27-[(2S)-butan-2-yl]-12-[(3-iodophenyl)methyl]-1,6,6,7,16,19,22,25,31-nonamethyl-9-[(2-methylphenyl)methyl]-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (107) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1-carbonyl)-9-[[3-(trifluoromethyl)phenyl]methyl]-1,4,7,10,13,16,19,22,25,28,31-undecazacyclotetratriacontane-2,5,8,11,14,17,20,23,26,29,32-undecone,
   (108) (3S,6S,9S,13S,16S,22S,25S,31S,34S)-22-benzyl-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (109) (3S,6S,9S,13S,16S,22S,25S,31S,34S)-6-[(2S)-butan-2-yl]-25-[(3-iodophenyl)methyl]-22-[(2-methoxyphenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (110) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-13-(piperidine-1-carbonyl)-22-[[2-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
   (111) (3S,6S,9S,12S,18S,27S,30S,34S)-27-[(2S)-butan-2-yl]-9-butyl-18-[(4-chlorophenyl)methyl]-6-[(2-fluorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (112) (3S,6S,9S,12S,18S,27S,30S,34S)-27-[(2S)-butan-2-yl]-9-butyl-18-[(4-chlorophenyl)methyl]-6-[(4-fluorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (113) (3S,6S,9S,12S,18S,27S,30S,34S)-27-[(2S)-butan-2-yl]-9-butyl-6-[(2-chlorophenyl)methyl]-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (114) (3S,6S,9S,12S,18S,27S,30S,34S)-27-[(2S)-butan-2-yl]-9-butyl-6-[(3-chlorophenyl)methyl]-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (115) (3S,6S,9S,12S,18S,27S,30S,34S)-27-[(2S)-butan-2-yl]-9-butyl-6,18-bis[(4-chlorophenyl)methyl]-12-[(3-iodophenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (116) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-6-[(2-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (117) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-6-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (118) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[2-(trifluoromethyl)phenyl]methyl]-1,4,7,10,13,16,19,22,25,28,31-undecazacyclotetratriacontane-2,5,8,11,14,17,20,23,26,29,32-undecone,
   (119) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[3-(trifluoromethyl)phenyl]methyl]-1,4,7,10,13,16,19,22,25,28,31-undecazacyclotetratriacontane-2,5,8,11,14,17,20,23,26,29,32-undecone,
   (120) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[4-(trifluoromethyl)phenyl]methyl]-1,4,7,10,13,16,19,22,25,28,31-undecazacyclotetratriacontane-2,5,8,11,14,17,20,23,26,29,32-undecone,
   (121) (3S,6S,9S,12S,18S,27S,30S,34S)-27-[(2S)-butan-2-yl]-9-butyl-18-[(4-chlorophenyl)methyl]-12-[(3-iodophenyl)methyl]-6-[(2-methoxyphenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (122) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[2-(trifluoromethoxy)phenyl]methyl]-1,4,7,10,13,16,19,22,25,28,31-undecazacyclotetratriacontane-2,5,8,11,14,17,20,23,26,29,32-undecone,
   (123) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[3-(trifluoromethoxy)phenyl]methyl]-1,4,7,10,13,16,19,22,25,28,31-undecazacyclotetratriacontane-2,5,8,11,14,17,20,23,26,29,32-undecone,
   (124) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[4-(trifluoromethoxy)phenyl]methyl]-1,4,7,10,13,16,19,22,25,28,31-undecazacyclotetratriacontane-2,5,8,11,14,17,20,23,26,29,32-undecone,
   (125) (6S,9S,13S,16S,19S,22S,25S,31S,34S)-19-benzyl-6-[(2S)-butan-2-yl]-22-butyl-31-(cyclohexylmethyl)-25-[(3-iodophenyl)methyl]-4,10,14,20,29,32-hexamethyl-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (126) (6S,9S,13S,16S,19S,22S,25S,31S,34S)-19-benzyl-6-[(2S)-butan-2-yl]-22-butyl-25-[(3-iodophenyl)methyl]-31-[(4-methoxyphenyl)methyl]-4,10,14,19,20,29,32-heptamethyl-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (127) (3S,6S,9S,12S,18S,27S,30S,34S)-27-[(2S)-butan-2-yl]-9-butyl-12-[(2-fluorophenyl)methyl]-6,18-bis[(4-methoxyphenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
   (128) (3S,9S,12S,17S,20S,23S,27S,30S,36S)-20-[(2S)-butan-2-yl]-9-(cyclohexylmethyl)-7,10,17,18,23,24,28,31-octamethyl-3-(3-phenylpropyl)-27-(piperidine-1-carbonyl)-30-propan-2-ylspiro[1,4,7,10,15,18,21,24,28,31,34-undecazatricyclo[34.3.0.012,15]nonatriacontane-33,1'-cyclopentane]-2,5,8,11,16,19,22,25,29,32,3 5-undecone,
   (129) (3S,6S,9S,13S,16S,22S,25S,31S,34S)-6-[(2S)-butan-2-yl]-22-butyl-25-[(2,3-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (130) (3S,6S,9S,13S,16S,22S,25S,31S,34S)-6-[(2S)-butan-2-yl]-22-butyl-25-[(2,5-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (131) (3S,6S,9S,13S,16S,22S,25S,31S,34S)-6-[(2S)-butan-2-yl]-22-butyl-25-[(2,6-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (132) (3S,6S,9S,13S,16S,22S,25S,31S,34S)-6-[(2S)-butan-2-yl]-22-butyl-9-ethyl-25-[(2-fluoro-3-iodophenyl)methyl]-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (133) (3S,6S,9S,13S,16S,22S,25S,31S,34S)-25-[(3-bromo-2-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (134) (3S,6S,9S,13S,16S,22S,25S,31S,34S)-6-[(2S)-butan-2-yl]-22-butyl-9-ethyl-25-[(3-fluoro-5-iodophenyl)methyl]-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (135) (3S,6S,9S,13S,16S,22S,25S,31S,34S)-6-[(2S)-butan-2-yl]-22-butyl-25-[(3-chloro-5-iodophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (136) (3S,6S,9S,13S,16S,22S,25S,31S,34S)-25-[(5-bromopyridine-3-yl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (137) (3S,6S,9S,13S,16S,22S,25S,31S,34S)-25-[(3-bromo-5-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (138) (3S,6S,9S,13S,16S,22S,25S,31S,34S)-25-[(2-bromo-5-iodophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
   (139) (3S,6S,9S,13S,16S,22S,25S,31S,34S)-25-[(5-bromo-2-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (140) (3S,6S,9S,13S,16S,22S,25S,31S,34S)-25-[(5-bromo-2-chlorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
   (141) (3S,9S,18S,21S,25S,28S,34S)-18-[(2S)-butan-2-yl]-28-cyclohexyl-9-(cyclohexylmethyl)-7,10,13,16,21,22,26,29-octamethyl-3-(3-phenylpropyl)-25-(piperidine-1- carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone,
   (142) (3S,6S,9S,12S,18S,27S,30S,34S)-6-benzyl-27-[(2S)-butan-2-yl]-12-[(3-iodophenyl)methyl]-1,7,9,16,19,22,25,31-octamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
   (143) (3S,6S,9S,13S,16S,19S,22S,25S,31S,34S)-19-benzyl-6-[(2S)-butan-2-yl]-25-[(3-iodophenyl)methyl]-31-[(4-methoxyphenyl)methyl]-3,4,9,10,14,20,22,29,32-nonamethyl-16-(2-methylpropyl)-13-(pyrrolidine-1-carbonyl)-1,4,7,10,14,17,20,23,26,29,32-undecazabicyclo[32.2.0]hexatriacontane-2,5,8,11,15,18,21,24,27,30,33-undecone,
   (144) 3-[(3S,6S,9S,13S,16S,19S,22S,25S,31S,34S)-6-[(2S)-butan-2-yl]-22-butyl-25-[(3-iodophenyl)methyl]-31-[(4-methoxyphenyl)methyl]-3,4,10,14,20,29,32-heptamethyl-9,16-bis(2-methylpropyl)-2, 5, 8,11,15,18,21,24,27,3 0,33-undecaoxo-13-(piperidine-1-carbonyl)-1,4,7,10,14,17,20,23,26,29,32-undecazabicyclo[32.2.0]hexatriacontane-19-yl]-N,N-dimethylpropanamide,
   (145) 3-[(3S,6S,9S,12S,18S,27S,30S,34S)-27-[(2S)-butan-2-yl]-9-butyl-12-[(3-iodophenyl)methyl]-1,7,16,19,22,25,31-heptamethyl-18-[(4-methylphenyl)methyl]-3,30-bis(2-methylpropyl)-2, 5, 8,11,14,17,20,23,26,29,32-undecaoxo-34-(piperidine-1-carbonyl)-1,4,7,10,13,16,19,22,25,28,31-undecazacyclotetratriacont-6-yl]-N,N-dimethylpropanamide,
   (146) (3S,9S,12S,18S,27S,30S,34S)-27-[(2S)-butan-2-yl]-34-(4,4-difluoropiperidine-1-carbonyl)-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-1,4,7,10,13,16,19,22,25,28,31-undecazacyclotetratriacontane-2,5,8,11,14,17,20,23,26,29,32-undecone,
   (147) (3S,9S,12S,18S,27S,30S,34S)-27-[(2S)-butan-2-yl]-34-(3,3-difluoropiperidine-1-carbonyl)-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-1,4,7,10,13,16,19,22,25,28,31-undecazacyclotetratriacontane-2,5,8,11,14,17,20,23,26,29,32-undecone,
   (148) (3S,9S,18S,21S,25S,28S,34S)-18-[(2S)-butan-2-yl]-28-cyclohexyl-9-(cyclohexylmethyl)-7,10,13,16,21,22,26,29-octamethyl-25-(piperidine-1-carbonyl)-3-[3-[4-(trifluoromethyl)phenyl]propyl]spiro[1,4,7,10,13,16,19,22,26,29,32-undecazabicyclo[32.3.0]heptatriacontane-31,1'-cyclopentane]-2,5,8,11,14,17,20,23,27,30,33-undecone, and
   (149) (3S,6S,9S,12S,18S,27S,30S,34S)-6,12-dibenzyl-27-[(2S)-butan-2-yl]-18-[(4-chlorophenyl)methyl]-4,7,16,19,22,25,30,31-octamethyl-3,9-bis(2-methylpropyl)-34-(pyrrolidine-1 -carbonyl)-1,4,7,10,13,16,19,22,25,28,31 -undecazacyclotetratriacontane-2,5,8,11,14,17,20,23,26,29,32-undecone.
[53] A pharmaceutical composition, comprising the binding molecule according to any one of [1] to [52].
[54] A cancer therapeutic agent, comprising the binding molecule according to any one of [1] to [52].

### [Advantageous Effects of Invention]

The present invention has provided a novel binding molecule having high binding selectivity for mutant RAS (G12D). The binding molecule of the present disclosure has binding selectivity for mutant RAS (G12D) 5 times or more over wild type RAS. The present disclosure has also revealed binding form between mutant RAS (G12D) and the binding molecule required for achieving high selectivity for mutant RAS (G12D). A molecule binding to mutant RAS (G12D) through the binding form revealed by the present disclosure is expected as a therapeutic candidate molecule in treatment and/or prevention of a disease in which expression of mutant RAS (G12D) involves.

### [Brief Description of Drawings]

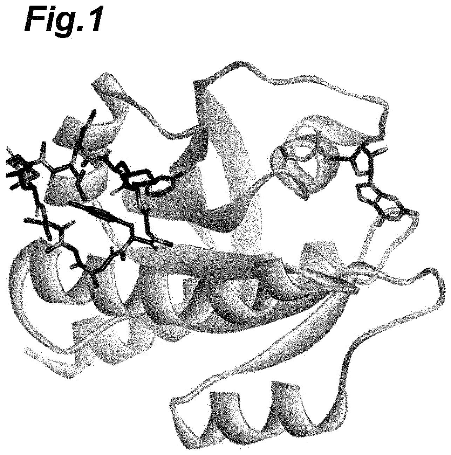

[Figure 1] Figure 1 illustrates the entire structure of an X-ray crystal structure of a complex of a compound 1 described in Example 1 and mutant RAS (G12D). The mutant RAS (G12D) is illustrated in the shape of a ribbon, and the compound 1 and guanosine diphosphate (GDP) are illustrated in the shape of sticks.

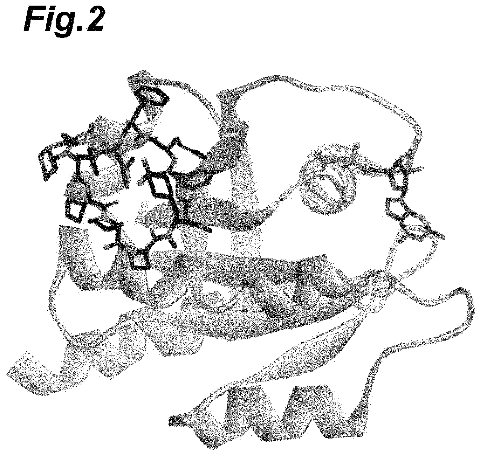

[Figure 2] Figure 2 illustrates the entire structure of an X-ray crystal structure of a complex of a compound 2 described in Example 2 and mutant RAS (G12D). The mutant RAS (G12D) is illustrated in the shape of a ribbon, and the compound 2 and guanosine diphosphate (GDP) are illustrated in the shape of sticks.

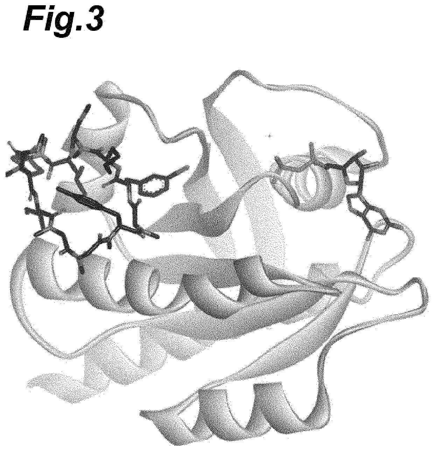

[Figure 3] Figure 3 illustrates the entire structure of an X-ray crystal structure of a complex of a compound 3 described in Example 3 and mutant RAS (G12D). The mutant RAS (G12D) is illustrated in the shape of a ribbon, and the compound 3 and guanosine diphosphate (GDP) are illustrated in the shape of sticks.

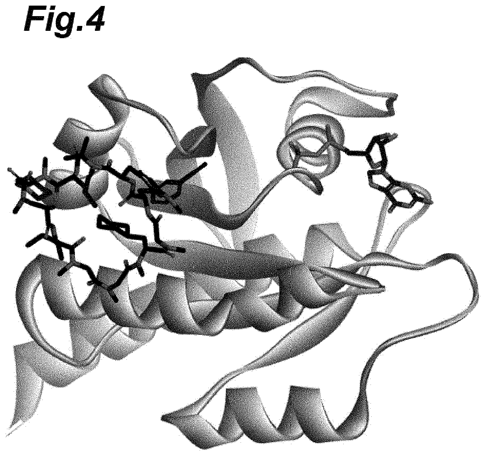

[Figure 4] Figure 4 illustrates the entire structure of an X-ray crystal structure of a complex of a compound 4 described in Example 4 and mutant RAS (G12D). The mutant RAS (G12D) is illustrated in the shape of a ribbon, and the compound 4 and guanosine diphosphate (GDP) are illustrated in the shape of sticks.

### [Description of Embodiments]

### Definitions of Functional Groups and the like

Herein, "alkyl" refers to a monovalent group induced by removing one arbitrary hydrogen atom from aliphatic hydrocarbon, and does not contain, in the skeleton, a hetero atom (namely, an atom excluding carbon and hydrogen atoms) or an unsaturated carbon-carbon bond but has a subset of hydrocarbyl or hydrocarbon group structure containing hydrogen and a carbon atom. The alkyl encompasses not only a linear group but also a branched group. Specifically, the alkyl is an alkyl having 1 to 20 carbon atoms (C₁-C₂₀; hereinafter "Cₚ-C_{q}" means that the number of carbon atoms is p to q), is preferably C₁-C₁₀ alkyl, and more preferably C₁-C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

Herein, "alkenyl" refers to a monovalent group having at least one double bond (two adjacent SP² carbon atoms). Depending on configuration of the double bond and a substituent (if present), the geometric form of the double bond can be in the Entgegen (E) or Zusammen (Z) configuration, or the cis or trans configuration. The alkenyl encompasses not only linear groups but also branched groups. The alkenyl is preferably C₂-C₁₀ alkenyl, and more preferably C₂-C₆ alkenyl, and specific examples include vinyl, ally, 1-propenyl, 2-propenyl, 1-butenyl, 2-butenyl (including those of cis and trans configurations), 3-butenyl, pentenyl, 3-methyl-2-butenyl, and hexenyl.

Herein, "alkynyl" refers to a monovalent group having at least one triple bond (two adjacent SP carbon atoms). The alkynyl encompasses not only linear groups but also branched groups. The alkynyl is preferably C₂-C₁₀ alkynyl, and more preferably C₂-C₆ alkynyl, and specific examples include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

Herein, "cycloalkyl" means a saturated or partially saturated monovalent cyclic aliphatic hydrocarbon group, and encompasses monocyclic, bicyclic, and spirocyclic groups. The cycloalkyl is preferably C₃-C₈ cycloalkyl, and specific examples include cyclopropyl, cyclobutyl, cyclopentyl, cyclohexyl, cycloheptyl, cyclooctyl, bicyclo[2.2.1]heptyl, and spiro[3.3]heptyl.

Herein, "aryl" means a monovalent aromatic hydrocarbon ring, and is preferably C₆-C₁₀ aryl. Specific examples of the aryl include phenyl and naphthyl (such as 1-naphthyl and 2-naphthyl).

"Haloalkyl" used herein means a group obtained by replacing one or more hydrogen atoms of the "alkyl" defined above with halogen atoms, and is preferably C₁-C₆ haloalkyl, and more preferably C₁-C₆ fluoroalkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, and 5,5-difluoropentyl.

"Alkoxyalkyl" used herein means a group obtained by replacing one or more hydrogen atoms of the "alkyl" defined above with the "alkoxy" defined above, and is preferably C₁-C₆ alkoxy C₁-C₆ alkyl, and more preferably C₁-C₆ alkoxy C₁-C₂ alkyl. Specific examples of the alkoxyalkyl include methoxymethyl, ethoxymethyl, 1-propoxymethyl, 2-propoxymethyl, n-butoxymethyl, i-butoxymethyl, s-butoxymethyl, t-butoxymethyl, pentyloxymethyl, 3-methylbutoxymethyl, 1-methoxyethyl, 2-methoxyethyl, and 2-ethoxyethyl.

Herein, "alkylene" means a divalent group induced by removing one arbitrary hydrogen atom from the "alkyl", and is preferably C₁-C₈ alkylene. Specific examples of the alkylene include -CH₂-, -(CH₂)₂-, -(CH₂)₃-, -CH(CH₃)CH₂-, -C(CH₃)₂-, -(CH₂)₄-, -CH(CH₃)CH₂CH₂-, - C(CH₃)₂CH₂-, -CH₂CH(CH₃)CH₂-, -CH₂C(CH₃)₂-, -CH₂CH₂CH(CH₃)-, -(CH₂)₅-, -(CH₂)₆-, - (CH₂)₇-, and -(CH₂)₈-.

Herein, "arylene" means a divalent group induced by removing one arbitrary hydrogen atom from the "aryl". The arylene may be monocyclic or condensed cyclic. The number of atoms constituting the ring is not especially limited, and is preferably 6 to 10 (C₆₋₁₀ arylene). Specific examples of the arylene include 1,2-phenylene, 1,3-phenylene, 1,4-phenylene, 1,2-naphthylene, 1,3-naphthylene, and 1,4-naphthylene.

A "heterocycle" used herein means a non-aromatic heterocycle containing preferably 1 to 5, and more preferably 1 to 3 heteroatoms in atoms constituting the ring. The heterocycle may have a double and/or triple bond in the ring, a carbon atom contained in the ring may be oxidized to form carbonyl, and may be monocyclic, condensed cyclic, or spiro cyclic. The number of atoms constituting the ring is preferably 3 to 12 (3- to 12-membered heterocycle), and more preferably 4 to 8 (4- to 8-membered heterocycle).

Specific examples of the heterocycle include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an azocane ring, or a ring obtained by substituting one or more single bonds of any of these saturated heterocycles with a double bond or a triple bond.

A "saturated heterocycle" used herein means a non-aromatic heterocycle containing 1 to 5 heteroatoms in addition to a carbon atom, and not containing a double bond and/or triple bond in the ring. The saturated heterocycle may be monocyclic, or may form a condensed ring together with another ring, for example, an aromatic ring such as a benzene ring. The saturated heterocycle is preferably a 4- to 7-membered saturated heterocycle, and specific examples include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, and an indoline ring.

Herein, the term "optionally substituted" means that a given group may be substituted with an arbitrary substituent.

Herein, a "peptide" refers to a peptide in which 1, 2, 3, 4, 5 or more natural amino acids and/or non-natural amino acids are linked through an amide bond and/or an ester bond.

The term "amino acid" used herein encompasses natural amino acids, and non-natural amino acids. The "natural amino acid" used herein refers to Gly, Ala, Ser, Thr, Val, Leu, Ile, Phe, Tyr, Trp, His, Glu, Asp, Gln, Asn, Cys, Met, Lys, Arg, and Pro. The non-natural amino acid is not especially limited, and examples include β-amino acid, γ-amino acid, D-amino acid, N-substituted amino acid, α,α-disubstituted amino acid, an amino acid different in a side chain from a natural one, and hydroxycarboxylic acid. The amino acid herein may have an arbitrary configuration. Selection of a side chain of an amino acid is not especially limited, and the side chain may be freely selected from, for example, an alkyl group, an alkenyl group, an alkynyl group, an aryl group, a heteroaryl group, an aralkyl group, and a cycloalkyl group in addition to a hydrogen atom, and one or two methylene groups not adjacent to each other in these groups may be substituted with an oxygen atom, a carbonyl group (-CO-), or a sulfonyl group (-SO₂-). These may be provided with substituents, and the substituents are not also limited but may be one, two or more independently and freely selected from arbitrary substituents including, for example, a halogen atom, an O atom, a S atom, a N atom, a B atom, a Si atom, or a P atom. In other words, examples include optionally substituted alkyl group, alkenyl group, alkynyl group, aryl group, heteroaryl group, aralkyl group, and cycloalkyl group. In one nonrestrictive aspect, the amino acid used herein may be a compound having a carboxy group and an amino group in the same molecule (even in this case, imino acids such as proline and hydroxyproline are encompassed in the amino acid).

A main chain amino group of the amino acid may be unsubstituted (NH₂ group), or may be substituted (namely, -NHR group: wherein R represents alkyl, alkenyl, alkynyl, aryl, heteroaryl, aralkyl, or cycloalkyl optionally having a substituent, one or two methylene groups not adjacent to each other in these groups may be substituted with an oxygen atom, a carbonyl group (-CO-) or a sulfonyl group (-SO₂-), or a carbon chain bound to a N atom like proline and a carbon atom in the α-position may form a ring). The substituent of R is selected in the same manner as the substituent in the amino acid side chain described above. When the main chain amino group is substituted, the R is included in the "side chain of the amino acid" herein. Such an amino acid having a substituted main chain amino group is herein designated as "N-substituted amino acid". Preferable examples of the "N-substituted amino acid" herein include, but are not limited to, N-alkylamino acid, N-C₁-C₆ alkylamino acid, N-C₁-C₄ alkylamino acid, and N-methylamino acid.

Each "amino acid" constituting a peptide compound herein encompasses all corresponding isotopes. An isotope of "amino acid" is one in which at least one atom is substituted with another atom having the same atomic number (proton number) but a different mass number (total number of protons and neutrons). Examples of the isotope encompassed in the "amino acid" contained in the peptide compound of the present disclosure include a hydrogen atom, a carbon atom, a nitrogen atom, an oxygen atom, a phosphorus atom, a sulfur atom, a fluorine atom, and a chlorine atom, and these encompass ²H, ³H, ¹³C, ¹⁴C, ¹⁵N, ¹⁷O, ¹⁸O, ³¹P, ³²P, ³⁵S, ¹⁸F, ³⁶Cl and the like.

Examples of a substituent containing a halogen atom herein include an alkyl group, a cycloalkyl group, an alkenyl group, an alkynyl group, an aryl group, a heteroaryl group, and an aralkyl group each having halogen as a substituent, and more specific examples include fluoroalkyl, difluoroalkyl, and trifluoroalkyl.

Examples of a substituent containing an O atom include groups such as hydroxy (-OH), oxy (-OR), carbonyl (-C=O-R), carboxy (-CO₂H), oxycarbonyl (-C=O-OR), carbonyloxy (-O-C=O-R), thiocarbonyl (-C=O-SR), carbonylthio (-S-C=O-R), aminocarbonyl (-C=O-NHR), carbonylamino (-NH-C=O-R), oxycarbonylamino (-NH-C=O-OR), sulfonylamino (-NH-SO₂-R), aminosulfonyl (-SO₂-NHR), sulfamoylamino (-NH-SO₂-NHR), thiocarboxy (-C=O-SH), and carboxycarbonyl (-C=O-CO₂H).

Examples of oxy (-OR) include alkoxy, cycloalkoxy, alkenyloxy, alkynyloxy, aryloxy, heteroaryloxy, and aralkyloxy. As alkoxy, C₁-C₄ alkoxy and C₁-C₂ alkoxy are preferred, and in particular, methoxy or ethoxy is preferred.

Examples of carbonyl (-C=O-R) include formyl (-C=O-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C=O-OR) include alkyloxycarbonyl, cycloalkyloxycarbony, alkenyloxycarbonyl, alkynyloxycarbonyl, aryloxycarbonyl, heteroaryloxycarbonyl, and aralkyloxycarbonyl.

Examples of carbonyloxy (-O-C=O-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C=O-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbony.

Examples of carbonylthio (-S-C=O-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of aminocarbonyl (-C=O-NHR) include alkylaminocarbonyl (such as C₁-C₆ or C₁-C₄ alkylaminocarbonyl, examples of which include ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. In addition, examples include groups obtained by further substituting a H atom bound to a N atom of -C=O-NHR with alkyl, cycloalkyl, alkenyl, alkynyl, aryl, heteroaryl, or aralkyl.

Examples of carbonylamino (-NH-C=O-R) include alkylcarbonylamino, cycloalkylcarbonylamino, alkenylcarbonylamino, alkynylcarbonylamino, arylcarbonylamino, heteroarylcarbonylamino, and aralkylcarbonylamino. In addition, examples include groups obtained by further substituting a H atom bound to a N atom of -NH-C=O-R with alkyl, cycloalkyl, alkenyl, alkynyl, aryl, heteroaryl, or aralkyl.

Examples of oxycarbonylamino (-NH-C=O-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. In addition, examples include groups obtained by further substituting a H atom bound to a N atom of -NH-C=O-OR with alkyl, cycloalkyl, alkenyl, alkynyl, aryl, heteroaryl, or aralkyl.

Examples of sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. In addition, examples include groups obtained by further substituting a H atom bound to a N atom of -NH-SO₂-R with alkyl, cycloalkyl, alkenyl, alkynyl, aryl, heteroaryl, or aralkyl.

Examples of aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. In addition, examples include groups obtained by further substituting a H atom bound to a N atom of -SO₂-NHR with alkyl, cycloalkyl, alkenyl, alkynyl, aryl, heteroaryl, or aralkyl.

Examples of sulfamoylamino (-NH-SO₂-NHR) include alkylsulfamoylamino, cycloalkylsulfamoylamino, alkenyl sulfamoyl amino, alkynylsulfamoylamino, arylsulfamoylamino, heteroaryl sulfamoyl amino, and aralkylsulfamoylamino. In addition, two H atoms bound to a N atom of -NH-SO₂-NHR may be substituted with a substituent independently selected from the group consisting of alkyl, cycloalkyl, alkenyl, alkynyl, aryl, heteroaryl, and aralkyl, or these two substituents may form a ring.

Examples of a substituent containing a S atom include groups such as thiol (-SH), thio (-S-R), sulfinyl (-S=O-R), sulfonyl (-SO₂-R), and sulfo (-SO₃H).

An example of thio (-S-R) is selected from alkylthio, cycloalkylthio, alkenylthio, alkynylthio, arylthio, heteroarylthio, and aralkylthio.

Examples of sulfonyl (-SO₂-R) include alkylsulfonyl, cycloalkylsulfonyl, alkenylsulfonyl, alkynylsulfonyl, arylsulfonyl, heteroarylsulfonyl, and aralkylsulfonyl.

Examples of a substituent containing a N atom include groups such as azide (-N₃; also referred to as an "azido group"), cyano (-CN), primary amino (-NH₂), secondary amino (-NH-R; also referred to as mono-substituted amino), tertiary amino (-NR(R'); also referred to as di-substituted amino), amidino (-C(=NH)-NH₂), substituted amidino (-C(=NR)-NR'R"), guanidino (-NH-C(=NH)-NH₂), substituted guanidino (-NR-C(=NR"')-NR'R"), aminocarbonylamino (-NR-CO-NR'R"), pyridyl, piperidino, morpholino, and azetidinyl.

Examples of the secondary amino (-NH-R; mono-substituted amino) include alkylamino, cycloalkylamino, alkenylamino, alkynylamino, arylamino, heteroarylamino, and aralkylamino.

An example of tertiary amino (-NR(R'); di-substituted amino) includes an amino group having arbitrary two substituents each independently selected from, for example, alkyl(aralkyl)amino, alkyl, cycloalkyl, alkenyl, alkynyl, aryl, heteroaryl, aralkyl and the like, in which these arbitrary two substituents may form a ring. Specific examples include dialkylamino, and particularly, C₁-C₆ dialkylamino, C₁-C₄ dialkylamino, dimethylamino, and diethylamino. Herein, "Cₚ-C_{q} dialkylamino group" refers to a group obtained by substituting two Cₚ-C_{q} alkyl groups with amino groups, in which these Cₚ-C_{q} alkyl groups may be the same or different.

An example of substituted amidino (-C(=NR)-NR'R") includes a group in which three substituents R, R', and R" on a N atom are each independently selected from alkyl, cycloalkyl, alkenyl, alkynyl, aryl, heteroaryl, and aralkyl, such as alkyl(aralkyl)(aryl)amidino.

An example of substituted guanidino (-NR-C(=NR"')-NR'R") includes a group in which R, R', R", and R‴ are each independently selected from alkyl, cycloalkyl, alkenyl, alkynyl, aryl, heteroaryl, and aralkyl, or these groups form a ring.

An example of aminocarbonylamino (-NR-CO-NR'R") includes a group in which R, R', and R" are each independently selected from a hydrogen atom, alkyl, cycloalkyl, alkenyl, alkynyl, aryl, heteroaryl, and aralkyl, or these groups form a ring.

Herein, an "amino acid residue" contained in a peptide compound is sometimes referred to simply as an "amino acid", and a "peptide residue" is sometimes referred to simply as a "peptide".

Herein, the binding molecule can be a salt thereof, and preferably a chemically or pharmaceutically acceptable salt thereof. Besides, the binding molecule or a salt thereof of the present disclosure can be a solvate thereof, and preferably a chemically or pharmaceutically acceptable solvate thereof. The salt of the binding molecule of the present disclosure encompasses, for example, hydrochloride; hydrobromide; hydroiodide; phosphate; phosphonate; sulfate; sulfonate such as methanesulfonate, or p-toluenesulfonate; carboxylate such as acetate, citrate, malate, tartrate, succinate, or salicylate; or an alkali metal salt such as sodium salt, or potassium salt; an alkaline earth metal salt such as magnesium salt, or calcium salt; and an ammonium salt such as ammonium salt, alkylammonium salt, dialkylammonium salt, trialkylammonium salt, or tetraalkylammonium salt. Such a salt can be produced by, for example, contacting the binding molecule with an acid or a base usable in production of a pharmaceutical. In the present disclosure, a solvate of the binding molecule refers to a phenomenon that a solute molecule strongly attracts a solvent molecule in a solution to form one molecular group, which is designated as a hydrate when the solvent is water. A solvate of the binding molecule of the present disclosure is preferably a hydrate, and specific examples of the hydrate include mono- to decahydrates, preferably mono- to pentahydrates, and more preferably mono- to trihydrates. The solvate of the binding molecule of the present disclosure encompasses not only a solvate with a single solvent such as water, alcohol (such as methanol, ethanol, 1-propanol, or 2-propanol), or dimethylformamide, but also a solvate with a plurality of solvents.

Herein, the term "one or more" means one, or the number of two or more. When the term "one or more" is used in context relating to a substituent of a given group, the term means the number from one to the maximum number of substituents allowable in the group. Specifically, "one or more" can be, for example, 1, 2, 3, 4, 5, 6, 7, 8, 9, 10 and/or a larger number.

Herein, the sign "-" indicating a range includes precedent and subsequent values, and for example, "A-B" means a range of A or more and B or less.

Herein, the term "about" used in combination with a numerical value means a numerical range between +10% and -10% of the numerical value.

Herein, the definition of the term "and/or" encompasses all the appropriate combinations of "and" and "or". Specifically, for example, the term "A, B, and/or C" encompasses the following 7 variations:
(i) A, (ii) B, (iii) C, (iv) A and B, (v) A and C, (vi) B and C, and (vii) A, B, and C.

### Binding Molecule

The present disclosure relates to a binding molecule having the following features (1) and (2), and having binding selectivity for mutant RAS (G12D) 5 times or more over wild type RAS:
(1) the binding molecule interacts with Asp12 of the mutant RAS (G12D); and
(2) the binding molecule interacts with the Switch 2 region of the mutant RAS (G12D), and an amino acid residue present in the Switch 2 region intermolecularly interacts with Asp12 in the mutant RAS (G12D).

The binding molecule having the selective binding activity for the mutant RAS (G12D) of the present disclosure may further have the following features:
(3) the binding molecule interacts with the α3 helix region of the mutant RAS (G12D), and/or
(4) when an amino acid residue present in the Switch 2 region is intermolecularly interacting with Asp12, a distance between an α carbon atom of Asp69 present in the Switch 2 region and an α carbon atom of Gln99 present in the α3 helix region is 15.0 angstroms (Å) or less in the mutant RAS (G12D).

Besides, the present disclosure relates to a complex of the binding molecule having the features (1) to (4) described above and mutant RAS (G12D). It is noted that the "binding molecule" means a molecule capable of binding to a given molecule in the present disclosure. For example, when a molecule A is capable of binding to a molecule B, the molecule A is expressed as a binding molecule of the molecule B.

The binding molecule of the present disclosure is a molecule selectively binding to the mutant RAS (G12D). In the present disclosure, the mutant RAS (G12D) refers to RAS having a mutation of glycine (Gly) to aspartic acid (Asp) in the 12th amino acid residue of wild type RAS. The mutant RAS (G12D) of the present disclosure may have a mutation in an amino acid residue in another position as long as it has the mutation of glycine (Gly) to aspartic acid (Asp) in the 12th amino acid residue. Examples of the mutation in another position include a mutation of glycine (Gly) to aspartic acid (Asp) or valine (Val) in the 13th amino acid residue, a mutation of alanine (Ala) to serine (Ser) or glycine (Gly) in the 59th amino acid residue, a mutation of glutamine (Gln) to histidine (His), lysine (Lys), or leucine (Leu) in the 61st amino acid residue, and a mutation of tyrosine (Tyr) to aspartic acid (Asp), leucine (Leu), or serine (Ser) in the 96th amino acid residue. When RAS having such a mutation is expressed and accumulated in the body, a signaling pathway constantly accelerating growth of a cell is activated, which is presumed to easily cause cancer. Accordingly, a compound targeting RAS having such a mutation is expected as a candidate compound useful for treating and/or preventing cancer.

A source of the mutant RAS (G12D) is not especially limited in the present disclosure, and mutant RAS (G12D) derived from various animals such as a human, a mouse, a rat, a rabbit, a dog, a cat, a bovine, a horse, a pig, a goat, a rhesus monkey, a crab-eating monkey, a chimpanzee, and chicken can be encompassed, but the mutant RAS (G12D) of the present disclosure is preferably human-derived mutant RAS (G12D). As isoforms of RAS, three isoforms of NRAS, HRAS, and KRAS are known. An amino acid sequence of human-derived wild type NRAS is shown in SEQ ID NO: 2, an amino acid sequence of human-derived wild type HRAS is shown in SEQ ID NO: 3, and an amino acid sequence of human-derived wild type KRAS is shown in SEQ ID NO: 4.

The mutant RAS (G12D) of the present disclosure is not especially limited as long as it has a mutation of glycine (Gly) to aspartic acid (Asp) in the 12th amino acid residue in the wild type NRAS, HRAS, or KRAS, and is preferably mutant KRAS (G12D), and more preferably human-derived mutant KRAS (G12D). An amino acid sequence of human-derived KRAS (G12D) is shown in SEQ ID NO: 5.

Besides, RAS is known to be in an inactive state binding to GDP, or an active state binding to GTP. The mutant RAS (G12D) of the present disclosure may be in any of the GTP form or the GDP form, and is preferably in the GDP form.

### Binding Selectivity/Binding Activity

In one nonrestrictive aspect, the binding molecule of the present disclosure has high binding selectivity in binding to the mutant RAS (G12D) over wild type RAS. In another nonrestrictive aspect, the binding molecule of the present disclosure has high binding activity for the mutant RAS (G12D).

The binding selectivity for the mutant RAS (G12D) over wild type RAS can be obtained based on a ratio between binding activity of the binding molecule of the present disclosure for wild type RAS and binding activity of the binding molecule of the present disclosure for the mutant RAS (G12D). For example, assuming that binding selectivity for the mutant RAS (G12D) as compared with that for wild type RAS is defined as a value obtained by dividing [a dissociation constant (K_{D} value) of the binding molecule of the present disclosure for wild type RAS] by [a dissociation constant (K_{D} value) of the binding molecule of the present disclosure for the mutant RAS (G12D)], when the value is large, the binding selectivity for the mutant RAS (G12D) over wild type RAS is high, and on the contrary, when the value is small, the binding selectivity for the mutant RAS (G12D) over wild type RAS is low. Although not limited thereto, the binding molecule of the present disclosure has binding selectivity for the mutant RAS (G12D) preferably 5 times or more, 6 times or more, 7 times or more, 8 times or more, 9 times or more, 10 times or more, 11 times or more, 12 times or more, 13 times or more, 14 times or more, 15 times or more, 16 times or more, 17 times or more, 18 times or more, 19 times or more, 20 times or more, 21 times or more, 22 times or more, 23 times or more, 24 times or more, or 25 times or more over wild type RAS. An upper limit of the binding selectivity is not especially limited, and for example, is 100 times or less, 90 times or less, 80 times or less, 70 times or less, 60 times or less, 50 times or less, 40 times or less, 30 times or less, 25 times or less, 20 times or less, or 15 times or less. The value of the binding selectivity of the binding molecule of the present disclosure for the mutant RAS (G12D) over wild type RAS can be a value falling in a range specified by an arbitrary combination of these upper limits and lower limits.

In the present disclosure, the "binding activity" means inherent binding affinity reflecting 1: 1 interaction between members of a binding pair (for example, the binding molecule of the present disclosure and the mutant RAS (G12D) or wild type RAS).

A method for measuring binding affinity and a dissociation constant (K_{D}) is not especially limited, and a surface plasmon resonance method (BIACORE or the like) can be employed. A binding rate constant (kon) and a dissociation rate constant (koff) can be calculated by using one-to-one Langmuir binding model by simultaneously fitting binding and dissociation sensorgrams (Biacore Insight Evaluation Software (GE Healthcare)) or the like. A dissociation constant (K_{D}) is calculated as a koff/kon ratio. Besides, a temperature at the time of the measurement can be 30°C, HBS (10 mM HEPES-NaOH, 150 mM NaCl, pH 7.4) containing 1 mM DTT, 10 mM MgCl2, 0.01% Tween 20, 10 µM GDP, and 4% DMSO can be used as a running buffer, and the measurement can be performed in a state where biotinylated Avi-tag fused wild type RAS or mutant RAS is immobilized on a surface of Sensor Chip CAP (Cytiva) coated with Biotin CAPture Reagent.

The binding molecule of the present disclosure has high binding activity for the mutant RAS (G12D). Specifically, examples of the dissociation constant (K_{D}) of the binding molecule of the present disclosure for the mutant RAS (G12D) include, but are not limited to, 10.0 nM or less, 9.0 nM or less, 8.0 nM or less, 7.0 nM or less, 6.0 nM or less, 5.0 nM or less, 4.0 nM or less, 3.0 nM or less, 2.0 nM or less, or 1.0 nM or less.

### Direct Interaction

In one nonrestrictive aspect, the binding molecule of the present disclosure interacts with Asp12 of the mutant RAS (G12D). Accordingly, the binding molecule of the present disclosure can be a molecule capable of interacting with Asp12 of the mutant RAS (G12D). In the present disclosure, the interaction between the binding molecule and Asp12 of the mutant RAS (G12D) is preferably a direct interaction not via another molecule. Accordingly, the binding molecule of the present disclosure can be a molecule capable of directly interacting with Asp12 amino acid residue of the mutant RAS (G12D) not via presence of another molecule or the like. An example of another molecule includes, but is not limited to, a water molecule.

In the present disclosure, such interaction between the binding molecule and Asp12 of the mutant RAS (G12D) is designated as a "direct interaction". The binding molecule of the present disclosure can be a molecule capable of causing a direct interaction with Asp12 of the mutant RAS (G12D).

In the present disclosure, when the direct interaction is caused between the binding molecule and Asp12 of the mutant RAS (G12D), a distance between a non-hydrogen atom present in the binding molecule at the shortest interatomic distance from one of two oxygen atoms of a carboxy group contained in Asp12 of the mutant RAS (G12D) (hereinafter sometimes referred to as a "direct interaction atom") and an atom contained in Asp12 can be, but is not limited to, 4.50 angstroms (Å) or less, 4.40 A or less, 4.30 Å or less, 4.20 Å or less, 4.10 Å or less, 4.00 Å or less, 3.95 Å or less, 3.90 Å or less, 3.85 Å or less, 3.80 Å or less, 3.75 Å or less, 3.70 Å or less, 3.65 Å or less, 3.60 Å or less, 3.55 Å or less, 3.50 Å or less, 3.45 Å or less, 3.40 Å or less, 3.35 Å or less, 3.30 Å or less, 3.20 Å or less, 3.10 Å or less, 3.00 Å or less, 2.90 Å or less, or smaller. Examples of the direct interaction atom includes a fluorine atom, a chlorine atom, a bromine atom, an iodine atom, a carbon atom on a hydrogen side of end alkyne in an acetylene group, or a nitrogen atom of an amine group. Such an atom or substituent may be directly bound to a skeleton constructing the binding molecule, or may be bound via an optionally substituted linear or branched alkylene group, an optionally substituted cycloalkylene group, an optionally substituted cycloalkyl group, an optionally substituted arylene group, and/or an optionally substituted aryl group. Some of carbon atoms contained in such a linear or branched alkylene group, cycloalkylene group, cycloalkyl group, arylene group, or alkyl group may be substituted with other atoms. Such other atoms are preferably an oxygen atom, a nitrogen atom, a phosphorus atom, or a sulfur atom, and are more preferably a carbon atom, an oxygen atom, or a nitrogen atom. The number of atoms contained in a skeleton of the alkylene group is preferably 1 or more and 6 or less, and the number of atoms contained in a ring portion of the cycloalkylene group, the cycloalkyl group, the arylene group, or the aryl group is preferably 3 or more and 8 or less. Besides, arbitrary two, three, four or five groups selected from the group consisting of the alkylene group, the cycloalkylene group, the cycloalkyl group, the arylene group, and the aryl group may be bound to one another.

More specific examples of the direct interaction atom include an iodine atom substituted in the 3-position in a phenyl group of phenylalanine, or a carbon atom on hydrogen side of end alkyne of an acetylene group substituted in the 3-position of a phenyl group of phenylalanine, or a nitrogen atom located in the 4-position of a piperazine group. Besides, the interatomic distance is preferably a shorter one of an interatomic distance between the iodine atom substituted in the 3-position of a phenyl group of phenylalanine contained in the binding molecule, the carbon atom on hydrogen side of end alkyne of an acetylene group substituted in the 3-position of a phenyl group of phenylalanine, or the nitrogen atom located in the 4-position of a piperazine group, and one oxygen atom of two oxygen atoms of a carboxy group contained in Asp12 of the mutant RAS (G12D), and an interatomic distance between the iodine atom substituted in the 3-position of a phenyl group of phenylalanine contained in the binding molecule, the carbon atom on hydrogen side of end alkyne of an acetylene group substituted in the 3-position of a phenyl group of phenylalanine, or the nitrogen atom located in the 4-position of a piperazine group, and the other oxygen atom of the two oxygen atoms of a carboxy group contained in Asp12 of the mutant RAS (G12D).

When one or more hydrogen atoms are bound to the direct interaction atom, an atom, among the direct interaction atom and the one or plurality of hydrogen atoms bound to the direct interaction atom, most strongly interacting with an atom contained in a carboxy group contained in Asp12 of the mutant RAS (G12D) is preferably an atom causing the direct interaction between the binding molecule and Asp 12 of the mutant RAS (G12D). In other words, the direct interaction atom and an atom causing the direct interaction are not always the same. For example, when the iodine atom substituted in the 3-position of a phenyl group in phenylalanine is the direct interaction atom, the iodine atom is preferably an atom causing the direct interaction, when the carbon atom on hydrogen side of end alkyne of an acetylene group substituted in the 3-position of a phenyl group of phenylalanine is the direct interaction atom, a hydrogen atom of the end alkyne of the acetylene group is preferably an atom causing the direct interaction, and when the nitrogen atom located in the 4-position of a piperazine group is the direct interaction atom, hydrogen bound to the nitrogen atom is preferably an atom causing the direct interaction.

The binding molecule of the present disclosure can have a structure capable of causing the direct interaction with Asp12 of the mutant RAS (G12D). In the present disclosure, the structure capable of causing the direct interaction with Asp 12 of the mutant RAS (G12D) is not especially limited, and from the viewpoint of easily causing the direct interaction with Asp12, an example includes a structure (1) of *-(linear or branched C₁-C₈ alkylene)-X, or *-(linear or branched Ci-Cs alkylene)-(C₆-C₁₀ arylene)-X. Here, X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4- to 8-membered heterocycle containing 1 to 3 hetero atoms selected from the group consisting of N, O, and S), C₁-C₃ alkyl, C₁-C₃ haloalkyl, -C=CH, or optionally substituted C₃-C₈ cycloalkyl, and * means a binding point. If the binding molecule comprises *-(linear or branched C₁-C₈ alkylene)-X, X does not encompass C₁-C₃ alkyl.

It is noted that the structure (1) may be involved in an effect of easily causing intermolecular interaction between an amino acid residue present in the Switch 2 region and Asp12.

A preferable aspect of the structure (1) is, for example, an aspect in which -(linear or branched C₁-C₈ alkylene)-(C₆-C₁₀ arylene)- is benzyl or phenethyl, X is ethyl in the 3-position of a phenyl group, cyclopropyl, Cl, Br, I, or -C=CH, and is more preferably an aspect in which X is I in the 3-position of a phenyl group, or -C=CH.

Particularly preferable examples of the structure (1) include the following:

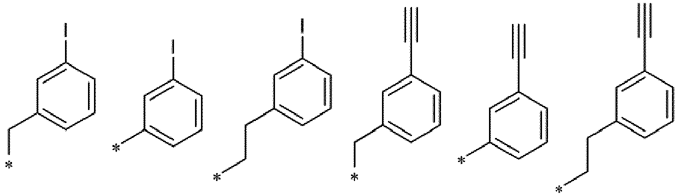

Further preferable examples of the structure (1) include the following:

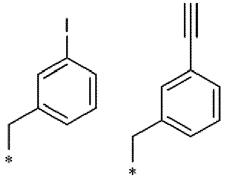

In one nonrestrictive aspect, an example of an atom contained in Asp 12 of the mutant RAS (G12D) involved in the direct interaction includes two oxygen atoms of a carboxy group.

In one nonrestrictive aspect, when the binding molecule of the present disclosure is a peptide compound, the peptide compound contains one or more amino acid residues that interact with Asp 12 of the mutant RAS (G12D). From the viewpoint of more easily causing the direct interaction with Asp12, the amino acid residue is preferably an α amino acid residue. A more specific example includes an α amino acid residue having the structure (1) in the side chain.

### Interaction between Binding Molecule and Switch 2 Region

In one nonrestrictive aspect, the binding molecule of the present disclosure interacts with the Switch 2 region of the mutant RAS (G12D). In the present disclosure, the Switch 2 region includes Ala59 to Glu76 of the mutant RAS (G12D) shown in SEQ ID NO: 6. The Switch 2 region of the present disclosure preferably consists of Ala59 to Glu76 of the mutant RAS (G12D). Accordingly, the binding molecule of the present disclosure can be a binding molecule capable of interacting with any one or more amino acid residues selected from the group consisting of Ala59 to Glu76 of the Switch 2 region of the mutant RAS (G12D).

In one nonrestrictive aspect, from the viewpoint of easily causing the interaction with the Switch 2 region, the binding molecule of the present disclosure can be a molecule that interacts with one or more (1, 2, 3, 4, 5, 6, 7, 8, 9, 10, or 11) amino acid residues selected from the group consisting of Gln61 to Met72 (amino acid sequence shown in SEQ ID NO: 7) of the Switch 2 region, or a molecule that interacts with one or more (1, 2, 3, 4, 5, 6, 7, 8, 9, 10, 11, 12, or 13) amino acid residues selected from the group consisting of Gln61 to Arg73 (amino acid sequence shown in SEQ ID NO: 10) of the Switch 2 region. Besides, from a similar viewpoint, the binding molecule of the present disclosure can be preferably a molecule that interacts with one or more (1, 2, 3, 4, 5, or 6) amino acid residues selected from the group consisting of Gln61, Glu62, Arg68, Asp69, Met72, and Arg73 of the Switch 2 region. Furthermore, from a similar viewpoint, the binding molecule of the present disclosure can be preferably a molecule that interacts with one or more (1, 2, 3, 4, or 5) amino acid residues selected from the group consisting of Gln61, Glu62, Arg68, Asp69, and Met72 of the Switch 2 region. In addition, from a similar viewpoint, the binding molecule of the present disclosure can be more preferably a molecule that interacts with Gln61 of the Switch 2 region. Particularly when the binding molecule of the present disclosure is a peptide compound, the peptide compound preferably contains one or more amino acid residues that interacts with these amino acid residues of the Switch 2 region of the mutant RAS (G12D). Besides, from the viewpoint of easily causing the interaction with the Switch 2 region, the amino acid residue is preferably an α amino acid residue.

More specific examples of such a binding molecule include, but are not limited to, a molecule having one or more (for example, 2, 3, or 4) amino acid residues that interacts with one or more (for example, 2, 3, or 4) Gln61, Glu62, Arg68, and Met72 of the Switch 2 region, a molecule having one or more (for example, 2 or 3) amino acid residues that interacts with one or more (for example, 2 or 3) Gln61, Arg68, and Met72, a molecule having one or more (for example, 2, 3, 4, or 5) amino acid residues that interacts with one or more (for example, 2, 3, 4, or 5) Gln61, Glu62, Arg68, Asp69, and Met72, and a molecule having one or more (for example, 2, 3, 4, 5, or 6) amino acid residues that interacts with one or more (for example, 2, 3, 4, 5, or 6) Gln61, Glu62, Arg68, Asp69, Met72, and Arg73.

In one nonrestrictive aspect, an interaction energy between the binding molecule of the present disclosure and Gln61 of the Switch 2 region has a large negative value, and hence it is understood that the binding molecule and the Switch 2 region strongly interact with each other. In one nonrestrictive aspect, when the binding molecule of the present disclosure is a peptide compound having an amino acid residue that interacts with Gln61 of the Switch 2 region, a sum of interaction energies between all atoms of the peptide compound and all atoms of the Gln61 amino acid residue (both of atoms contained in a main chain, and atoms contained in a side chain) can be, but is not limited to, for example, -7.0 kcal/mol or less, preferably -10 kcal/mol or less, more preferably -11.0 kcal/mol or less, further preferably -13.0 kcal/mol or less, further preferably -15.0 kcal/mol or less, further preferably -16.0 kcal/mol or less, further preferably - 18.0 kcal/mol or less, further preferably -19.0 kcal/mol or less, and particularly preferably -20.0 kcal/mol or less from the viewpoint of easily causing the interaction with the Switch 2 region. The interaction energy of the present disclosure may be either of one calculated with coordinates of a hydrogen atom automatically optimized with a program used for the energy calculation, and one calculated without optimizing coordinates of a hydrogen atom, and is preferably one calculated with the coordinates of a hydrogen atom optimized.

From the viewpoint of easily causing the interaction with the Switch 2 region, the interaction between the binding molecule and the Switch 2 region of the mutant RAS (G12D) is preferably an interaction not via another molecule.

### Interaction between Binding Molecule and α3 Helix Region

In one nonrestrictive aspect, the binding molecule of the present disclosure interacts with the α3 helix region of the mutant RAS (G12D). The α3 helix region of the mutant RAS (G12D) of the present disclosure contains Thr87 to Lys104 of the mutant RAS (G12D) shown in SEQ ID NO: 8. The α3 helix region of the present disclosure preferably contains Thr87 to Lys104 of the mutant RAS (G12D). Accordingly, the binding molecule of the present disclosure can be a binding molecule capable of interacting with amino acid residues including Thr87 to Lys104 of the α3 helix region of the mutant RAS (G12D).

In one nonrestrictive aspect, from the viewpoint of easily causing the interaction between the binding molecule and the α3 helix region, the binding molecule of the present disclosure can be a molecule that interacts with any one or more (1, 2, 3, 4, 5, 6, 7, 8, 9, 10, 11, 12, 13, 14, 15, 16, 17, or 18) amino acid residues selected from the group consisting of Thr87 to Lys104 of the α3 helix region of the mutant RAS (G12D). Besides, the binding molecule of the present disclosure can be preferably a molecule that interacts with any one or more (1, 2, 3, 4, 5, 6, 7, 8, or 9) amino acid residues selected from the group consisting of His95 to Val103 (SEQ ID NO: 9) of the α3 helix region. The binding molecule of the present disclosure more preferably can be a molecule that interacts with any one or more (1, 2, 3, 4, or 5) amino acid residues selected from the group consisting of His95, Tyr96, Gln99, Arg102, and Val103 of the α3 helix region, and particularly preferably can be a molecule that interacts with Gln99 of the α3 helix region.

In particular, when the binding molecule of the present disclosure is a peptide compound, the peptide compound contains one or more amino acid residues that interacts with these amino acid residues of the α3 helix region of the mutant RAS (G12D). From the viewpoint of easily causing the interaction between the binding molecule and the α3 helix region, the amino acid residue is preferably an α amino acid residue.

More specific examples of such a binding molecule include, but are not limited to, a molecule having one or more (for example, 2, 3, 4, or 5) amino acid residues that interacts with one or more (for example, 2, 3, 4, or 5) His95, Tyr96, Gln99, Arg102, and Val103 of the α3 helix region, a molecule having one or more (for example, 2, 3, or 4) amino acid residues that interacts with one or more (for example, 2, 3, or 4) His95, Tyr96, Gln99, and Val 103, and a molecule having one or more (for example, 2, 3, 4, or 5) amino acid residues that interacts with one or more example, 2, 3, 4, or 5) His95, Tyr96, Gln99, Arg102, and Val 103.

From the viewpoint of easily causing the interaction with the α3 helix region, the interaction between the binding molecule and the α3 helix region of the mutant RAS (G12D) is preferably an interaction not via another molecule.

### Interaction between Switch 2 Region and α3 Helix Region

In one nonrestrictive aspect, when the binding molecule of the present disclosure and the mutant RAS (G12D) together form a complex, the Switch 2 region and the α3 helix region are close to each other in the mutant RAS (G12D). Specifically, these are close to each other to have a distance between an α carbon atom of Asp69 present in the Switch 2 region of the mutant RAS (G12D) and an α carbon atom of Gln99 present in the α3 helix region of about 15.0 angstroms (Å) or less, preferably about 14.0 Å or less, more preferably about 13.0 Å or less, further preferably about 12.0 Å or less, and particularly preferably about 11.0 Å or less. When the binding molecule of the present disclosure and the mutant RAS (G12D) together form a complex, an α carbon atom of Asp69 present in the Switch 2 region and an α carbon atom of Gln99 present in the α3 helix region are close to each other to have such a distance therebetween in the mutant RAS (G12D), which probably easily causes intramolecular interaction between the amino acid residue present in the Switch 2 region and Asp12, and corresponds to one of features of the binding molecule of the present disclosure. Accordingly, the binding molecule of the present disclosure can be a molecule capable of making an α carbon atom of Asp69 present in the Switch 2 region and an α carbon atom of Gln99 present in the α3 helix region to be close to each other to have a distance therebetween of 15.0 angstroms (Å) or less, 14.0 Å or less, 13.0 Å or less, 12.0 Å or less, or 11.0 Å or less, in the mutant RAS (G12D).

### Pre-orientation

In one nonrestrictive aspect, when the binding molecule of the present disclosure and the mutant RAS (G12D) together form a complex, specifically, when the binding molecule of the present disclosure is interacting with the Switch 2 region of the mutant RAS (G12D), intramolecular interaction is caused between an amino acid residue present in the Switch 2 region and Asp12 in the mutant RAS (G12D). In the present disclosure, such an interaction is designated as "pre-orientation". In one nonrestrictive aspect, from the viewpoint of further improving binding selectivity of the binding molecule for the mutant RAS (G12D) over wild type RAS, the intramolecular interaction of the present disclosure between an amino acid residue present in the Switch 2 region and Asp12 is preferably an interaction not via another molecule. In one nonrestrictive aspect, from a similar viewpoint, the amino acid residue present in the Switch 2 region, which interacts with Asp12 is preferably any one or more (1, 2, 3, or 4) selected from the group consisting of Ala59, Gly60, Gln61, and Glu62. From a similar viewpoint, non-hydrogen atoms contained in at least 1, at least 2, at least 3, or at least 4 of these amino acid residues are present preferably within a distance of about 4.5 angstroms (Å) or less from one or both of two oxygen atoms of a carboxy group of Asp12. An example of the amino acid residues having such non-hydrogen atoms includes a combination of Ala59, Gly60, and Gln61.

From the viewpoint of further improving the binding selectivity of the binding molecule for the mutant RAS (G12D) over wild type RAS, when there are, in Ala59, Gly60, Gln61, and Glu62 of the mutant RAS (G12D), one or more amino acid residues having one or more non-hydrogen atoms present within a distance of about 4.5 angstroms (Å) or less from either of two oxygen atoms of a carboxy group in the side chain of Asp12 in the complex of the binding molecule of the present disclosure and the mutant RAS (G12D), a sum of interaction energies between all atoms contained in the main chains of the one or plurality of amino acid residues and all atoms of Asp12 can be, but is not limited to, for example, -0.5 kcal/mol or less, -1.0 kcal/mol or less, -3.0 kcal/mol or less, -5.0 kcal/mol or less, -7.5 kcal/mol or less, -10.5 kcal/mol or less, - 13.0 kcal/mol or less, -15.0 kcal/mol or less, -15.5 kcal/mol or less, -16.0 kcal/mol or less, -16.5 kcal/mol or less, -17.0 kcal/mol or less, -17.5 kcal/mol or less, or -18.0 kcal/mol or less. The sum of the interaction energies may be either of one calculated with coordinates of a hydrogen atom automatically optimized with a program used for the energy calculation, and one calculated without optimizing coordinates of a hydrogen atom, and is preferably one calculated with the coordinates of a hydrogen atom optimized.

Besides, from the viewpoint of further improving the binding selectivity of the binding molecule for the mutant RAS (G12D) over wild type RAS, when there are, in Ala59, Gly60, Gln61, and Glu62 of the mutant RAS (G12D), one or more amino acid residues having one or more non-hydrogen atoms present within a distance of about 4.5 angstroms (Å) or less from either of two oxygen atoms of a carboxy group of the side chain of Asp12 in the complex of the binding molecule of the present disclosure and the mutant RAS (G12D), a sum of interaction energies between all atoms contained in the one or plurality of amino acid residues (for example, all atoms contained in a main chain and all atoms contained in a side chain) and all atoms of Asp12 can be, but is not limited to, for example, -0.5 kcal/mol or less, -1.0 kcal/mol or less, -3.0 kcal/mol or less, -5.0 kcal/mol or less, -7.5 kcal/mol or less, -10.5 kcal/mol or less, -13.0 kcal/mol or less, -14.0 kcal/mol or less, -15.0 kcal/mol or less, -15.5 kcal/mol or less, -16.0 kcal/mol or less, -16.5 kcal/mol or less, -17.0 kcal/mol or less, -17.5 kcal/mol or less, or -18.0 kcal/mol or less in the complex of the binding molecule of the present disclosure and the mutant RAS (G12D). The sum of the interaction energies may be either of one calculated with coordinates of a hydrogen atom automatically optimized with a program used for the energy calculation, and one calculated without optimizing coordinates of a hydrogen atom, and is preferably one calculated with the coordinates of a hydrogen atom optimized.

In one nonrestrictive aspect, it can be determined through the following stages whether or not "pre-orientation" is being caused, and how strong the "pre-orientation" being caused is:
(I) A stage of determining amino acid residues, among Ala59, Gly60, Gln61, and Glu62 of the mutant RAS (G12D), having one or more non-hydrogen atoms present within a distance of 4.5 angstroms (Å) or less from either of two oxygen atoms of a carboxy group of a side chain of Asp12. When the amino acid residues determined at this stage are present, it is found that the "pre-orientation" is being caused.
(II) A stage of calculating the sum of interaction energies between all atoms contained in a main chain, or a main chain and a side chain, of the one or plurality of amino acid residues determined at the stage (I) and all atoms of Asp12; and
(III) (i) when all the atoms contained in the main chains of the one or plurality of amino acid residues determined at the stage (I) are used for obtaining the interaction energies, a stage of determining that strong pre-orientation is being caused when the sum of the interaction energies is -0.5 kcal/mol or less, -1.0 kcal/mol or less, -3.0 kcal/mol or less, -5.0 kcal/mol or less, -7.5 kcal/mol or less, -10.5 kcal/mol or less, -13.0 kcal/mol or less, -15.0 kcal/mol or less, -15.5 kcal/mol or less, -16.0 kcal/mol or less, -16.5 kcal/mol or less, -17.0 kcal/mol or less, -17.5 kcal/mol or less, or -18.0 kcal/mol or less, or (ii) when all the atoms contained in the main chains and the side chains of the one or plurality of amino acid residues determined at the stage (I) are used for obtaining the interaction energies, a stage of determining that strong pre-orientation is being caused when the sum of the interaction energies is -0.5 kcal/mol or less, -1.0 kcal/mol or less, -3.0 kcal/mol or less, -5.0 kcal/mol or less, -7.5 kcal/mol or less, -10.5 kcal/mol or less, - 13.0 kcal/mol or less, -14.0 kcal/mol or less, -15.0 kcal/mol or less, -15.5 kcal/mol or less, -16.0 kcal/mol or less, -16.5 kcal/mol or less, -17.0 kcal/mol or less, -17.5 kcal/mol or less, or -18.0 kcal/mol or less.

In other words, for determining amino acid residues to be used for the calculation of the interaction energies at the stage (I), non-hydrogen atoms contained in Ala59, Gly60, Gln61, and Glu62 are used, and on the other hand, for the calculation itself of the sum of the interaction energies at the stage (II), all the atoms contained in the main chains, or the main chains and the side chains, are used, and hydrogen atoms are included therein.

The binding molecule of the present disclosure can be a molecule capable of causing pre-orientation in the mutant RAS (G12D). Besides, the binding molecule of the present disclosure can have a structure capable of causing pre-orientation in the mutant RAS (G12D). In the present disclosure, the structure capable of causing pre-orientation in the mutant RAS (G12D) is not especially limited, and an example includes the structure (2) of *-(linear or branched C₁-C₈ alkylene)-Y. Here, Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and * means a binding point.

Examples of a more preferable aspect of the structure (2) include *-(CH₂)-Y and *-(CH₂)₂-Y, wherein Y is C₃-C₈ alkyl, C₃-C₈ cycloalkyl, C₃-C₈ alkenyl, or C₆-C₁₀ aryl optionally substituted by C₁-C₆ alkoxy.

Particularly preferable examples of the structure (2) include the following:

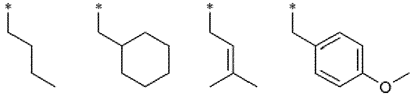

The binding molecule of the present disclosure can contain the following structure (3) as the structure capable of causing pre-orientation in the mutant RAS (G12D): 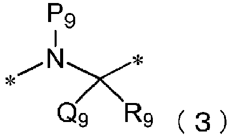
wherein R₉ is C₁-C₆ alkyl, optionally substituted C₁-C₆ alkoxy C₁-C₆ alkyl, or optionally substituted C₆-C₁₀ aryl, or
R₉ forms a 4- to 7-membered saturated heterocycle together with carbon atoms to which P₉ and R₉ are bound, and a nitrogen atom to which P₉ is bound,
P₉ is hydrogen or C₁-C₃ alkyl unless R₉ and P₉ form a 4- to 7-membered saturated heterocycle,
Q₉ is hydrogen or C₁-C₆ alkyl, and
* means a binding point.

In one aspect, R₉ is preferably C₁-C₄ alkyl, phenyl C₁-C₂ alkyl, or C₁-C₃ alkoxy C₁-C₂ alkyl. When R₉ is C₁-C₄ alkyl, for example, methyl, Q₉ is preferably C₁-C₄ alkyl, and more preferably methyl. When R₉ is phenyl C₁-C₂ alkyl, or C₁-C₃ alkoxy C₁-C₂ alkyl, Q₉ is preferably hydrogen. P₉ is preferably methyl.

In one aspect, when R₉ and P₉ form a 4- to 7-membered saturated heterocycle, the 4- to 7-membered saturated heterocycle is preferably an azetidine ring, a pyrrolidine ring, or a piperidine ring, and more preferably a pyrrolidine ring. When R₉ and P₉ form a 4- to 7-membered saturated heterocycle, Q₉ is preferably C₁-C₆ alkyl, and more preferably methyl.

Particularly preferable examples of the structure (3) include the following:

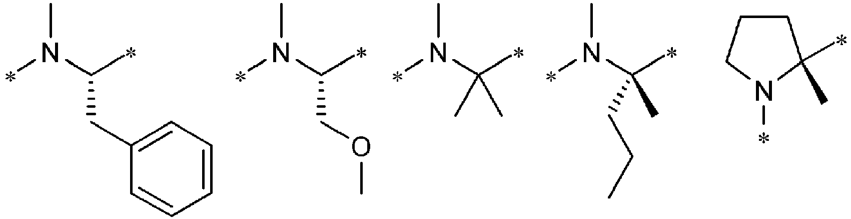

In the binding molecule of the present disclosure, from the viewpoint of easily simultaneously causing the direct interaction and the pre-orientation, an atom farthest from the binding point of the structure (1) among atoms contained in X of the structure (1), and an atom farthest from the binding point of the structure (2) among atoms contained in Y of the structure (2) are preferably bound to each other via 20 or less (for example, 19, 18, 17, 16, 15, 14, or less) atoms.

In the binding molecule of the present disclosure, from the viewpoint of easily simultaneously causing the direct interaction and the pre-orientation, unless R₉ and P₉ form a 4-to 7-membered saturated heterocycle, an atom farthest from the binding point of the structure (1) among atoms contained in X of the structure (1), and an atom farthest from a carbon atom bound to Q₉ and R₉ in the structure (3) among atoms contained in R₉ of the structure (3) are preferably bound to each other via 25 or less (for example, 24, 23, 22, 21, 20, 19, 18, 17, 16, or less) atoms.

The "interaction" used in the present disclosure encompasses a state of an interaction between the mutant RAS (G12D) and the binding molecule with an interatomic distance between an atom of a side chain or a main chain of an amino acid residue contained in the mutant RAS (G12D) and an atom contained in the binding molecule of the present disclosure capable of providing at least one, preferably two, more preferably three, and particularly preferably four of the following features (1) to (4), or with an interatomic energy capable of providing at least one, preferably two, more preferably three, and particularly preferably four of the following features (1) to (4):
(1) the binding molecule interacts with Asp12 of the mutant RAS (G12D);
(2) the binding molecule interacts with the Switch 2 region of the mutant RAS (G12D), and an amino acid residue present in the Switch 2 region intermolecularly interacts with Asp12 in the mutant RAS (G12D);
(3) the binding molecule interacts with the α3 helix region of the mutant RAS (G12D), and
(4) when an amino acid residue present in the Switch 2 region is intermolecularly interacting with Asp12, a distance between an α carbon atom of Asp69 present in the Switch 2 region and an α carbon atom of Gln99 present in the α3 helix region is 15.0 angstroms (Å) or less in the mutant RAS (G12D).

It is noted that it can be determined by a method well-known to those skilled in the art which amino acid residue out of amino acid residues contained in the mutant RAS (G12D) is interacting with the binding molecule. For example, it can be determined which amino acid residue out of amino acid residues contained in the mutant RAS (G12D) is interacting with the binding molecule by causing a software program used in molecular modeling or molecular simulation, such as Discovery studio 2020 Client, MOE (Molecular Operating Environment), or Maestro, to read structural information of a complex of the binding molecule of the present disclosure and the mutant RAS (G12D) to use a function provided in the software program (for example, Display receptor-ligand interactions Function in using Discovery studio 2020 Client). Details of conditions and criteria employed for the determination of the presence of the interaction by the used software can be checked based on instructions, specifications or the like attached to the software (for example, in using Discovery studio 2020 Client, the details of conditions and criteria employed for the determination of the presence of interaction can be checked by opening a web page of specifications using a help button, selecting "Receptor-Ligand Interactions Tools", further selecting "Theory-Receptor-Ligand Interactions", and subsequently selecting "Non-bond Interactions").

The "interaction" used in the present disclosure means non-covalent binding interaction, such as electrostatic interaction (including ionic bond, hydrogen bond, and dipole interaction), or van der Waals interaction (including hydrophobic interaction). The interaction of the present disclosure may be one via another molecule such as a water molecule, or one not via another molecule such as a water molecule, and is preferably an interaction not via another molecule such as a water molecule.

In the present disclosure, it can be determined whether or not a non-hydrogen atom contained in a specific amino acid residue of the mutant RAS (G12D) and a non-hydrogen atom contained in a molecule that interacts therewith are interacting with each other in accordance with an interatomic distance between these non-hydrogen atoms (in a bond via another molecule such as a water molecule, an interatomic distance between these non-hydrogen atoms with the another molecule ignored). For example, when the interatomic distance is 4.6 angstroms (Å) or less, it can be determined that these non-hydrogen atoms interact with each other. In some aspects, the interatomic distance between these two non-hydrogen atoms interacting with each other can be, for example, 4.5 Å or less, 4.2 Å or less, 4.0 Å or less, 3.7 Å or less, 3.5 Å or less, 3.2 Å or less, or 3.0 Å or less. Besides, it can be 2.0 Å or more, 2.1 Å or more, or 2.5 Å or more.

If one or both of these non-hydrogen atoms have a charge, however, it can be determined that these non-hydrogen atoms interact with each other when, for example, the interatomic distance is 5.6 Å or less. In this case, in some aspects, the interatomic distance between these two non-hydrogen atoms interacting with each other can be, for example, 5.5 Å or less, 5.0 Å or less, 4.5 Å or less, 4.2 Å or less, 4.0 Å or less, 3.7 Å or less, 3.5 Å or less, or 3.2 Å or less.

In the present disclosure, the interatomic distance can be measured, for example, through analysis of a three-dimensional structure of a complex of the mutant RAS (G12D) and the binding molecule of the present disclosure. Specifically, a crystal of the complex of the mutant RAS (G12D) and the binding molecule of the present disclosure is prepared. The crystal is subjected to X-ray diffraction to obtain X-ray diffraction intensity data of a space group, a unit cell and the like. The thus obtained X-ray diffraction intensity data is applied to a program, well-known to those skilled in the art, for determining an initial structure or a refined structure, such as Coot (Emsley, P. et al., 2010), Phenix (Adams, P. D. et al., 2010), Phaser (J. Appl. Cryst. 40: 658-674 (2007)), Refmac5 (Acta Cryst. D67: 355-467 (2011), and ARP/wARP (Cohen, S. X. et al., 2008), and thus, the three-dimensional structure of the complex of the mutant RAS (G12D) and the binding molecule of the present disclosure can be determined.

When the three-dimensional structure of the complex of the mutant RAS (G12D) and the binding molecule of the present disclosure can be determined, the interatomic distance can be measured by a method well-known to those skilled in the art. For example, the interatomic distance can be measured by causing a software program used in molecular modeling or molecular simulation, such as Discovery studio 2020 Client, MOE (Molecular Operating Environment), or Maestro, to read structural information of the complex of the binding molecule of the present disclosure and the mutant RAS (G12D) to use a function provided in the software program (for example, Distance Monitor Function in using Discovery studio 2020 Client). Details of conditions and criteria employed for the determination of the presence of the interaction by the used software can be checked based on instructions, specifications or the like attached to the software (for example, in using Discovery studio 2020 Client, the details of conditions and criteria employed for the determination of the presence of the interaction can be checked by opening a web page of specifications using a help button, selecting "Receptor-Ligand Interactions Tools", further selecting "Theory-Receptor-Ligand Interactions", and subsequently selecting "Non-bond Interactions").

The crystal of the complex of the mutant RAS (G12D) and the binding molecule of the present disclosure can be obtained by a method well-known to those skilled in the art. For example, a solution containing the binding molecule of the present disclosure and a solution containing the mutant RAS (G12D) are mixed with each other to obtain the complex of the mutant RAS (G12D) and the binding molecule of the present disclosure. The thus obtained complex is subjected to a crystallization method well-known to those skilled in the art, such as a vapor diffusion method, a batch method (a bulk-batch method or a micro-batch method), a dialysis method, or a counter-diffusion method, and thus, the crystal of the complex of the mutant RAS (G12D) and the binding molecule of the present disclosure can be prepared. As the vapor diffusion method, a sitting drop method, a hanging drop method, and a sandwich drop method are known.

It is noted that the mutant RAS (G12D) can be also obtained by a method known to those skilled in the art. For example, the mutant RAS (G12D) can be prepared by employing a recombinant polypeptide expression method using a cell, but the method is not limited thereto. In one aspect, a nucleic acid encoding the mutant RAS (G12D) of the present disclosure is inserted into an appropriate expression vector, the vector is introduced into an appropriate cell to culture a transformed cell, and the thus expressed and modified protein is isolated, purified and cultured. Such a protein can be expressed in the form of a fused protein with another protein for purposes of easing purification and the like. For example, a method in which *E.coli* is used as a host to prepare it as a fused protein with a maltose binding protein (vectors of pMAL series available from New England BioLabs, USA), a method in which it is prepared as a fused protein with glutathione-S-transferase (GST) (vectors of pGEX series available from Amersham Pharmacia Biotech), a method in which it is prepared with a histidine tag added (pET series of Novagen), a method in which it is prepared with a HAT tag added, and the like can be employed. The host cell is not especially limited as long as it is a cell suitable for expression of a recombinant protein, and in addition to *E.coli* mentioned above, yeasts, various animal and plant cells, insect cells and the like can be used, for example. For introducing a vector into the host cell, various methods known to those skilled in the art can be employed. For example, for introduction into *E.coli,* an introduction method using a calcium ion (Mandel, M., Higa, A. (1970) Journal of Molecular Biology, 53, 158-162, Hanahan, D. (1983) Journal of Molecular Biology, 166, 557-580) can be employed. The protein expressed in the host cell can be purified and collected from the host cell, or a cell culture or a culture supernatant thereof by a method known to those skilled in the art. When the protein is expressed in the form of a fused protein with the maltose binding protein, the HAT tag or the like, affinity purification or gel filtration chromatography (size exclusion chromatography, SEC) purification can be easily performed. For affinity chromatography purification and SEC purification, AKTAxpress(TM) apparatus (GE Healthcare), NGC(TM) chromatography system (Bio-Rad), BioLogic DuoFlow(TM) chromatography system (Bio-Rad), or the like can be used.

The interatomic energy can be also measured by a method well-known to those skilled in the art. For example, it can be easily calculated by causing a molecular simulation program well-known to those skilled in the art, such as Discovery studio 2020 Client, MOE (Molecular Operating Environment), or Maestro, to read a three-dimensional structure of a substance to be measured, and selecting a force field (such as Amber, or CHARM) to be used for the calculation, and an atom corresponding to the target of energy calculation in accordance with instruction of the program. For example, in using Discovery studio 2020 Client, the interatomic energy can be calculated by using Calculate Interaction Energy Function thereof. In this case, the calculation may be performed with coordinates of a hydrogen atom automatically optimized with the program used for the energy calculation. Herein, if there is a difference between an interatomic energy calculated with coordinates of a hydrogen atom optimized, and an interatomic energy calculated without optimizing coordinates of a hydrogen atom, the interatomic energy calculated with the coordinates of a hydrogen atom optimized is employed as the interatomic energy.

In one nonrestrictive aspect, in the complex of the binding molecule of the present disclosure and the mutant RAS (G12D), the binding molecule of the present disclosure interacts with Asp 12 of the mutant RAS (G12D) (direct interaction).

In one nonrestrictive aspect, in the complex of the binding molecule of the present disclosure and the mutant RAS (G12D), the binding molecule of the present disclosure and the Switch 2 region of the mutant RAS (G12D) interact with each other. Therefore, an atom contained in the Switch 2 region of the mutant RAS (G12D) (specifically, an α carbon atom of Asp69) and an atom contained in the α3 helix region (specifically, an α carbon atom of amino acid residue Gln99) come close to each other. This probably relates to intramolecular interaction between an amino acid residue contained in the Switch 2 region of the mutant RAS (G12D) (for example, Gln61) and Asp12 (pre-orientation).

Although not constrained by a specific theory, it is presumed that formation of the complex of the binding molecule of the present disclosure and the mutant RAS (G12D) in such a form relates to contribution to the large binding activity and binding selectivity of the binding molecule of the present disclosure for the mutant RAS (G12D).

In one nonrestrictive aspect, the molecular weight of the binding molecule of the present disclosure is, from the viewpoint of further improving the binding selectivity of the binding molecule for the mutant RAS (G12D) over wild type RAS, preferably 5,000 or less, more preferably 3,000 or less, further preferably 2,500 or less, further preferably 2,300 or less, and further preferably 2,000 or less, and preferably 500 or more, more preferably 800 or more, and further preferably 1,000 or more. For example, the molecular weight of a peptide compound can be preferably 500 or more and 5,000 or less, more preferably 800 or more and 3,000 or less, and further preferably 1,000 or more and 2,000 or less.

In one nonrestrictive aspect, as for the ClogP of the binding molecule of the present disclosure, the CLogP of a peptide compound is preferably 25 or less, more preferably 22 or less, further preferably 20 or less, further preferably 18 or less, further preferably 16 or less, and further preferably 15 or less, and preferably 5 or more, more preferably 10 or more, and further preferably 12 or more. The CLogP of a peptide compound can be, for example, preferably 5 or more and 25 or less, more preferably 10 or more and 20 or less, and further preferably 12 or more and 18 or less. The ClogP used herein refers to a partition coefficient calculated with a computer, and can be calculated with Daylight Version 4.9 of Daylight Chemical Information Systems, Inc.

In one nonrestrictive aspect, when the binding molecule of the present disclosure is a peptide compound, ClogP/total aa of the peptide compound is preferably 1.0 or more, more preferably 1.1 or more, further preferably 1.2 or more, preferable examples include 1.8 or less, 1.7 or less, 1.6 or less, and 1.5 or less, and examples include 1.0 or more and 1.8 or less, 1.0 or more and 1.7 or less, 1.1 or more and 1.6 or less, and 1.1 or more and 1.5 or less. The term "total aa" (sometimes expressed as "total AA") used herein means the number of amino acids contained in the peptide compound.

In one nonrestrictive aspect, the binding molecule of the present disclosure encompasses, but is not limited to, a low molecular weight compound, a peptide compound, a polypeptide, a protein, an antibody, a carbohydrate, a nucleic acid, and derivatives thereof. In one nonrestrictive aspect, the binding molecule of the present disclosure encompasses salts thereof, or solvates thereof. The binding molecule of the present disclosure may be a peptide compound, and the peptide compound may be a cyclic peptide compound. In some aspects, the peptide compound of the present disclosure may contain one or more natural amino acid residues and non-natural amino acid residues. A ratio between these amino acids is not especially limited. A non-natural amino acid residue of the present disclosure may be a N-substituted amino acid residue. In other words, the peptide compound of the present disclosure can contain one or more N-substituted amino acid residues. The N-substituted amino acid may be preferably N-alkylamino acid, and more preferably N-methylamino acid. Alternatively, the N-substituted amino acid may be preferably one in which a carbon chain bound to a N atom and a carbon atom in the α position form a ring as in proline.

In some aspects, the total number of amino acid residues contained in the peptide compound of the present disclosure may be, from the viewpoint of further improving the binding selectivity of the binding molecule for the mutant RAS (G12D) over wild type RAS, preferably 4 or more, more preferably 5 or more, further preferably 6 or more, further preferably 8 or more, further preferably 9 or more, and further preferably 10 or more, and preferably 30 or less, more preferably 20 or less, further preferably 15 or less, further preferably 14 or less, further preferably 13 or less, further preferably 12 or less, and most preferably 11. In one aspect, the total number of amino acids contained in a ring portion of the cyclic peptide compound of the present disclosure may be preferably 5 or more, more preferably 7 or more, further preferably 8 or more, further preferably 9 or more, and further preferably 10 or more, and preferably 15 or less, more preferably 13 or less, further preferably 12 or less, and most preferably 11.

In some aspects, when the cyclic peptide compound of the present disclosure has a straight chain portion, the number of amino acid residues of the straight chain portion is preferably 0 or more and 17 or less, more preferably 0 or more and 8 or less, further preferably 0 or more and 5 or less, and particularly preferably 0 or more and 3 or less. It is noted, in one nonrestrictive aspect, that the "straight chain portion" herein may contain a natural amino acid residue or a non-natural amino acid residue (including a chemically modified or skeleton transformed amino acid) in some cases.

In one nonrestrictive aspect, the number of non-natural amino acids contained in the peptide compound of the present disclosure is, for example, preferably 3 or more, more preferably 4 or more, 5 or more, or 6 or more, further preferably 7 or more, further preferably 8 or more, and particularly preferably 9 or more, and is preferably 20 or less, 15 or less, 14 or less, 13 or less, 12 or less, or 10 or less. The number of non-natural amino acids contained in the peptide compound of the present disclosure is, for example, 30% or more, 40% or more, 50% or more, 60% or more, 70% or more, or 80% or more of the number of amino acids contained in the peptide compound.

In one nonrestrictive aspect, the number of N-substituted amino acid residues contained in the peptide compound of the present disclosure is, for example, preferably 3 or more, more preferably 4 or more, 5 or more, or 6 or more, and further preferably 7 or more, and preferably 20 or less, 15 or less, 14 or less, 13 or less, 12 or less, 10 or less, 9 or less, or 8 or less. The number of N-substituted amino acids contained in the peptide compound of the present disclosure is, for example, 30% or more, 40% or more, 50% or more, or 60% or more of the number of amino acids contained in the peptide compound.

In one nonrestrictive aspect, the number of non-natural amino acid residues excluding N-substituted amino acid residues contained in the peptide compound of the present disclosure is, for example, preferably 3 or more, more preferably 4 or more, 5 or more, or 6 or more, further preferably 7 or more, and particularly preferably 8 or more, and preferably 20 or less, 15 or less, 14 or less, 13 or less, 12 or less, 10 or less, or 9 or less. The number of N-substituted amino acids contained in the peptide compound of the present disclosure is, for example, 30% or more, 40% or more, 50% or more, 60% or more, 70% or more, or 80% or more of the number of amino acids contained in the peptide compound.

In some aspects, when the binding molecule of the present disclosure is a peptide compound, the peptide compound can have the following structure (4) in which the structure (1) and the structure (2) of the present disclosure are linked to each other via an amide bond: 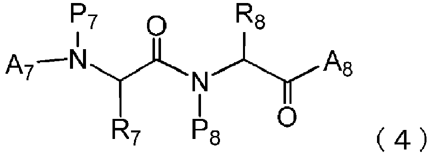
wherein A₇ and A₈ independently are an arbitrary amino acid residue or an arbitrary peptide residue, A₇ and A₈ being optionally linked to each other,
R₇ is -(linear or branched C₁-C₈ alkylene)-X, or -(linear or branched C₁-C₈ alkylene)-(C₆-C₁₀ arylene)-X,
X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4-to 8-membered heterocycle containing 1 to 3 hetero atoms selected from the group consisting of N, O, and S), C₁-C₃ alkyl, C₁-C₃ haloalkyl, -C=CH, or optionally substituted C₃-C₈ cycloalkyl, and if R₇ is -(linear or branched C₁-C₈ alkylene)-X, X is not C₁-C₃ alkyl,
P₇ is hydrogen or C₁-C₃ alkyl,
R₈ is -(linear or branched C₁-C₈ alkylene)-Y,
Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and
P₈ is hydrogen or C₁-C₃ alkyl.

An example of a preferable aspect of R₇ and X includes an aspect in which R₇ is benzyl or phenethyl, and X is ethyl in the 3-position of a phenyl group, cyclopropyl, Cl, Br, I, or -C---CH, and more preferably includes an aspect in which R₇ is benzyl or phenethyl, and X is I in the 3-position of a phenyl group, or -C=CH. In either aspect, P₇ is preferably hydrogen or methyl.

R₈ is preferably -(CH₂)-Y, or -(CH₂)₂-Y, and Y is preferably C₃-C₈ alkyl, C₃-C₈ cycloalkyl, C₃-C₈ alkenyl, or C₆-C₁₀ aryl optionally substituted by C₁-C₆ alkoxy. P₈ is preferably hydrogen or methyl.

When A₇ and/or A₈ is an amino acid residue, the amino acid residue can be an arbitrary natural or non-natural amino acid residue.

When A₇ and/or A₈ is a peptide residue, the peptide residue contains arbitrary type and number of natural and/or non-natural amino acid residues. The number of amino acid residues contained in the peptide residue is preferably 2 to 13, and more preferably 2 to 9.

In one aspect, A₇ and A₈ can be linked to each other. A peptide residue formed through linkage of A₇ and A₈ can contain arbitrary number and type of natural and/or non-natural amino acid residues. The peptide residue contains preferably 5 to 15 amino acid residues, and more preferably 8 to 9 amino acid residues.

Particularly preferable examples of the structure (4) include the following: 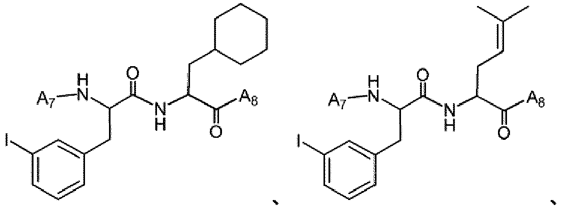 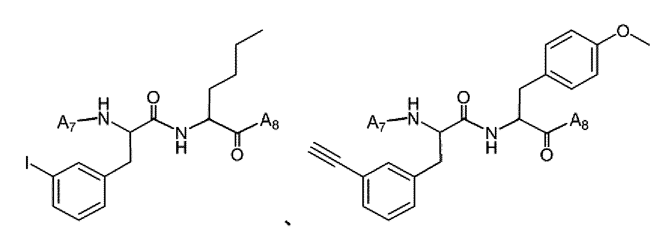 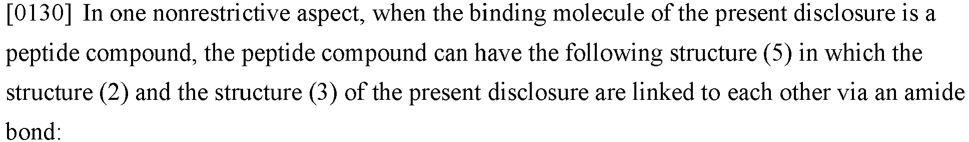 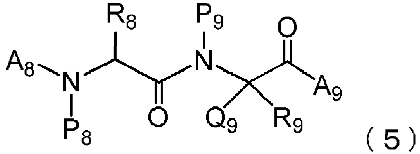
wherein A₈ and A₉ independently are an arbitrary amino acid residue, or an arbitrary peptide residue, A₈ and A₉ being optionally linked to each other,
R₈ is -(linear or branched C₁-C₈ alkylene)-Y,
Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and
P₈ is hydrogen or C₁-C₃ alkyl,
R₉ is C₁-C₆ alkyl, optionally substituted C₁-C₆ alkoxy C₁-C₆ alkyl, or optionally substituted C₆-C₁₀ aryl, or
R₉ forms a 4- to 7-membered saturated heterocycle together with carbon atoms to which P₉ and R₉ are bound, and a nitrogen atom to which P₉ is bound,
P₉ is hydrogen or C₁-C₃ alkyl unless R₉ and P₉ form a 4- to 7-membered saturated heterocycle, and
Q₉ is hydrogen or C₁-C₆ alkyl.

R₈ is preferably -(CH₂)-Y, or -(CH₂)₂-Y, and Y is preferably C₃-C₈ alkyl, C₃-C₈ cycloalkyl, C₃-C₈ alkenyl, or C₆-C₁₀ aryl optionally substituted by C₁-C₆ alkoxy. P₈ is preferably hydrogen or methyl.

R₉ is preferably C₁-C₄ alkyl, phenyl C₁-C₂ alkyl, or C₁-C₃ alkoxy C₁-C₂ alkyl. When R₉ is C₁-C₄ alkyl, for example, methyl, Q₉ is preferably C₁-C₄ alkyl, and more preferably methyl. When R₉ is phenyl C₁-C₂ alkyl, or C₁-C₃ alkoxy C₁-C₂ alkyl, Q₉ is preferably hydrogen. P₉ is preferably methyl.

When R₉ and P₉ form a 4- to 7-membered saturated heterocycle, the 4- to 7-membered saturated heterocycle is preferably an azetidine ring, a pyrrolidine ring, or a piperidine ring, and more preferably a pyrrolidine ring. When R₉ and P₉ form a 4- to 7-membered saturated heterocycle, Q₉ is preferably C₁-C₆ alkyl, and more preferably methyl.

When A₈ and/or A₉ is an amino acid residue, the amino acid residue can be an arbitrary natural or non-natural amino acid residue.

When A₈ and/or A₉ is a peptide residue, the peptide residue contains arbitrary type and number of natural and/or non-natural amino acid residues. The number of amino acid residues contained in the peptide residue is preferably 2 to 13, and more preferably 2 to 9.

In one aspect, A₈ and A₉ can be linked to each other. A peptide residue formed through linkage of A₈ and A₉ can contain arbitrary number and type of natural and/or non-natural amino acid residues. The peptide residue contains preferably 5 to 15 amino acid residues, and more preferably 8 or 9 amino acid residues.

Particularly preferable examples of the structure (5) include the following:

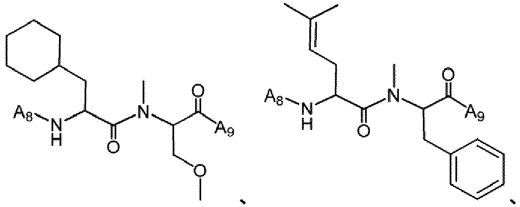

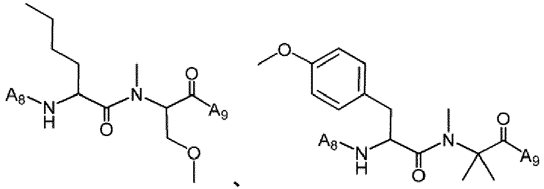

In another nonrestrictive aspect, when the binding molecule of the present disclosure is a peptide compound, the peptide compound can have the following structure (6) in which the structures (1) to (3) of the present disclosure are linked to one another via an amide bond: 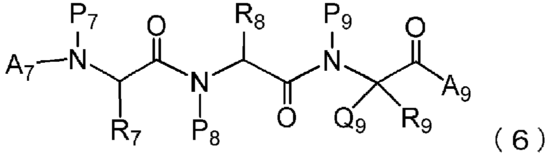
wherein A₇ and A₉ independently are an arbitrary amino acid residue, or an arbitrary peptide residue, A₇ and A₉ being optionally linked to each other,
R₇ is -(linear or branched Ci-Cs alkylene)-X, or -(linear or branched C₁-C₈ alkylene)-(C₆-C₁₀ arylene)-X,
X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4-to 8-membered heterocycle containing 1 to 3 hetero atoms selected from the group consisting of N, O, and S), C₁-C₃ alkyl, C₁-C₃ haloalkyl, -C≡CH, or optionally substituted C₃-C₈ cycloalkyl, and if R₇ is -(linear or branched C₁-C₈ alkylene)-X, X is not C₁-C₃ alkyl,
P₇ is hydrogen or C₁-C₃ alkyl,
R₈ is -(linear or branched C₁-C₈ alkylene)-Y,
Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl,
P₈ is hydrogen or C₁-C₃ alkyl,
R₉ is C₁-C₆ alkyl, optionally substituted C₁-C₆ alkoxy C₁-C₆ alkyl, or optionally substituted C₆-C₁₀ aryl, or
R₉ forms a 4- to 7-membered saturated heterocycle together with carbon atoms to which P₉ and R₉ are bound, and a nitrogen atom to which P₉ is bound,
P₉ is hydrogen or C₁-C₃ alkyl unless R₉ and P₉ form a 4- to 7-membered saturated heterocycle, and
Q₉ is hydrogen or C₁-C₆ alkyl.

An example of a preferable aspect of R₇ and X includes an aspect in which R₇ is benzyl or phenethyl, and X is ethyl in the 3-position of a phenyl group, cyclopropyl, Cl, Br, I, or -C---CH, and more preferably includes an aspect in which R₇ is benzyl or phenethyl, and X is I in the 3-position of a phenyl group, or -C≡CH. In either aspect, P₇ is preferably hydrogen or methyl.

R₈ is preferably -(CH₂)-Y, or -(CH₂)₂-Y, and Y is preferably C₃-C₈ alkyl, C₃-C₈ cycloalkyl, C₃-C₈ alkenyl, or C₆-C₁₀ aryl optionally substituted by C₁-C₆ alkoxy. P₈ is preferably hydrogen or methyl.

R₉ is preferably C₁-C₄ alkyl, phenyl C₁-C₂ alkyl, or C₁-C₃ alkoxy C₁-C₂ alkyl. When R₉ is C₁-C₄ alkyl, for example, methyl, Q₉ is preferably C₁-C₄ alkyl, and more preferably methyl. When R₉ is phenyl C₁-C₂ alkyl, or C₁-C₃ alkoxy C₁-C₂ alkyl, Q₉ is preferably hydrogen. P₉ is preferably methyl.

When R₉ and P₉ form a 4- to 7-membered saturated heterocycle, the 4- to 7-membered saturated heterocycle is preferably an azetidine ring, a pyrrolidine ring, or a piperidine ring, and more preferably a pyrrolidine ring. When R₉ and P₉ form a 4- to 7-membered saturated heterocycle, Q₉ is preferably C₁-C₆ alkyl, and more preferably methyl.

When A₇ and/or A₉ is an amino acid residue, the amino acid residue can be an arbitrary natural or non-natural amino acid residue.

When A₇ and/or A₉ is a peptide residue, the peptide residue contains arbitrary type and number of natural and/or non-natural amino acid residues. The number of amino acid residues contained in the peptide residue is preferably 2 to 13, and more preferably 2 to 9.

In one aspect, A₇ and A₉ can be linked to each other. A peptide residue formed through linkage of A₇ and A₉ can contain arbitrary number and type of natural and/or non-natural amino acid residues. The peptide residue contains preferably 5 to 15 amino acid residues, and more preferably 7 or 8 amino acid residues.

Particularly preferable examples of the structure (6) include the following:

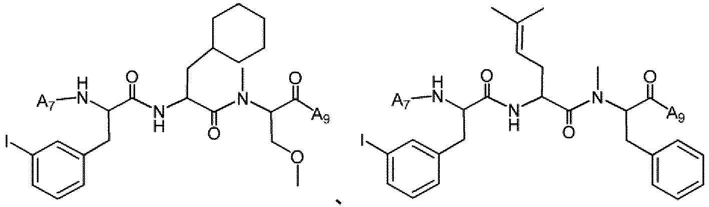

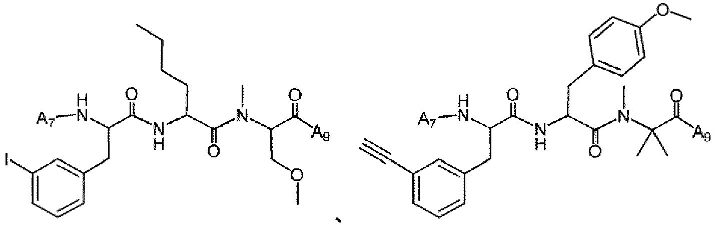

In another nonrestrictive aspect, when the binding molecule of the present disclosure is a peptide compound, from the viewpoint of easily causing the direct interaction and the preorientation, unless R₉ and P₉ form a 4- to 7-membered saturated heterocycle, an atom farthest from an α carbon atom to which R₇ is bound among atoms contained in X, and an atom farthest from an α carbon atom to which R₉ is bound among atoms contained in R₉ are preferably bound to each other via 25 or less (for example, 24, 23, 22, 21, 20, 19, 18, 17, 16, or less) atoms.

It is preferable that the binding molecule of the present disclosure is not any of the following compounds:
(1) (6S,9S,13S,16S,22S,25S,31S,34S)-6-[(2S)-butan-2-yl]-25-[(3-iodophenyl)methyl]-22-[(4-methoxyphenyl)methyl]-4,10,14,19,19,20,29,32-octamethyl-31-[(4-methylphenyl)methyl]-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
(2) (3S,6S,9S,12S,18S,27S,30S,34S)-6-benzyl-27-[(2S)-butan-2-yl]-9-butyl-12-[(3-iodophenyl)methyl]-18-[(4-methoxyphenyl)methyl]-1,6,7,16,19,22,25,31-octamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(3) (3S,6S,9S,13S,16S,22S,25S,31S,34S)-6-[(2S)-butan-2-yl]-31-(cyclohexylmethyl)-25-[(3-iodophenyl)methyl]-22-[(4-methoxyphenyl)methyl]-3,4,10,14,19,19,20,29,32-nonamethyl-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
(4) (3S,6S,9S,13S,16S,22S,25S,31S,34S)-6-[(2S)-butan-2-yl]-25-[(3-iodophenyl)methyl]-22-[(4-methoxyphenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(5) (3S,9S,12S,18S,27S,30S,34S)-27-[(2S)-butan-2-yl]-12-[(3-iodophenyl)methyl]-9-[(2-methoxyphenyl)methyl]-1,6,6,7,16,19,22,25,31-nonamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(6) (3S,9S,12S,18S,27S,30S,34S)-27-[(2S)-butan-2-yl]-18-(cyclohexylmethyl)-12-[(3-iodophenyl)methyl]-9-[(4-methoxyphenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
(7) (3S,6S,9S,12S,18S,27S,30S,34S)-27-[(2S)-butan-2-yl]-9-butyl-18-[(4-chlorophenyl)methyl]-6-[(3-fluorophenyl)methyl]-12-[(3-iodophenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
(8) (3S,9S,12S,18S,27S,30S,34S)-27-[(2S)-butan-2-yl]-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-34-(piperidine-1-carbonyl)-1,4,7,10,13,16,19,22,25,28,31 - undecazacyclotetratriacontane-2,5,8,11,14,17,20,23,26,29,32-undecone,
(9) (3S,6S,9S,13S,16S,22S,25S,31S,34S)-6-[(2S)-butan-2-yl]-22-butyl-9-ethyl-25-[(5-iodo-2-methylphenyl)methyl]-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(10) (3S,9S,12S,18S,27S,30S,34S)-27-[(2S)-butan-2-yl]-18-(cyclohexylmethyl)-12-[(3-iodophenyl)methyl]-1,6,6,7,16,19,22,25,31-nonamethyl-9-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(11) (3S,6S,9S,13S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-22-(3-methylbut-2-enyl)-9,16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
(12) (6S,9S,13S,16S,22S,25S,31S,34S)-6-[(2S)-butan-2-yl]-25-[(3-iodophenyl)methyl]-4,10,14,19,19,20,29,32-octamethyl-9,16-bis(2-methylpropyl)-13-(piperidine-1-carbonyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
(13) (3S,9S,12S,18S,27S,30S,34S)-12-[(3-bromophenyl)methyl]-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(14) (3S,6S,9S,12S,18S,27S,30S,34S)-27-[(2S)-butan-2-yl]-9-butyl-12-[(3-iodophenyl)methyl]-1,6,7,16,19,22,25,31-octamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(15) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-31-[[4-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
(16) (3S,9S,12S,18S,27S,30S,34S)-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-18-[[4-(trifluoromethoxy)phenyl]methyl]-1,4,7,10,13,16,19,22,25,28,31-undecazacyclotetratriacontane-2,5,8,11,14,17,20,23,26,29,32-undecone,
(17) (3S,9S,12S,18S,27S,30S,34S)-27-[(2S)-butan-2-yl]-18-(cyclohexylmethyl)-9-[(4-fluorophenyl)methyl]-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(18) (3S,6S,9S,13S,16S,22S,25S,31S,34S)-25-[(3-bromophenyl)methyl]-6-[(2S)-butan-2-yl]-22-butyl-31-[(4-chlorophenyl)methyl]-3,4,10,14,19,19,20,29,32-nonamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(19) (3S,9S,12S,18S,27S,30S,34S)-12-benzyl-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(20) (3S,6S,9S,13S,16S,22S,25S,31S,34S)-25-[(5-bromo-2-methylphenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(21) (3S,9S,12S,18S,27S,30S,34S)-27-[(2S)-butan-2-yl]-9-butyl-18-[(4-chlorophenyl)methyl]-12-[(3-ethynylphenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(22) (3S,6S,9S,13S,16S,22S,25S,31S,34S)-31-benzyl-6-[(2S)-butan-2-yl]-25-[(3-iodophenyl)methyl]-3,4,10,14,19,19,20,29,32-nonamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(23) (3S,9S,12S,18S,27S,30S,34S)-27-[(2S)-butan-2-yl]-9-butyl-18-[(4-chlorophenyl)methyl]-12-[(5-chlorothiophen-2-yl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(24) (3S,9S,12S,18S,27S,30S,34S)-12-[(5-bromothiophen-2-yl)methyl]-27-[(2S)-butan-2-yl]-9-butyl-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(25) (3S,9S,12S,18S,27S,30S,34S)-27-[(2S)-butan-2-yl]-9-butyl-18-[(4-chlorophenyl)methyl]-12-[(4-fluorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(26) (3S,6S,12S,15S,20S,23S,26S,30S,33S,36R)-23-[(2S)-butan-2-yl]-3-butyl-6-[(3-iodophenyl)methyl]-10,13,20,21,27,31-hexamethyl-12-[(4-methylphenyl)methyl]-26,33-bis(2-methylpropyl)-30-(piperidine-1-carbonyl)-1,4,7,10,13,18,21,24,27,31,34-undecazatricyclo[34.3.0.015,18]nonatriacontane-2,5,8,11,14,19,22,25,28,32,35-undecone,
(27) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,12,16,19,22,25,31-decamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(28) (3S,6S,9S,13S,16S,22S,25S,31S,34S)-6-[(2S)-butan-2-yl]-31-(cyclopentylmethyl)-25-[(3-iodophenyl)methyl]-3,4,10,14,19,19,20,29,32-nonamethyl-22-(2-methylprop-2-enyl)-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(29) (3S,9S,12S,18S,27S,30S,34S)-27-[(2S)-butan-2-yl]-9-butyl-18-(cyclohexylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(30) (3S,6S,9S,13S,16S,22S,25S,31S,34S)-6-[(2S)-butan-2-yl]-22-butyl-31-(cyclobutylmethyl)-25-[(3-iodophenyl)methyl]-3,4,10,14,19,19,20,29,32-nonamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(31) (6S,9S,13S,16S,22S,25S,31S,34S)-6-[(2S)-butan-2-yl]-22-butyl-31-(cyclohexylmethyl)-25-[(3-iodophenyl)methyl]-4,10,14,19,19,20,29,32-octamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(32) (3S,6S,9S,13 S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-22-(2-methylprop-2-enyl)-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(33) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-9-(2-methylprop-2-enyl)-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(34) (3S,9S,12S,18S,27S,30S,34R)-27-[(2S)-butan-2-yl]-18-[(4-chlorophenyl)methyl]-12-[(3-iodophenyl)methyl]-1,6,6,7,16,19,22,25,31,34-decamethyl-9-(3-methylbut-2-enyl)-3,30-bis(2-methylpropyl)-1,4,7,10,13,16,19,22,25,28,31-undecazacyclotetratriacontane-2,5,8,11,14,17,20,23,26,29,32-undecone,
(35) (6S,9S,13R,16S,22S,25S,31S,34S)-6-[(2S)-butan-2-yl]-31-[(4-chlorophenyl)methyl]-25-[(3 -iodophenyl)methyl]-4,10,13,14,19,19,20,29,32-nonamethyl-22-(3 -methylbut-2-enyl)-9,16-bis(2-methylpropyl)-1,4,7,10,14,17,20,23,26,29,32-undecazabicyclo[32.2.0]hexatriacontane-2,5,8,11,15,18,21,24,27,30,33-undecone,
(36) (6S,9S,13S,16S,22S,25S,31S,34S)-6-[(2S)-butan-2-yl]-31-[(4-chlorophenyl)methyl]-25-[(3-iodophenyl)methyl]-4,10,14,19,19,20,29,32-octamethyl-22-(2-methylprop-2-enyl)-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(37) (3S,9S,12S,18S,27S,30S,34S)-27-[(2S)-butan-2-yl]-9-butyl-12-[(3-iodophenyl)methyl]-1,6,6,7,16,19,22,25,31-nonamethyl-3-(3-methylbutyl)-30-(2-methylpropyl)-34-(piperidine-1-carbonyl)-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
(38) (3S,9S,12S,18S,27S,30S,34R)-27-[(2S)-butan-2-yl]-12-[(3-iodophenyl)methyl]-1,6,6,7,16,19,22,25,31,34-decamethyl-3,30-bis(2-methylpropyl)-9-prop-2-inyl-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
(39) (6S,9S,13R,16S,22S,25S,31S,34S)-6-[(2S)-butan-2-yl]-25-[(3-iodophenyl)methyl]-4,10,13,14,19,19,20,29,32-nonamethyl-9,16-bis(2-methylpropyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
(40) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,30-bis(2-methylpropyl)-34-(piperidine-1 -carbonyl)-9-prop-2-inyl-1,4,7,10,13,16,19,22,25,28,31 - undecazacyclotetratriacontane-2,5,8,11,14,17,20,23,26,29,32-undecone,
(41) (3S,6S,9S,13S,16S,22S,25S,31S,34S)-6-[(2S)-butan-2-yl]-31-[(4-chlorophenyl)methyl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(42) (3S,9S,12S,18S,27S,30S,34S)-27-[(2S)-butan-2-yl]-9-butyl-18-[(4-chlorophenyl)methyl]-3-(cyclobutylmethyl)-12-[(3-iodophenyl)methyl]-1,6,6,7,16,19,22,25,31-nonamethyl-30-(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(43) (3S,9S,12S,18S,27S,30S,34S)-27-[(2S)-butan-2-yl]-9-butyl-18-(cyclopentylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(44) (3S,9S,12S,18S,27S,30S,34S)-27-[(2S)-butan-2-yl]-3,9-dibutyl-18-[(4-chlorophenyl)methyl]-12-[(3-iodophenyl)methyl]-1,6,6,7,16,19,22,25,31-nonamethyl-30-(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(45) (1S,4S,7S,12S,15S,21S,24S,30S,33S)-4-[(2S)-butan-2-yl]-24-butyl-15-(cyclopentylmethyl)-21-[(3-iodophenyl)methyl]-6,7,14,17,26,27,27,32-octamethyl-30-(2-methylpropyl)-33-(piperidine-1-carbonyl)-3,6,9,14,17,20,23,26,29,32,36-undecazatricyclo[34.4.0.09,12]tetracontane-2,5,8,13,16,19,22,25,28,31,35-undecone,
(46) (3S,6S,9S,13S,16S,22S,25S,31S,34S)-6-[(2S)-butan-2-yl]-22-butyl-31-(cyclopentylmethyl)-25-[(3-iodophenyl)methyl]-3,4,10,14,19,19,20,29,32-nonamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(47) (3S,9S,12S,18S,27S,30S,34S)-27-[(2S)-butan-2-yl]-9-butyl-18-[(4-chlorophenyl)methyl]-19-ethyl-12-[(3-iodophenyl)methyl]-1,6,6,7,16,22,25,31-octamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(48) (3S,9S,12S,18S,27S,30S,34S)-27-[(2S)-butan-2-yl]-9-butyl-18-[(4-chlorophenyl)methyl]-12-[(3,5-dichlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(49) (3S,9S,12S,18S,27S,30S,34S)-27-[(2S)-butan-2-yl]-9-butyl-12-[(3-chloro-5-fluorophenyl)methyl]-18-[(4-chlorophenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
(50) (3S,9S,12S,18S,27S,30S,34S)-27-[(2S)-butan-2-yl]-9-butyl-12-[(3-chloro-4-fluorophenyl)methyl]-18-[(4-chlorophenyl)methyl]-1,6,6,7,16,19,22,25,31-nonamethyl-3,30-bis(2-methylpropyl)-34-(piperidine-1 -carbonyl)-1,4,7,10,13,16,19,22,25,28,31 - undecazacyclotetratriacontane-2,5,8,11, 14, 17,20,23,26,29,32-undecone,
(51) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,9,30-tris(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(52) (3S,9S,12S,18S,27S,30S,34S)-27-[(2S)-butan-2-yl]-18-[(4-chlorophenyl)methyl]-9-ethyl-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(53) (3S,9S,12S,18S,27S,30S,34S)-3-benzyl-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-34-(pyrrolidine-1 -carbonyl)-1,4,7,10,13,16,19,22,25,28,31 - undecazacyclotetratriacontane-2,5,8,11,14,17,20,23,26,29,32-undecone,
(54) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-3-propyl-34-(pyrrolidine-1-carbonyl)-1,4,7,10,13,16,19,22,25,28,31-undecazacyclotetratriacontane-2,5,8,11,14,17,20,23,26,29,32-undecone,
(55) (3S,9S,12S,18S,27S,30S,34S)-27-[(2S)-butan-2-yl]-9-butyl-18-[(4-chlorophenyl)methyl]-12-[(3-iodophenyl)methyl]-1,6,6,7,16,19,22,25,31-nonamethyl-30-(2-methylpropyl)-3-(2-phenylethyl)-34-(pyrrolidine-1-carbonyl)-1,4,7,10,13,16,19,22,25,28,31-undecazacyclotetratriacontane-2,5,8,11,14,17,20,23,26,29,32-undecone,
(56) (3S,9S,12S,18S,27S,30S,34S)-27-[(2S)-butan-2-yl]-18-[(4-chlorophenyl)methyl]-12-[(3-iodophenyl)methyl]-1,6,6,7,16,19,22,25,31-nonamethyl-3,30-bis(2-methylpropyl)-9-prop-2-enyl-34-(pyrrolidine-1 -carbonyl)-1,4,7,10,13,16,19,22,25,28,31 -undecazacyclotetratriacontane-2,5,8,11,14,17,20,23,26,29,32-undecone,
(57) (3S,9S,12S,18S,27S,30S,34S)-27-[(2S)-butan-2-yl]-9-butyl-12-[(3-chlorophenyl)methyl]-18-[(4-chlorophenyl)methyl]-1,6,6,7,16,19,22,25,31-nonamethyl-3,30-bis(2-methylpropyl)-34-(pyrrolidine-1 -carbonyl)-1,4,7,10,13,16,19,22,25,28,31 -undecazacyclotetratriacontane-2,5,8,11,14,17,20,23,26,29,32-undecone,
(58) (3S,9S,12S,18S,27S,30S,34S)-27-[(2S)-butan-2-yl]-9-butyl-18-[(4-chlorophenyl)methyl]-12-[(3-methoxyphenyl)methyl]-1,6,6,7,16,19,22,25,31-nonamethyl-3,30-bis(2-methylpropyl)-34-(pyrrolidine-1-carbonyl)-1,4,7,10,13,16,19,22,25,28,31-undecazacyclotetratriacontane-2,5,8,11,14,17,20,23,26,29,32-undecone,
(59) (3S,6S,9S,12S,18S,27S,30S,34S)-6,9-dibenzyl-27-[(2S)-butan-2-yl]-18-[(4-chlorophenyl)methyl]-4,7,16,19,22,25,30,31-octamethyl-12-[(3-methylphenyl)methyl]-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(60) (3S,6S,9S,12S,18S,27S,30S,34S)-6,9-dibenzyl-27-[(2S)-butan-2-yl]-18-[(4-chlorophenyl)methyl]-12-[(3-fluorophenyl)methyl]-4,7,16,19,22,25,30,31-octamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(61) (3S,6S,9S,12S,18S,27S,30S,34S)-6,9-dibenzyl-27-[(2S)-butan-2-yl]-18-[(4-chlorophenyl)methyl]-12-[(3,5-difluorophenyl)methyl]-4,7,16,19,22,25,30,31-octamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(62) (3S,6S,9S,12S,18S,27S,30S,34S)-6,9-dibenzyl-27-[(2S)-butan-2-yl]-12-[(3-chlorophenyl)methyl]-18-[(4-chlorophenyl)methyl]-25-ethyl-4,7,16,19,22,30,31-heptamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(63) (3S,6S,9S,12S,18S,27S,30S,34S)-6,9-dibenzyl-27-[(2S)-butan-2-yl]-12-[(3-chlorophenyl)methyl]-18-[(4-chlorophenyl)methyl]-16-ethyl-4,7,19,22,25,30,31-heptamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(64) (3S,9S,18S,21S,25S,28S,31S,34S,36R)-18-[(2S)-butan-2-yl]-3-[(3-chlorophenyl)methyl]-9-[(4-chlorophenyl)methyl]-3 6-ethoxy-7,10,13,16,21,22,29,32-octamethyl-31 -(3 - methylbutoxymethyl)-28-(2-methylpropyl)-25-(pyrrolidine-1-carbonyl)-1,4,7,10,13,16,19,22,26,29,32-undecazabicyclo[32.3.0]heptatriacontane-2,5,8,11,14,17,20,23,27,30,33-undecone,
(65) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,16,19,22,25,30,31-octamethyl-3,9-bis(2-methylpropyl)-34-(pyrrolidine-1-carbonyl)-1,4,7,10,13,16,19,22,25,28,31 - undecazacyclotetratriacontane-2,5,8,11,14,17,20,23,26,29,32-undecone,
(66) (3S,6S,9S,12S,18S,27S,30S,34S)-6-benzyl-27-[(2S)-butan-2-yl]-12-[(3-chloro-5-fluorophenyl)methyl]-18-[(4-chlorophenyl)methyl]-4,7,16,19,22,25,30,31-octamethyl-3,9-bis(2-methylpropyl)-34-(pyrrolidine-1-carbonyl)-1,4,7,10,13,16,19,22,25,28,31 - undecazacyclotetratriacontane-2,5,8,11,14,17,20,23,26,29,32-undecone,
(67) (3S,6S,9S,12S,18S,27S,30S,34S)-6-benzyl-27-[(2S)-butan-2-yl]-18-[(4-chlorophenyl)methyl]-12-[(3-iodophenyl)methyl]-4,7,16,19,22,25,30,31-octamethyl-3,9-bis(2-methylpropyl)-34-(piperidine-1-carbonyl)-1,4,7,10,13,16,19,22,25,28,31 - undecazacyclotetratriacontane-2,5,8,11,14,17,20,23,26,29,32-undecone,
(68) (3S,6S,9S,12S,18S,27S,30S,34R)-6-benzyl-27-[(2S)-butan-2-yl]-18-[(4-chlorophenyl)methyl]-12-[(3-iodophenyl)methyl]-4,7,16,19,22,25,30,31,34-nonamethyl-3,9-bis(2-methylpropyl)-1,4,7,10,13,16,19,22,25,28,31-undecazacyclotetratriacontane-2,5,8,11,14,17,20,23,26,29,32-undecone,
(69) 3-[[(3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-2, 5, 8,11,14,17,20,23,26,29,32-undecaoxo-34-(pyrrolidine-1-carbonyl)-1,4,7,10,13,16,19,22,25,28,31-undecazacyclotetratriacont-12-yl]methyl]benzonitrile,
(70) (3S,6S,9S,12S,18S,27S,30S,34S)-6-benzyl-27-[(2S)-butan-2-yl]-18-[(4-chlorophenyl)methyl]-12-[(3-iodophenyl)methyl]-4,7,16,19,22,25,30,31-octamethyl-3,9-bis(2-methylpropyl)-34-(pyrrolidine-1-carbonyl)-1,4,7,10,13,16,19,22,25,28,31 - undecazacyclotetratriacontane-2,5,8,11,14,17,20,23,26,29,32-undecone,
(71) (3S,9S,18S,21S,25S,28S,31S,34S,36R)-31-benzyl-18-[(2S)-butan-2-yl]-9-[(4-chlorophenyl)methyl]-36-ethoxy-3-[(3-iodophenyl)methyl]-7,10,13,16,21,22,29,32-octamethyl-28-(2-methylpropyl)-25-(pyrrolidine-1 -carbonyl)-1,4,7,10,13,16,19,22,26,29,32-undecazabicyclo[32.3.0]heptatriacontane-2,5,8,11,14,17,20,23,27,30,33-undecone,
(72) (3S,6S,9S,12S,18S,27S,30S,34S)-6-benzyl-27-[(2S)-butan-2-yl]-12-[(3-chlorophenyl)methyl]-18-[(4-chlorophenyl)methyl]-7,16,19,22,25,30,31-heptamethyl-3,9-bis(2-methylpropyl)-34-(pyrrolidine-1-carbonyl)-1,4,7,10,13,16,19,22,25,28,31 - undecazacyclotetratriacontane-2,5,8,11,14,17,20,23,26,29,32-undecone,
(73) 3-[[(3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-2, 5, 8,11,14,17,20,23,26,29,32-undecaoxo-34-(piperidine-1-carb onyl)-1,4,7,10,13,16,19,22,25,28,31-undecazacyclotetratriacont-12-yl]methyl]benzonitrile,
(74) (3S,6S,9S,12S,18S,27S,30S,34S)-6-benzyl-27-[(2S)-butan-2-yl]-18-[(4-chlorophenyl)methyl]-4,7,16,19,22,25,30,31-octamethyl-3,9-bis(2-methylpropyl)-34-(piperidine-1-carbonyl)-12-[[3-(trifluoromethoxy)phenyl]methyl]-1,4,7,10,13,16,19,22,25,28,31-undecazacyclotetratriacontane-2,5,8,11,14,17,20,23,26,29,32-undecone,
(75) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,16,19,22,25,30,31-octamethyl-9-[(3-methylphenyl)methyl]-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(76) (3S,6S,9S,12S,18S,27S,30S,34S)-6-benzyl-27-[(2S)-butan-2-yl]-12-[(3-chlorophenyl)methyl]-18-[(4-chlorophenyl)methyl]-4,7,9,16,19,22,25,30,31-nonamethyl-3-propan-2-yl-34-(pyrrolidine-1-carbonyl)-1,4,7,10,13,16,19,22,25,28,31-undecazacyclotetratriacontane-2,5,8,11,14,17,20,23,26,29,32-undecone,
(77) (3S,9S,12S,18S,27S,30S,34S)-27-[(2S)-butan-2-yl]-9-butyl-18-[(4-chlorophenyl)methyl]-12-[(2-fluoro-5-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(78) (3S,9S,12S,18S,27S,30S,34S)-27-[(2S)-butan-2-yl]-9-butyl-12-[(2-chloro-5-iodophenyl)methyl]-18-[(4-chlorophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(79) (1S,10S,13S,17S,20S,26S,29S)-10-[(2S)-butan-2-yl]-29-[(3-iodophenyl)methyl]-2, 5, 8,14,18,23,23,24,33-nonamethyl-13,20-bis(2-methylpropyl)-17-(piperidine-1-carbonyl)-2,5,8,11, 14, 18,21,24,27,30,33-undecazabicyclo[24.8.6]tetracontane-3,6,9,12,15,19,22,25,28,31,34-undecone,
(80) (6S,9S,13S,16S,22S,25S,31S,34S)-6-[(2S)-butan-2-yl]-22-butyl-9-hexyl-25-[(3-iodophenyl)methyl]-31-[(4-methoxyphenyl)methyl]-4,10,14,19,19,20,29,32-octamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(81) (6S,9S,13S,16S,22S,25S,31S,34S)-6-[(2S)-butan-2-yl]-25-[(3-iodophenyl)methyl]-22-[(2-methoxyphenyl)methyl]-4, 10, 14, 19, 19,20,29,32-octamethyl-31-[(4-methylphenyl)methyl]-9, 16-bis(2-methylpropyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
(82) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-22-(phenoxymethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(83) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-22-(2-phenylethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(84) (3S,9S,12S,18S,27S,30S,34S)-27-[(2S)-butan-2-yl]-18-(cyclohexylmethyl)-12-[(3-iodophenyl)methyl]-1,6,6,7,16,19,22,25,31-nonamethyl-3,30-bis(2-methylpropyl)-34-(piperidine-1-carbonyl)-9-[[4-(trifluoromethyl)phenyl]methyl]-1,4,7,10,13,16,19,22,25,28,31-undecazacyclotetratriacontane-2,5,8,11,14,17,20,23,26,29,32-undecone,
(85) (3S,9S,12S,18S,27S,30S,34S)-27-[(2S)-butan-2-yl]-9-[(4-chlorophenyl)methyl]-18-(cyclohexylmethyl)-12-[(3-iodophenyl)methyl]-1,6,6,7,16,19,22,25,31-nonamethyl-3,30-bis(2-methylpropyl)-34-(piperidine-1-carbonyl)-1,4,7,10,13,16,19,22,25,28,31 - undecazacyclotetratriacontane-2,5,8,11,14,17,20,23,26,29,32-undecone,
(86) (3S,6S,9S,13S,16S,22S,25S,31S,34S)-6-[(2S)-butan-2-yl]-31-[(4-fluorophenyl)methyl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(87) (6S,9S,13S,16S,22S,25S,31S,34S)-6-[(2S)-butan-2-yl]-25-[(3-iodophenyl)methyl]-4,10,14,19,19,20,29,32-octamethyl-31-[(4-methylphenyl)methyl]-9,16-bis(2-methylpropyl)-13-(piperidine-1-carbonyl)-22-[[2-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
(88) (6S,9S,13S,16S,22S,25S,31S,34S)-6-[(2S)-butan-2-yl]-22-[(2-chlorophenyl)methyl]-25-[(3-iodophenyl)methyl]-4,10,14,19,19,20,29,32-octamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(89) (6S,9S,13S,16S,22S,25S,31S,34S)-6-[(2S)-butan-2-yl]-22-[(2-fluorophenyl)methyl]-25-[(3-iodophenyl)methyl]-4,10,14,19,19,20,29,32-octamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(90) (6S,9S,13S,16S,22S,25S,31S,34S)-6-[(2S)-butan-2-yl]-25-[(3-iodophenyl)methyl]-4,10,14,19,19,20,29,32-octamethyl-22-[(2-methylphenyl)methyl]-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(91) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-22-(2-phenylethyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(92) (6S,9S,13S,16S,22S,25S,31S,34S)-6-[(2S)-butan-2-yl]-31-(cyclohexylmethyl)-25-[(3-iodophenyl)methyl]-4,10,14,19,19,20,29,32-octamethyl-9,16-bis(2-methylpropyl)-22-(2-phenylethyl)-13 -(piperidine-1 -carbonyl)-1,4,7,10,14,17,20,23,26,29,32-undecazabicyclo[32.2.0]hexatriacontane-2,5,8,11,15,18,21,24,27,30,33-undecone,
(93) (3S,9S,12S,18S,27S,30S,34S)-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-18-[[4-(trifluoromethyl)phenyl]methyl]-1,4,7,10,13,16,19,22,25,28,31-undecazacyclotetratriacontane-2,5,8,11,14,17,20,23,26,29,32-undecone,
(94) (3S,9S,12S,18S,27S,30S,34S)-27-[(2S)-butan-2-yl]-12-[(3-iodophenyl)methyl]-18-[(4-methoxyphenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
(95) (3S,9S,12S,18S,27S,30S,34S)-18-benzyl-27-[(2S)-butan-2-yl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31-undecazacyclotetratriacontane-2,5,8,11,14,17,20,23,26,29,32-undecone,
(96) (3S,9S,12S,18S,27S,30S,34S)-27-[(2S)-butan-2-yl]-18-[(4-fluorophenyl)methyl]-12-[(3-iodophenyl)methyl]-1,6,6,7,16,19,22,25,31-nonamethyl-3,30-bis(2-methylpropyl)-34-(piperidine-1-carbonyl)-9-prop-2-inyl-1,4,7,10,13,16,19,22,25,28,31 - undecazacyclotetratriacontane-2,5,8,11,14,17,20,23,26,29,32-undecone,
(97) (6S,9S,13S,16S,22S,25S,31S,34S)-6-[(2S)-butan-2-yl]-25-[(3-iodophenyl)methyl]-4,10,14,19,19,20,29,32-octamethyl-9,16-bis(2-methylpropyl)-13-(piperidine-1-carbonyl)-22-prop-2-inyl-31-[[4-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
(98) (6S,9S,13S,16S,22S,25S,31S,34S)-6-[(2S)-butan-2-yl]-25-[(3-iodophenyl)methyl]-31-[(4-methoxyphenyl)methyl]-4,10,14,19,19,20,29,32-octamethyl-9,16-bis(2-methylpropyl)-13-(piperidine-1 -carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(99) (6S,9S,13S,16S,22S,25S,31S,34S)-6-[(2S)-butan-2-yl]-31-[(4-fluorophenyl)methyl]-25-[(3-iodophenyl)methyl]-4,10,14,19,19,20,29,32-octamethyl-9,16-bis(2-methylpropyl)-13-(piperidine-1-carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(100) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-9-(2-phenylethyl)-34-(piperidine-1 -carbonyl)-1,4,7,10,13,16,19,22,25,28,31 - undecazacyclotetratriacontane-2,5,8,11,14,17,20,23,26,29,32-undecone,
(101) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1 -carbonyl)-9-[[2-(trifluoromethyl)phenyl]methyl]-1,4,7,10,13,16,19,22,25,28,31 - undecazacyclotetratriacontane-2,5,8,11,14,17,20,23,26,29,32-undecone,
(102) (3S,9S,12S,18S,27S,30S,34S)-27-[(2S)-butan-2-yl]-9-[(2-chlorophenyl)methyl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
(103) (3S,9S,12S,18S,27S,30S,34S)-27-[(2S)-butan-2-yl]-9-[(2-fluorophenyl)methyl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(104) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-9,16-bis(2-methylpropyl)-13-(piperidine-1-carbonyl)-22-prop-2-inyl-31-[[4-(trifluoromethyl)phenyl]methyl]-1,4,7,10,14,17,20,23,26,29,32-undecazabicyclo[32.2.0]hexatriacontane-2,5,8,11,15,18,21,24,27,30,33-undecone,
(105) (3S,6S,9S,13S,16S,22S,25S,31S,34S)-6-[(2S)-butan-2-yl]-25-[(3-iodophenyl)methyl]-31-[(4-methoxyphenyl)methyl]-3,4,10,14,19,19,20,29,32-nonamethyl-9,16-bis(2-methylpropyl)-13-(piperidine-1-carbonyl)-22-prop-2-inyl-1,4,7,10,14,17,20,23,26,29,32-undecazabicyclo[32.2.0]hexatriacontane-2,5,8,11,15,18,21,24,27,30,33-undecone,
(106) (3S,9S,12S,18S,27S,30S,34S)-27-[(2S)-butan-2-yl]-12-[(3-iodophenyl)methyl]-1,6,7,16,19,22,25,31-nonamethyl-9-[(2-methylphenyl)methyl]-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(107) (3S,9S,12S,18S,27S,30S,34S)-27-[(2S)-butan-2-yl]-12-[(3-iodophenyl)methyl]-1,6,6,7,16,19,22,25,31-nonamethyl-18-[(4-methylphenyl)methyl]-3,30-bis(2-methylpropyl)-34-(piperidine-1-carbonyl)-9-[[3-(trifluoromethyl)phenyl]methyl]-1,4,7,10,13,16,19,22,25,28,31-undecazacyclotetratriacontane-2,5,8,11,14,17,20,23,26,29,32-undecone,
(108) (3S,6S,9S,13S,16S,22S,25S,31S,34S)-22-benzyl-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(109) (3S,6S,9S,13S,16S,22S,25S,31S,34S)-6-[(2S)-butan-2-yl]-25-[(3-iodophenyl)methyl]-22-[(2-methoxyphenyl)methyl]-3,4,10,14,19,19,20,29,32-nonamethyl-31-[(4-methylphenyl)methyl]-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(110) (3S,6S,9S,13S,16S,22S,25S,31S,34S)-6-[(2S)-butan-2-yl]-25-[(3-iodophenyl)methyl]-3,4,10,14,19,19,20,29,32-nonamethyl-31-[(4-methylphenyl)methyl]-9,16-bis(2-methylpropyl)-13-(piperidine-1-carbonyl)-22-[[2-(trifluoromethoxy)phenyl]methyl]-1,4,7,10,14,17,20,23,26,29,32-undecazabicyclo[32.2.0]hexatriacontane-2,5,8,11,15,18,21,24,27,30,33-undecone,
(111) (3S,6S,9S,12S,18S,27S,30S,34S)-27-[(2S)-butan-2-yl]-9-butyl-18-[(4-chlorophenyl)methyl]-6-[(2-fluorophenyl)methyl]-12-[(3-iodophenyl)methyl - 1,7,16,19,22,25,31-heptamethyl-3,30-bis(2-methylpropyl)-34-(piperidine-l-carbonyl)-1,4,7,10,13,16,19,22,25,28,31-undecazacyclotetratriacontane-2,5,8,11,14,17,20,23,26,29,32-undecone,
(112) (3S,6S,9S,12S,18S,27S,30S,34S)-27-[(2S)-butan-2-yl]-9-butyl-18-[(4-chlorophenyl)methyl]-6-[(4-fluorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(113) (3S,6S,9S,12S,18S,27S,30S,34S)-27-[(2S)-butan-2-yl]-9-butyl-6-[(2-chlorophenyl)methyl]-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(114) (3S,6S,9S,12S,18S,27S,30S,34S)-27-[(2S)-butan-2-yl]-9-butyl-6-[(3-chlorophenyl)methyl]-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(115) (3S,6S,9S,12S,18S,27S,30S,34S)-27-[(2S)-butan-2-yl]-9-butyl-6,18-bis[(4-chlorophenyl)methyl]-12-[(3-iodophenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8,11,14,17,20,23,26,29,32-undecone,
(116) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-6-[(2-methylphenyl)methyl]-3,30-bis(2-methylpropyl)-34-(piperidine-1-carbonyl)-1,4,7,10,13, 16, 19,22,25,28,31-undecazacyclotetratriacontane-2,5,8, 11, 14, 17,20,23,26,29,32-undecone,
(117) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-6-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(118) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[2-(trifluoromethyl)phenyl]methyl]-1,4,7,10,13,16,19,22,25,28,31-undecazacyclotetratriacontane-2,5,8,11,14,17,20,23,26,29,32-undecone,
(119) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[3-(trifluoromethyl)phenyl]methyl]-1,4,7,10,13,16,19,22,25,28,31-undecazacyclotetratriacontane-2,5,8,11,14,17,20,23,26,29,32-undecone,
(120) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[4-(trifluoromethyl)phenyl]methyl]-1,4,7,10,13,16,19,22,25,28,31-undecazacyclotetratriacontane-2,5,8,11,14,17,20,23,26,29,32-undecone,
(121) (3S,6S,9S,12S,18S,27S,30S,34S)-27-[(2S)-butan-2-yl]-9-butyl-18-[(4-chlorophenyl)methyl]-12-[(3-iodophenyl)methyl]-6-[(2-methoxyphenyl)methyl]-1,7,16,19,22,25,31-heptamethyl-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(122) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[2-(trifluoromethoxy)phenyl]methyl]-1,4,7,10,13,16,19,22,25,28,31-undecazacyclotetratriacontane-2,5,8,11,14,17,20,23,26,29,32-undecone,
(123) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[3-(trifluoromethoxy)phenyl]methyl]-1,4,7,10,13, 16, 19,22,25,28,31-undecazacyclotetratriacontane-2,5,8, 11, 14, 17,20,23,26,29,32-undecone,
(124) (3S,6S,9S,12S,18S,27S,30S,34S)-27-[(2S)-butan-2-yl]-9-butyl-18-[(4-chlorophenyl)methyl]-12-[(3-iodophenyl)methyl]-1,7,16,19,22,25,31-heptamethyl-3,30-bis(2-methylpropyl)-34-(piperidine-1-carbonyl)-6-[[4-(trifluoromethoxy)phenyl]methyl]-1,4,7,10,13,16,19,22,25,28,31-undecazacyclotetratriacontane-2,5,8,11,14,17,20,23,26,29,32-undecone,
(125) (6S,9S,13S,16S,19S,22S,25S,31S,34S)-19-benzyl-6-[(2S)-butan-2-yl]-22-butyl-31-(cyclohexylmethyl)-25-[(3-iodophenyl)methyl]-4,10,14,20,29,32-hexamethyl-9,16-bis(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(126) (6S,9S,13S,16S,19S,22S,25S,31S,34S)-19-benzyl-6-[(2S)-butan-2-yl]-22-butyl-25-[(3-iodophenyl)methyl]-31-[(4-methoxyphenyl)methyl]-4,10,14,19,20,29,32-heptamethyl-9,16-bis(2-methylpropyl)-13 -(piperidine-1 -carbonyl)-1,4,7,10,14,17,20,23,26,29,32-undecazabicyclo[32.2. 0]hexatriacontane-2, 5,8, 11, 15, 18,21,24,27,30,33-undecone,
(127) (3S,6S,9S,12S,18S,27S,30S,34S)-27-[(2S)-butan-2-yl]-9-butyl-12-[(2-fluorophenyl)methyl]-6,18-bis[(4-methoxyphenyl)methyl]-1,7,16,19,22,25,31-heptamethyl-3,30-bis(2-methylpropyl)-34-(piperidine-1 -carbonyl)-1,4,7,10,13,16,19,22,25,28,31 - undecazacyclotetratriacontane-2,5,8, 11, 14, 17,20,23,26,29,32-undecone,
(128) (3S,9S,12S,17S,20S,23S,27S,30S,36S)-20-[(2S)-butan-2-yl]-9-(cyclohexylmethyl)-7,10,17,18,23,24,28,31-octamethyl-3-(3-phenylpropyl)-27-(piperidine-1-carbonyl)-30-propan-2-ylspiro[1,4,7,10,15,18,21,24,28,31,34-undecazatricyclo[34.3.0.012,15]nonatriacontane-33,1'-cyclopentane]-2,5,8, 11, 16, 19,22,25,29,32,35-undecone,
(129) (3S,6S,9S,13S,16S,22S,25S,31S,34S)-6-[(2S)-butan-2-yl]-22-butyl-25-[(2,3-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(130) (3S,6S,9S,13S,16S,22S,25S,31S,34S)-6-[(2S)-butan-2-yl]-22-butyl-25-[(2,5-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(131) (3S,6S,9S,13S,16S,22S,25S,31S,34S)-6-[(2S)-butan-2-yl]-22-butyl-25-[(2,6-difluorophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(132) (3S,6S,9S,13S,16S,22S,25S,31S,34S)-6-[(2S)-butan-2-yl]-22-butyl-9-ethyl-25-[(2-fluoro-3-iodophenyl)methyl]-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2. 0]hexatriacontane-2, 5,8, 11, 15, 18,21,24,27,30,33-undecone,
(133) (3S,6S,9S,13S,16S,22S,25S,31S,34S)-25-[(3-bromo-2-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(134) (3S,6S,9S,13S,16S,22S,25S,31S,34S)-6-[(2S)-butan-2-yl]-22-butyl-9-ethyl-25-[(3-fluoro-5-iodophenyl)methyl]-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
(135) (3S,6S,9S,13S,16S,22S,25S,31S,34S)-6-[(2S)-butan-2-yl]-22-butyl-25-[(3-chloro-5-iodophenyl)methyl]-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2. 0]hexatriacontane-2, 5,8, 11, 15, 18,21,24,27,30,33-undecone,
(136) (3S,6S,9S,13S,16S,22S,25S,31S,34S)-25-[(5-bromopyridine-3-yl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
(137) (3S,6S,9S,13S,16S,22S,25S,31S,34S)-25-[(3-bromo-5-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(138) (3 S,6S,9S,13 S,16S,22S,25S,31S,34S)-25-[(2-bromo-5-iodophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1 -carbonyl)-1,4,7,10,14,17,20,23,26,29,32-undecazabicyclo[32.2.0]hexatriacontane-2,5,8,11,15,18,21,24,27,30,33-undecone,
(139) (3S,6S,9S,13S,16S,22S,25S,31S,34S)-25-[(5-bromo-2-fluorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(140) (3S,6S,9S,13S,16S,22S,25S,31S,34S)-25-[(5-bromo-2-chlorophenyl)methyl]-6-[(2S)-butan-2-yl]-22-butyl-9-ethyl-31-[(4-methoxyphenyl)methyl]-3,4,10,14,19,19,20,29,32-nonamethyl-16-(2-methylpropyl)-13-(piperidine-1-carbonyl)-1,4,7,10,14,17,20,23,26,29,32-undecazabicyclo[32.2.0]hexatriacontane-2,5,8,11,15,18,21,24,27,30,33-undecone,
(141) (3S,9S,18S,21S,25S,28S,34S)-18-[(2S)-butan-2-yl]-28-cyclohexyl-9-(cyclohexylmethyl)-7,10,13,16,21,22,26,29-octamethyl-3 -(3 -phenylpropyl)-25-(piperidine-1 - carbonyl)spiro[1,4,7,10,13,16,19,22,26,29,32-undecazabicyclo[32.3.0]heptatriacontane-31,1'-cyclopentane]-2,5,8,11,14,17,20,23,27,30,33-undecone,
(142) (3S,6S,9S,12S,18S,27S,30S,34S)-6-benzyl-27-[(2S)-butan-2-yl]-12-[(3-iodophenyl)methyl]-1,7,9,16,19,22,25,31-octamethyl-18-[(4-methylphenyl)methyl]-3,30-bis(2-methylpropyl)-34-(piperidine-1-carbonyl)-1,4,7,10,13,16,19,22,25,28,31-undecazacyclotetratriacontane-2,5,8,11,14,17,20,23,26,29,32-undecone,
(143) (3S,6S,9S,13S,16S,19S,22S,25S,31S,34S)-19-benzyl-6-[(2S)-butan-2-yl]-25-[(3-iodophenyl)methyl]-31-[(4-methoxyphenyl)methyl]-3,4,9,10,14,20,22,29,32-nonamethyl-16-(2-methylpropyl)-13-(pyrrolidine-1-carbonyl)-1,4,7,10,14,17,20,23,26,29,32-undecazabicyclo[32.2.0]hexatriacontane-2,5,8,11,15,18,21,24,27,30,33-undecone,
(144) 3-[(3S,6S,9S,13S,16S,19S,22S,25S,31S,34S)-6-[(2S)-butan-2-yl]-22-butyl-25-[(3-iodophenyl)methyl]-31-[(4-methoxyphenyl)methyl]-3,4,10,14,20,29,32-heptamethyl-9,16-bis(2-methylpropyl)-2,5,8,11,15,18,21,24,27,30,33-undecaoxo-13-(piperidine-1-carbonyl)-1,4,7,10, 14, 17,20,23,26,29,32-undecazabicyclo[32.2.0]hexatriacontane-19-yl]-N,N-dimethylpropanamide,
(145) 3-[(3S,6S,9S,12S,18S,27S,30S,34S)-27-[(2S)-butan-2-yl]-9-butyl-12-[(3-iodophenyl)methyl]-1,7,16,19,22,25,31-heptamethyl-18-[(4-methylphenyl)methyl]-3,30-bis(2-methylpropyl)-2, 5, 8,11,14,17,20,23,26,29,32-undecaoxo-34-(piperidine-1-carbonyl)-1,4,7,10,13,16,19,22,25,28,31-undecazacyclotetratriacont-6-yl]-N,N-dimethylpropanamide,
(146) (3S,9S,12S,18S,27S,30S,34S)-27-[(2S)-butan-2-yl]-34-(4,4-difluoropiperidine-1-carbonyl)-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-1,4,7,10,13, 16, 19,22,25,28,31-undecazacyclotetratriacontane-2,5,8, 11, 14, 17,20,23,26,29,32-undecone,
(147) (3S,9S,12S,18S,27S,30S,34S)-27-[(2S)-butan-2-yl]-34-(3,3-difluoropiperidine-1-carbonyl)-12-[(3-iodophenyl)methyl]-9-[(4-methoxyphenyl)methyl]-1,6,6,7,16,19,22,25,31-nonamethyl-18-[(4-methylphenyl)methyl]-3,30-bis(2-methylpropyl)-1,4,7,10,13,16,19,22,25,28,31-undecazacyclotetratriacontane-2,5,8,11,14,17,20,23,26,29,32-undecone,
(148) (3S,9S,18S,21S,25S,28S,34S)-18-[(2S)-butan-2-yl]-28-cyclohexyl-9-(cyclohexylmethyl)-7,10,13,16,21,22,26,29-octamethyl-25-(piperidine-1-carbonyl)-3-[3-[4-(trifluoromethyl)phenyl]propyl]spiro[1,4,7,10,13,16,19,22,26,29,32-undecazabicyclo[32.3.0]heptatriacontane-31, 1 '-cyclopentane]-2,5,8, 11, 14, 17,20,23,27,30,33-undecone, and
(149) (3S,6S,9S,12S,18S,27S,30S,34S)-6,12-dibenzyl-27-[(2S)-butan-2-yl]-18-[(4-chlorophenyl)methyl]-4,7,16,19,22,25,30,31-octamethyl-3,9-bis(2-methylpropyl)-34-(pyrrolidine-1 -carbonyl)-1,4,7,10,13,16,19,22,25,28,31 -undecazacyclotetratriacontane-2,5,8,11,14,17,20,23,26,29,32-undecone.

The binding molecule of the present disclosure can be obtained by selecting, from arbitrary molecules, a molecule having the features (1) to (4) described herein. Besides, the binding molecule of the present disclosure can be produced by employing, for example, a polypeptide chemical synthesis method well-known to those skilled in the art, or a recombinant polypeptide expression method using a cell.

Examples of a chemical synthesis method for the binding molecule of the present disclosure include a liquid-phase synthesis method, a solid-phase synthesis method using Fmoc, Boc, or the like, and a combination of these. In Fmoc synthesis, an amino acid having a main chain amino group protected with an Fmoc group, having a side chain functional group protected with a protecting group not cleaved with a basic compound such as piperidine if necessary, and not protecting main chain carboxylic acid is used as a base unit. The base unit is not especially limited as long as it is a combination having an Fmoc-protected amino group and a carboxylic acid group. For example, dipeptide may be used as the base unit. The base unit to be located at the N terminal may be one excluding Fmoc amino acid. For example, it may be Boc amino acid, or a carboxylic acid analog having no amino group. The main chain carboxylic acid group is carried on a solid phase by a chemical reaction with a functional group of the solid carrier. Subsequently, the Fmoc group is deprotected with a base such as piperidine or DBU, and a condensation reaction is caused between a newly generated amino group and protected amino acid having carboxylic acid of the base unit to be subsequently added, and thus, a peptide bond is generated. In the condensation reaction, various combinations such as a combination of DIC and HOBt, a combination of DIC and HOAt, and a combination of HATU and DIPEA can be employed. By repeating the Fmoc group deprotection and the subsequent peptide bond generation reaction, a desired peptide sequence can be generated. After obtaining a desired sequence, cut-out from the solid phase, and deprotection of the protecting group of the side chain functional group having been introduced if necessary are performed. Alternatively, before the cut-out from the solid phase, peptide structure can be converted or cyclized. The cut-out from the solid phase and the deprotection may be performed under the same conditions, for example, with 90:10 TFA/H₂O, or the deprotection may be performed under different conditions if necessary. As for the cut-out from the solid phase, the cut-out can be performed with weak acid of 1% TFA or the like in some cases, or with Pd or the like used as a protecting group, orthogonality of these chemical reactions can be utilized. Between or after these processes, a process of cyclization or the like can be performed. For example, the side chain carboxylic acid and an amino group of the N terminal main chain can be condensed, or the side chain amino group and carboxylic acid of the C terminal main chain can be condensed. At this point, reaction orthogonality is necessary between carboxylic acid on the C terminal side and the side chain carboxylic acid to be cyclized, or between the main chain amino group or hydroxy group on the N terminal side and the side chain amino group to be cyclized, and hence the protecting group is selected in consideration of the orthogonality of the protecting group as described above. Besides, when a chloroacetyl group is located at the N terminal, cyclization can be caused with a thiol group of the side chain of a cysteine residue. The thus obtained reaction product can be purified with a reverse phase column, a molecular sieving column or the like. Details of these processes are described, for example, in Solid Phase Synthesis Handbook issued by Merck on May 1, 2002.

The binding molecule of the present disclosure can also be produced by employing a recombination method or structure. In one aspect, a nucleic acid encoding the binding molecule of the present disclosure is inserted into an appropriate expression vector, the vector is introduced into an appropriate cell to culture a transformed cell, and the thus expressed and modified protein is isolated, purified and cultured. Such a protein can also be expressed in the form of a fused protein with another protein for purposes of easing purification and the like. For example, a method in which *E.coli* is used as a host to prepare it as a fused protein with a maltose binding protein (vectors of pMAL series available from New England BioLabs, USA), a method in which it is prepared as a fused protein with glutathione-S-transferase (GST) (vectors of pGEX series available from Amersham Pharmacia Biotech), a method in which it is prepared with a histidine tag added (pET series of Novagen), and the like can be employed. The host cell is not especially limited as long as it is a cell suitable for expression of a recombinant protein, and in addition to *E.coli* mentioned above, yeasts, various animal and plant cells, insect cells and the like can be used, for example. For introducing a vector into the host cell, various methods known to those skilled in the art can be employed. For example, for introduction into *E.coli,* an introduction method using a calcium ion (Mandel, M., Higa, A. (1970) Journal of Molecular Biology, 53, 158-162, Hanahan, D. (1983) Journal of Molecular Biology, 166, 557-580) can be employed. The protein expressed in the host cell can be purified and collected from the host cell, or a cell culture or a culture supernatant thereof by a method known to those skilled in the art. When the protein is expressed in the form of a fused protein with the maltose binding protein, or the like, affinity purification can be easily performed.

In one nonrestrictive aspect, the production of the binding molecule of the present disclosure may include a process of specifying the binding molecule of the present disclosure. In other words, the binding molecule of the present disclosure can be obtained also by contacting test molecules with the mutant RAS (G12D) to select a binding molecule having the above-described features (1) to (4) and having binding selectivity for mutant RAS (G12D) 5 times or more over wild type RAS in accordance with the description given herein.

The test molecules of the present disclosure are not especially limited, and examples include a single compound such as a natural product, an organic compound, an inorganic compound, a protein, a peptide, and an amino acid, and an expression product of chemical library or gene library, a cell extract, a cell culture supernatant, a microbial fermentation product, a marine life extract, a plant extract, a prokaryotic cell extract, an eukaryotic single cell extract, or an animal cell extract. These may be purified products, or may be partially purified products like plant, animal, microorganism extracts and the like. Besides, a method for producing the test molecule is not also especially limited, and it may be one isolated from a natural product, one chemically or biochemically synthesized, or one prepared by genetic engineering.

The present disclosure also provides a pharmaceutical composition containing the binding molecule of the present disclosure.

The pharmaceutical composition of the present disclosure can be formulated by a known method with a pharmaceutically acceptable carrier introduced in addition to the binding molecule of the present disclosure, a salt of the binding molecule or a solvate thereof. For the formulation, an excipient, a binding agent, a lubricant, a colorant, and a corrigent usually used, and if necessary, a stabilizer, an emulsifier, an absorption accelerator, a surfactant, a pH adjuster, a preservative, an antioxidant and the like can be used, and the formulation is performed by compounding ingredients generally used as raw materials of a pharmaceutical formulation by an ordinary method.

For example, in producing an oral formulation, the binding molecule of the present disclosure or a salt thereof, an excipient, and, if necessary, a binding agent, a disintegrating agent, a lubricant, a colorant, a corrigent or the like are added, and then the resultant mixture is formed by an ordinary method into powders, fine granules, granules, tablets, coated tablets, capsules or the like.

Examples of these ingredients include animal and vegetable oils such as soybean oil, beef tallow, and synthetic glyceride; hydrocarbons such as liquid paraffin, squalane, and solid paraffin; ester oils such as octyldodecyl myristate, and isopropyl myristate; higher alcohols such as cetostearyl alcohol and behenyl alcohol; silicon resin; silicon oil; surfactants such as polyoxyethylene fatty acid ester, sorbitan fatty acid ester, glycerin fatty acid ester, polyoxyethylene sorbitan fatty acid ester, polyoxyethylene hydrogenated castor oil, and a polyoxyethylene polyoxypropylene block copolymer; water-soluble polymers such as hydroxyethyl cellulose, polyacrylic acid, a carboxyvinyl polymer, polyethylene glycol, polyvinyl pyrrolidone, and methyl cellulose; lower alcohols such as ethanol, and isopropanol; polyhydric alcohols such as glycerin, propylene glycol, dipropylene glycol, and sorbitol; sugars such as glucose and sucrose; inorganic powders such as silicic acid anhydride, aluminum magnesium silicate, and aluminum silicate, and purified water.

Examples of the excipient include lactose, corn starch, white sugar, glucose, mannitol, sorbit, crystalline cellulose, and silicon dioxide.

Examples of the binding agent include polyvinyl alcohol, polyvinyl ether, methylcellulose, ethylcellulose, gum arabic, tragacanth, gelatin, shellac, hydroxy propylmethylcellulose, hydroxypropyl cellulose, polyvinyl pyrrolidone, a polypropylene glycol-polyoxyethylene-block polymer, and meglumine.

Examples of the disintegrating agent include starch, agar, gelatin powder, crystalline cellulose, calcium carbonate, sodium bicarbonate, calcium citrate, dextrin, pectin, and carboxymethyl cellulose-calcium.

Examples of the lubricant include magnesium stearate, talc, polyethylene glycol, silica, and a hydrogenated vegetable oil.

As the colorant, one allowed to be added in a pharmaceutical is used, and as the corrigent, cocoa powder, menthol, an aromatic powder, peppermint oil, borneol, a cinnamon powder or the like is used.

It goes without saying that such a tablet/granule may be sugar-coated, or appropriately coated if necessary. Besides, for production of a syrup or a liquid such as an injectable preparation, a pH adjuster, a solvent, a tonicity agent, and if necessary, a dissolution assisting agent, a stabilizer or the like are added to the binding molecule of the present disclosure or a pharmacologically acceptable salt thereof, and the resultant is formulated by an ordinary method.

The pharmaceutical composition can be used parenterally in the form of, for example, a sterile solution with water or another pharmaceutically acceptable liquid, or an injection of a suspension agent. It can be presumed, for example, that the pharmaceutical composition is formulated by mixing with an appropriate combination with a pharmacologically acceptable carrier or medium, specifically such as sterile water, saline, a vegetable oil, an emulsifier, a suspension, a surfactant, a stabilizer, a flavoring agent, an excipient, a vehicle, a preservative, or a binding agent, in a unit dosage form required for generally accepted pharmaceutical practice. Specific examples of the carrier include light anhydrous silicic acid, lactose, crystalline cellulose, mannitol, starch, carmellose calcium, carmellose sodium, hydroxypropyl cellulose, hydroxypropylmethyl cellulose, polyvinylacetal diethylaminoacetate, polyvinyl pyrrolidone, gelatin, medium-chain triglyceride, polyoxyethylene hydrogenated castor oil 60, white sugar, carboxymethyl cellulose, corn starch, and inorganic salts. An active ingredient amount in such a preparation is set so that an appropriate dose falling in an instructed range can be obtained.

A sterile composition for injection can be prescribed in accordance with usual pharmaceutical practice by using a vehicle such as distilled water for injection.

Examples of an aqueous solution for injection include saline, a tonicity agent containing glucose or another adjuvant, such as D-sorbitol, D-mannose, D-mannitol, and sodium chloride, and an appropriate dissolution assisting agent, for example, alcohol, specifically ethanol, polyalcohol, for example, propylene glycol, polyethylene glycol, a nonionic surfactant, such as polysorbate 80(R), or HCO-50 may be used together.

Examples of an oily liquid include sesame oil, and soybean oil, and benzyl benzoate or benzyl alcohol may be used together as a dissolution assisting agent. Besides, a buffer, such as a phosphate buffer, or sodium acetate buffer, a soothing agent such as procaine chloride, a stabilizer such as benzyl alcohol, phenol, or an antioxidant may be compounded together. The thus prepared injection is usually filled in an appropriate ampoule.

Administration is preferably oral administration, but the administration method is not limited to oral administration. Examples of parenteral administration specifically include injection dosage form, nasal administration dosage form, pulmonary administration dosage form, and transdermal administration form. As an example of the injection dosage form, the composition can be systemically or locally administered by, for example, intravenous injection, intramuscular injection, intraperitoneal injection, subcutaneous injection or the like.

Besides, the administration method can be appropriately selected in accordance with the age and the symptom of a patient. A dose of the pharmaceutical composition containing the binding molecule of the present disclosure can be selected from a range from 0.0001 mg to 1,000 mg per kg of body weight per dose. Alternatively, the dose can be selected, for example, from a range from 0.001 to 100,000 mg/body per patient, but is not always limited by these numerical values. The dose and the administration method are varied depending on the weight, the age, and the symptom of a patient, and can be appropriately selected by those skilled in the art.

In one aspect, the binding molecule of the present disclosure can be used for inhibiting the function of the mutant RAS (G12D). In another aspect, the binding molecule of the present disclosure can be used for treating or preventing a disease related to the expression of the mutant RAS (G12D) in a subject. An example of such a disease includes cancer. Accordingly, the present disclosure provides a cancer therapeutic agent containing the binding molecule of the present disclosure, and a pharmaceutical composition for treating and/or preventing cancer containing the binding molecule of the present disclosure. The present disclosure also relates to a method for treating and/or preventing cancer including a step of administering the binding molecule of the present disclosure to a subject. The present disclosure also relates to use of the binding molecule of the present disclosure in preparation of a drug for treating and/or preventing cancer. The present disclosure further relates to the binding molecule of the present disclosure to be used for treating and/or preventing cancer. Examples of the cancer include, but are not limited to, pancreatic cancer, colon cancer, and lung cancer.

The "subject" used herein encompasses a mammal, and the mammal is preferably a human.

It is noted that all the prior art literatures cited herein are incorporated herein by reference.

### [Examples]

Contents of the present invention will now be further described by way of production examples, examples, comparative examples, and evaluation examples, and it is noted that the present invention is not limited to these contents.

### Production Example 1 Solid Phase Synthesis of Peptide Compound

All starting materials and reagents were obtained from commercial suppliers or synthesized by known methods unless otherwise described about production methods.

In accordance with a peptide synthesis method employing an Fmoc method described in WO2013/100132 or WO2018/225864, peptide elongation was performed through the following basic route, specifically, through processes of five stages of:
1) a peptide elongation reaction, from the N terminal of an amino acid by Fmoc method, with carboxylic acid of Asp side chain or carboxylic acid of D-3-Abu carried on a 2-chlorotrityl resin;
2) cut-out process of the peptide from the 2-chlorotrityl resin;
3) amide cyclization through coupling reaction of carboxylic acid of Asp side chain or carboxylic acid of D-3-Abu generated through removal from the 2-chlorotrityl resin by the cut-out process, and an amino group of a peptide chain N terminal (triangle unit shown in a scheme described below);
4) deprotection of a protecting group of a side chain functional group contained in the peptide chain if necessary; and
5) purification of a resultant compound by preparative HPLC.
In Examples and Comparative Examples, a peptide compound was synthesized based on this basic route unless otherwise described.

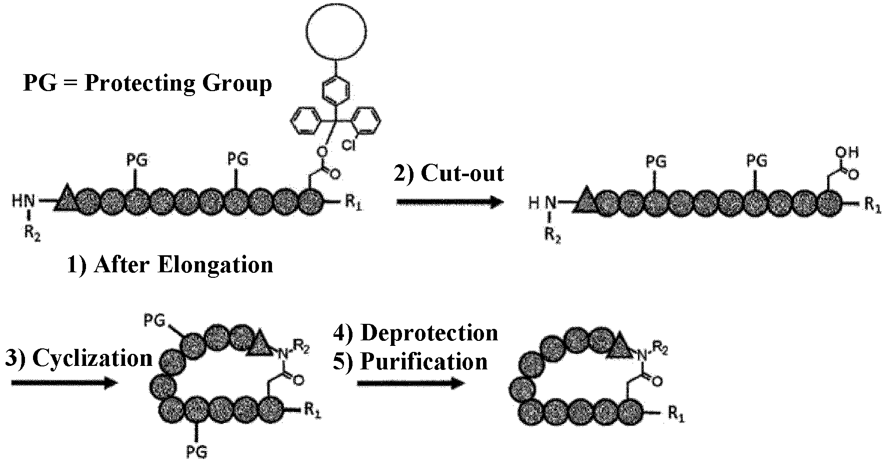

Analysis conditions in LC/MS are shown in Table 1.

**[Table 1]**

| Analysis Conditions | Apparatus | Column (I. D × length) (mm) | Mobile Phase | Gradient (A/B) | Flow Rate (mL/min) | Column Temperature (°C) | Wavelength | Note |
|---|---|---|---|---|---|---|---|---|
| SQDFA05 | Acquity UPLC/SQD or Acquity UPLC/SQD2 | Ascentis Express C18 (2. 1×50) | A) 0. 1% FA. water | 95/5 (initial) | 1.0 | 35 | 210-400nm PDA total | |
| | | | B) 0. 1% FA. acetonitrile | => 0/100 (1.0 min) | | | | |
| | | | | => 0/100 (0.4 min) | | | | |
| SQDFA3080 | Acquity UPLC/SQD2 | Ascentis Express C18 (2. 1×50) | A) 0. 1% FA. water | 70/30 (initial) | 1.0 (initial-4.51min) 0.1 (4.51-5. 00min) | 35 | 210-400nm PDA total | |
| | | | | => 20/80 (4.5 min) | | | | |
| | | | B) 0. 1% FA. acetonitrile | => 0/100 (0.01 min) | | | | |
| | | | | => 0/100 (0.49 min) | | | | |
| SMDmethod_16 | Shimadzu LCMS-2020 | Accucore C18 (2.1×50) | A) 0.1% FA. water | 90/10 (initial) | 1.0 | 40 | 190-400nm PDA total | |
| | | | B) 0.1% FA. acetonitrile | => 0/100 (1.1 min) | | | | |
| | | | | => 0/100 (0.5 min) | | | | |
| SMDmethod_17 | Shimadzu LCMS-2020 | Ascentis Express C18 (3.0x50) | A) 0.05% TFA, water | 95/5 (initial) | 1.0 | 40 | 190-400nm PDA total | |
| | | | B) 0.05% TFA, acetonitrile | => 5/95 (2.7 min) | | | | |
| | | | | => 5/95 (1.0 min) | | | | |
| SMDmethod_21 | Shimadzu LCMS-2010EV | Shim-Pack XR-ODS (3.0×50) | A) 0.05% TFA, water | 95/5 (initial) | 1.2 | 40 | 190-400nm PDA total | |
| | | | B) 0.05% TFA, acetonitrile | => 5/95 (2.0 min) | | | | |
| | | | | => 5/95 (0.7 min) | | | | |
| SSC-A-FA-01 | Nexera UC/2020 | XSelect CSH C18 (2.1×50) | A) 0.1% FA. water | 70/30 (initial) | 0.5 | 50 | 210-400nm PDA total | Loop Injection |
| | | | | => 10/90 (7.5 min) | | | | |
| | | | B) 0.1% FA. acetonitrile | => 0/100 (0.01 min) | | | | |
| | | | | => 0/100 (2.49 min) | | | | |
| SSC-A-AF-01 | Nexera UC/2020 | Ascent is Express C18 (2.1×50) | A) 10mM NH4HCO2. water | 70/30 (initial) | 0.5 | 50 | 210-400nm PDA total | Loop Injection |
| | | | B) methanol | => 0/100 (8.75 min) | | | | |
| | | | | => 0/100 (1.25 min) | | | | |
| SMDmethod_14 | Shimadzu LCMS-2020 | Shim-Pack XR-ODS (3.0×50) | A) 0.05% TFA, water | 70/30 (initial) | 1.2 | 40 | 190-400nm PDA total | |
| | | | | => 20/80 (3.8 min) | | | | |
| | | | B) 0.05% TFA, acetonitrile | => 0/100 (0.3 min) | | | | |
| | | | | => 0/100 (0.5 min) | | | | |
| SMDmethod 15 | Shimadzu LCMS-2020 | Shim-Pack XR-ODS (3.0x50) | A) 0.05% TFA, water | 95/5 (initial) | 1.2 | 40 | 190-400nm PDA total | |
| | | | B) 0.05% TFA, acetonitrile | => 0/100 (1.1 min) | | | | |
| | | | | => 0/100 (0.6 min) | | | | |

### 1. Fmoc-Amino Acid for Use in Peptide Synthesis with Peptide Synthesizer

In peptide synthesis described herein, Fmoc-amino acids shown in Tables 2 and 3 were used in synthesis with a peptide synthesizer.

The Fmoc-amino acids shown in Table 2 were purchased from commercial suppliers.

The Fmoc-amino acids shown in Table 3 were synthesized as described in this production example.

**[Table 2]**

| Abbreviation | Structural Formula | Name | CAS |
|---|---|---|---|
| Fmoc-Aze(2) -OH | 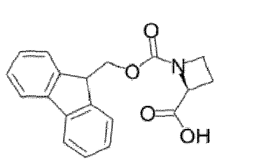 | (2S)-1-(9H-fluoren-9-ylmethoxycarbonyl)azeti dine-2-carboxylic acid | 136552-06-2 |
| Fmoc-Cha-OH | 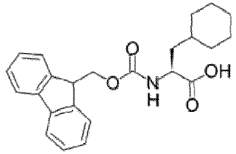 | (2S)-3-cyclohexyl-2-(9H-fluoren-9-ylmethoxycarbonylamin o)propanoic acid | 135673-97-1 |
| Fmoc-cLeu-OH | 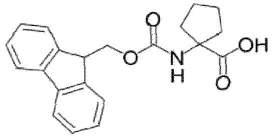 | 1-(9H-fluoren-9-ylmethoxycarbonylamin o)cyclopentane-1-carboxylic acid | 117322-30-2 |
| Fmoc-Gly(cBu) -OH | 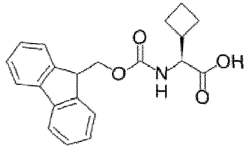 | (2S)-2-cyclobutyl-2-(9H-fluoren-9-ylmethoxycarbonylamin o)acetic acid | 1391630-31-1 |
| Fmoc-Ile-OH | 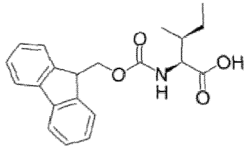 | (2S,3 S)-2-(9H-fluoren-9-ylmethoxycarbonylamino )-3-methylpentanoic acid | 71989-23-6 |
| Fmoc-Leu-OH | 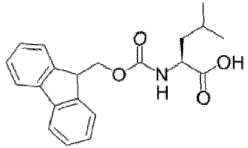 | (2S)-2-(9H-fluoren-9-ylmethoxycarbonylamino )-4-methylpentanoic acid | 35661-60-0 |
| Fmoc-MeAib -OH | 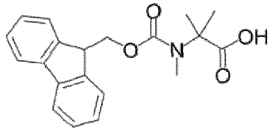 | 2-[9H-fluoren-9-ylmethoxycarbonyl(meth yl)amino]-2-methylpropanoic acid | 400779-65-9 |
| Fmoc-MeAla -OH | 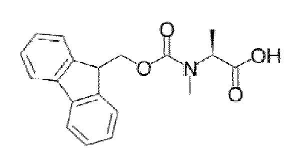 | (2S)-2-[9H-fluoren-9-ylmethoxycarbonyl(meth yl)amino]propanoic acid | 84000-07-7 |
| Fmoc-MeCha -OH | 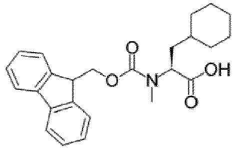 | (2S)-3-cyclohexyl-2-[9H-fluoren-9-ylmethoxycarbonyl(meth yl)amino]propanoic acid | 148983-03-3 |
| Fmoc-MeGly -OH | 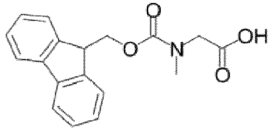 | 2-[9H-fluoren-9-ylmethoxycarbonyl(meth yl)amino]acetic acid | 77128-70-2 |
| Fmoc-MeLeu -OH | 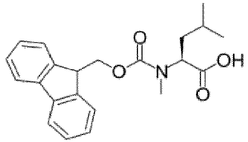 | (2S)-2-[9H-fluoren-9-ylmethoxycarbonyl(meth yl)amino]-4-methylpentanoic acid | 103478-62-2 |
| Fmoc-MePhe -OH | 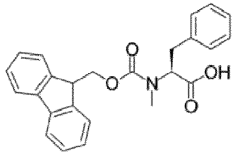 | (2S)-2-[9H-fluoren-9-ylmethoxycarbonyl(meth yl)amino]-3-phenylpropanoic acid | 77128-73-5 |
| Fmoc-MeTyr (Me)-OH | 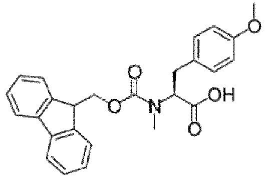 | (2S)-2-[9H-fluoren-9-ylmethoxycarbonyl(meth yl)amino]-3-(4-methoxyphenyl)propanoi c acid | 1260595-45-6 |
| Fmoc-MeVal -OH | 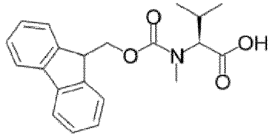 | (2S)-2-[9H-fluoren-9-ylmethoxycarbonyl(meth yl)amino]-3-methylbutanoic acid | 84000-11-3 |
| Fmoc-Nle-OH | 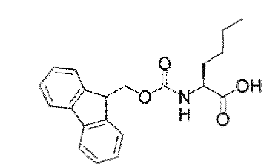 | (2S)-2-(9H-fluoren-9-ylmethoxycarbonylamino )hexanoic acid | 77284-32-3 |
| Fmoc-Phe (23-F2)-OH | 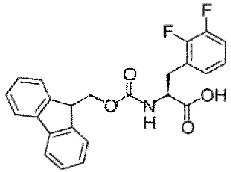 | (2S)-3-(2,3-difluorophenyl)-2-(9H-fluoren-9-ylmethoxycarbonylamino )propanoic acid | 1260605-30-8 |
| Fnmoc-Phe (3-I)-OH | 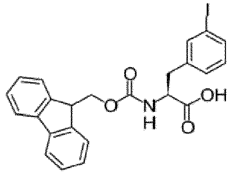 | (2S)-2-(9H-fluoren-9-ylmethoxycarbonylamino )-3-(3-iodophenyl)propanoic acid | 210282-31-8 |
| Fmoc-Pic(2) -OH | 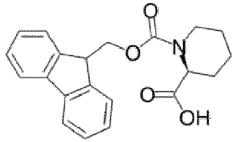 | (2S)-1-(9H-fluoren-9-ylmethoxycarbonyl)piperi dine-2-carboxylic acid | 86069-86-5 |
| Fmoc-Pregly -OH | 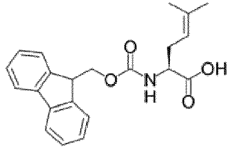 | (2S)-2-(9H-fluoren-9-ylmethoxycarbonylamino )-5-methylhex-4-enoic acid | 914486-08-1 |
| Fmoc-Pro-OH | 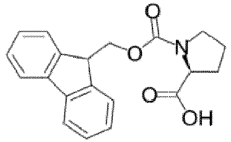 | (2S)-1-(9H-fluoren-9-ylmethoxycarbonyl)pyrro lidine-2-carboxylic acid | 71989-31-6 |
| Fmoc-Cha (4-F2)-OH | 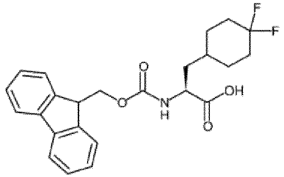 | (S)-2-((((9H-fluoren-9-yl)methoxy)carbonyl)ami no)-3-(4,4-difluorocyclohexyl)propa noic acid | 2276607-04-4 |
| Fmoc-MeChg -OH | 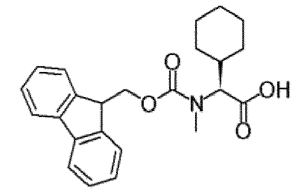 | (2S)-2-cyclohexyl-2-[9H-fluoren-9-ylmethoxycarbonyl(meth yl)amino]acetic acid | 925240-97-7 |
| Fmoc-Tyr(Me) -OH | 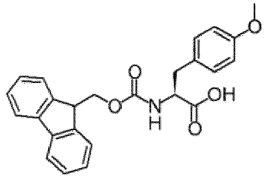 | (2S)-2-(9H-fluoren-9-ylmethoxycarbonylamino )-3-(4-methoxyphenyl)propanoi c acid | 77128-72-4 |
| Fmoc-Phe (3-CSC)-OH | 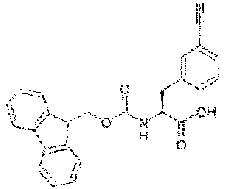 | (2S)-3-(3-ethynylphenyl)-2-(9H-fluoren-9-ylmethoxycarbonylamino )propanoic acid | 2350099-77-1 |

**[Table 3]**

| Compound No. | Abbreviation | Structural Formula | Name | CAS No. |
|---|---|---|---|---|
| aa001 | Fmoc-Hph(4-CF3-3-Cl)-OH | 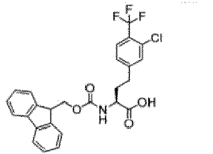 | ((2S)-4-[3-chloro-4-(trifluoromethyl)phenyl]-2-(9H-fluoren-9-ylmethoxycarbonylamino) butanoic acid | 2349480-51-7 |
| aa002 | Fmoc-MeHse(Me)-OH | 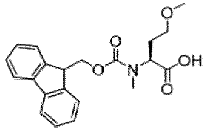 | (2S)-2-[9H-fluoren-9-ylmethoxycarbonyl(methy l)amino]-4-methoxybutanoic acid | 1979169-11-3 |
| aa003 | Fmoc-MePhe(4-Me)-OH | 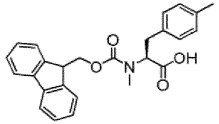 | (2S)-2-[9H-fluoren-9-ylmethoxycarbonyl(methy l)amino]-3-(4-methylphenyl)propanoic acid | 227616-20-8 |
| aa004 | Fmoc-MeSer(Me)-OH | 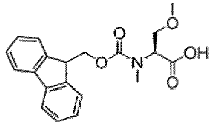 | (2S)-2-[9H-fluoren-9-ylmethoxycarbonyl(methy l)amino]-3-methoxypropanoic acid | 1589103-64-5 |
| aa005 | Fmoc-MeCha(4-F2)-OH | 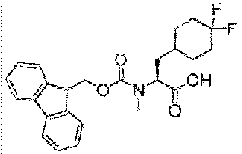 | (2S)-3-(4,4-difluorocyclohexyl)-2-[9H-fluoren-9-ylmethoxycarbonyl(methy l)amino]propanoic acid | |
| aa006 | Fmoc-R-MeAMPA -OH | 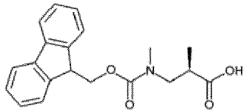 | (2R)-3-[9H-fluoren-9-ylmethoxycarbonyl(methy l)amino]-2-methyl-propanoic acid | |

### Synthesis of Fmoc-Amino Acids

### Synthesis of Compound aa001, ((2S)-4-[3-chloro-4-(trifluoromethyl)phenyl]-2-(9H-fluoren-9-ylmethoxycarbonylamino) butanoic acid Fmoc-Hph (4-CF3-3-Cl)-OH

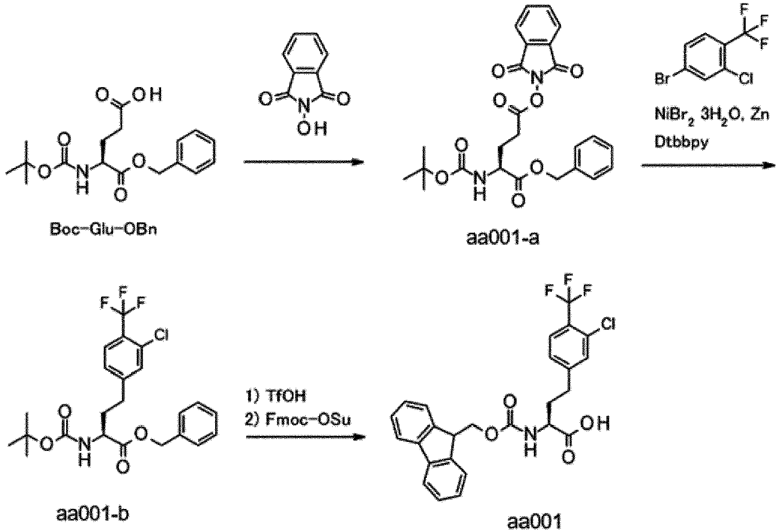

To a solution of (4S)-4-[(2-methylpropan-2-yl)oxycarbonylamino]-5-oxo-5-phenylmethoxypentanoic acid (Boc-Glu-OBn, CAS No. 30924-93-7) (200 g, 592.82 mmol), N-hydroxyphthalimide (106 g, 649.78 mmol, 1.10 equivalents), and 4-dimethylaminopyridine (DMAP, 3.6 g, 29.47 mmol, 0.05 equivalents) in tetrahydrofuran (THF, 2 L), N,N'-diisopropylcarbodiimide (DIC, 138 mL, 1.54 equivalents) was added in a dropwise manner in a nitrogen atmosphere at 0°C. The obtained reaction solution was stirred at 25°C for 16 hours, a solid content was removed by filtration, and the solvent was distilled off from the resultant filtrate under reduced pressure. The resultant residue was diluted with toluene, a solid content thus generated was removed by filtration, and the solvent was removed from the resultant filtrate under reduced pressure. The resultant residue was purified by recrystallization (acetone/heptane) to obtain a compound aa001-a (1-O-benzyl 5-O-(1,3-dioxoisoindol-2-yl) (2S)-2-[(2-methylpropan-2-yl)oxycarbonylamino]pentanedioate) (230 g, 80%).
LCMS (ESI) m/z = 505.2 (M + Na)+
Retention time: 0.992 min (analysis condition SMDmethod_16)

Nickel bromide trihydrate (NiBr₂·3H₂O) (4 g, 0.07 equivalents) and 4,4'-di-tert-butyl-2,2'-bipyridyl (dtbbpy, CAS No. 72914-19-3) (3.9 g, 14.55 mmol, 0.07 equivalents) were added to DMA (500 mL), and the resultant was stirred in a nitrogen atmosphere for 2 hours at 50°C to prepare a Ni solution.

To a mixed solution of the compound aa001-a (100 g, 207.3 mmol), a zinc powder (70 g, 5 equivalents), and 4-bromo-2-chloro-1-(trifluoromethyl)benzene (CAS No. 467435-07-0, 160 g, 617 mmol, 3 equivalents) in DMA (500 mL), the precedently prepared Ni solution was added, followed by stirring at 25°C for 16 hours. To the resultant reaction solution, an ethylenediamine tetraacetic acid disodium salt (EDTA·2Na) aqueous solution (10%) was added, and the resultant was extracted with ethyl acetate. The combined organic layers were washed with saturated saline, and dried over anhydrous sodium sulfate, and the solvent was removed under reduced pressure. The thus obtained residue was purified by silica gel column chromatography (ethyl acetate/petroleum ether) to obtain a compound aa001-b (75 g, 77%).
LCMS (ESI) m/z = 494 (M + Na)+
Retention time: 2.863 min (analysis condition SMDmethod_17)

A solution of the compound aa001-b (75 g, 158.93 mmol) in toluene (900 mL) was cooled to 0°C, and trifluoromethanesulfonic acid (TfOH) (42 mL, 3.00 equivalents) was added thereto in a dropwise manner. The resultant was stirred at room temperature for 1 hour, and water (75 mL) was added thereto. The resultant mixture was extracted with water, and the combined aqueous layers were extracted with ethyl acetate. The combined organic layers were washed with water, and dried over anhydrous sodium sulfate, and the solvent was removed under reduced pressure. To the resultant residue, acetonitrile/water (900/900 mL) was added, and pH was adjusted to 7 with a sodium hydroxide aqueous solution (48%). To the resultant solution, Fmoc-OSu (51.2 g, 151.93 mmol, 0.95 equivalents) was added, and the resultant was stirred at room temperature for 16 hours with keeping pH to 7.8 to 8.0. The resultant reaction solution was filtered, and the resultant filtrate was adjusted to pH 2 with 6 mol/L hydrochloric acid water. The thus precipitated solids were collected, and dried at 50°C to obtain a compound aa001 ((2S)-4-[3-chloro-4-(trifluoromethyl)phenyl]-2-(9H-fluoren-9-ylmethoxycarbonylamino) butanoic acid, Fmoc-Hph (4-CF3-3-Cl)-OH). (70 g, 87%)
LCMS (ESI) m/z = 525.8 (M + Na)+
Retention time: 2.180 min (analysis condition SMDmethod_21)
¹H-NMR (300 MHz, DMSO-d6) δ 12.70 (s, 1H), 7.91 (d, J = 7.5 Hz, 2H), 7.79-7.59 (m, 5H), 7.45-7.28 (m, 5H), 4.40-4.19 (m, 3H), 3.96-3.88 (m, 1H), 2.82-2.60 (m, 2H), 2.11-1.77 (m, 2H)

### Synthesis of Compound aa002, (2S)-2-[9H-fluoren-9-ylmethoxycarbonyl(methyl)amino 1-4-methoxybutanoic acid (Fmoc-MeHse(Me)-OH)

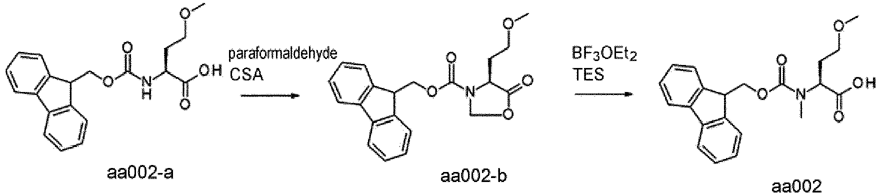

A compound aa002-a ((2S)-2-[9H-fluoren-9-ylmethoxycarbonylamino]-4-methoxybutanoic acid, Fmoc-Hse(Me)-OH) (5.0 g, 14.07 mmol), paraformaldehyde (1.27 g, 42.2 mmol), and 10-camphorsulfonic acid (CSA, 0.163 g, 0.70 mmol) were suspended in toluene (82 mL), followed by stirring at 80°C for 14 hours. The resultant reaction solution was cooled to room temperature, the filtrate obtained therefrom was diluted with ethyl acetate (100 mL), and washed with a saturated sodium bicarbonate aqueous solution/water (1/1) and saturated saline/water (1/1). The organic layer was dried over anhydrous sodium sulfate, filtered, and then concentrated under reduced pressure to obtain a compound aa002-b as a crude product (5.1 g, 99%).
LCMS (ESI) m/z = 368 (M + H)+
Retention time: 0.84 min (analysis condition SQDFA05)

To a solution of the obtained compound aa002-b (5.12 g, 13.94 mmol) in dichloromethane (DCM, 46.5 mL), triethylsilane (TES, 6.68 mL, 41.8 mmol), water (0.251 mL, 13.94 mmol), and a boron trifluoride diethyl ether complex (BF₃·OEt₂) (3.53 mL, 27.9 mmol) were added under ice cooling in a nitrogen atmosphere, followed by stirring for 2 hours. To the resultant reaction solution, a saturated ammonium chloride aqueous solution/water (1/1) and water (5 mL) were added to separate the organic layer. The aqueous layer was extracted with DCM, and the organic layer was washed with saturated saline/water (1/1). The resultant organic layer was dried over anhydrous sodium sulfate, filtered, and then concentrated under reduced pressure, and the thus obtained compound was dissolved in acetonitrile, and the resultant was washed with n-hexane and concentrated under reduced pressure to obtain a compound aa002 ((2S)-2-[9H-fluoren-9-ylmethoxycarbonyl(methyl)amino]-4-methoxybutanoic acid, Fmoc-MeHse(Me)-OH). (4.9 g, 95%)
LCMS (ESI) m/z = 370 (M + H)+
Retention time: 0.76 min (analysis condition SQDFA05)

### Synthesis of Compound aa003, (2S)-2-[9H-fluoren-9-ylmethoxycarbonyl(methyl)amino]-3-(4-methylphenyl) propanoic acid (Fmoc-MePhe(4-Me)-OH

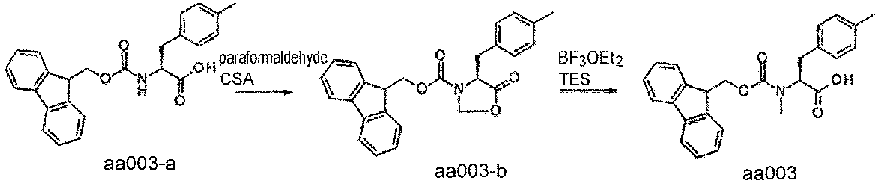

A compound aa003-a (2S)-2-[9H-fluoren-9-ylmethoxycarbonylamino]-3-(4-methylphenyl)propanoic acid, Fmoc-Phe(4-Me)-OH) (20 g, 49.8 mmol) was used as a starting material to obtain, in the same manner as in the synthesis of the compound aa002-b, a compound aa003-b as a crude product (22.66 g).
LCMS (ESI) m/z = 414 (M + H)+
Retention time: 1.04 min (analysis condition SQDFA05)

The obtained compound aa003-b (22.66 g) was used to perform a reaction in the same manner as in the synthesis of the compound aa002, and the obtained crude product was purified by reverse phase column chromatography (0.1% formic acid - water/0.1% formic acid - acetonitrile) to obtain a compound aa003 ((2S)-2-[9H-fluoren-9-ylmethoxycarbonyl(methyl)amino]-3-(4-methylphenyl)propanoic acid, Fmoc-MePhe(4-Me)-OH). (17.3 g, 83.5% through two steps).
LCMS (ESI) m/z = 416 (M + H)+
Retention time: 2.41 min (analysis condition SQDFA3080)

### Synthesis of Compound aa004, (2S)-2-[9H-fluoren-9-ylmethoxycarbonyl(methyl)amino]-3-methoxypropanoic acid (Fmoc-MeSer(Me)-OH)

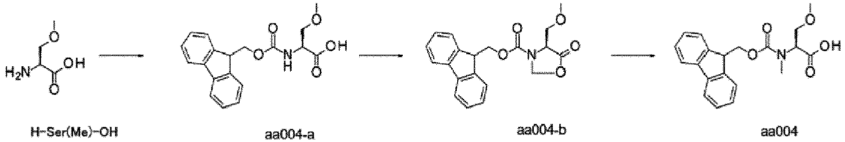

After dissolving commercially available O-methyl-L-serine (H-Ser(Me)-OH) (498 mg, 4.18 mmol) and sodium carbonate (1.329 g, 12.54 mmol) in water (8 mL), a solution of N-(9-fluorenylmethoxycarbonyloxy)succinimide (Fmoc-OSu) (1.41 g, 4.18 mmol) in 1,4-dioxane (12 mL) was added thereto at room temperature, followed by stirring overnight. Water was added to the resultant reaction solution, and the resultant was washed with t-butylmethyl ether/n-hexane = 1/10. To the obtained aqueous layer, 1N hydrochloric acid water was added to obtain acidic pH, followed by extraction with ethyl acetate twice. Then obtained organic layer was washed with saturated saline, dried over anhydrous magnesium sulfate, and filtered, and the solvent was concentrated under reduced pressure to obtain, as a crude product, a compound aa004-a (Fmoc-Ser(Me)-OH) (1.46 g). The obtained compound aa004-a was not further purified but was directly used in the next reaction.

To a solution of the crude product of the compound aa004-a (1.46 g) and paraformaldehyde (377 mg, 12.54 mmol) in toluene (12 mL), trifluoroacetic acid (TFA) (2.90 mL, 37.6 mmol) was added, followed by stirring at room temperature overnight. After concentrating the solvent under reduced pressure, ethyl acetate was added to the resultant, the organic layer was washed with a saturated sodium bicarbonate aqueous solution, and washed with saturated saline, and the resultant organic layer was dried over anhydrous magnesium sulfate, and filtered. The solvent was concentrated from the resultant filtrate under reduced pressure to obtain a crude product of a compound aa004-b (2.13 g). The obtained compound aa004-b was not further purified but was directly used in the next reaction.

To a solution of the crude product of the compound aa004-b (2.13 g) in dichloroethane (DCE) (15 mL), triethylsilane (TES) (6.02 mL, 37.7 mmol) and trifluoroacetic acid (TFA) (8.72 mL, 113 mmol) were added at room temperature, followed by stirring at 60°C for 3 hours. The resultant reaction solution was cooled to room temperature, and the crude product was obtained by concentrating the solvent under reduced pressure, t-butylmethyl ether/n-hexane = 1/4 was added, and a saturated sodium bicarbonate aqueous solution was added thereto, and thus, a white solid was generated. The organic solvent was concentrated under reduced pressure, a solution obtained by adding dimethylsulfoxide to the aqueous layer was purified by reverse phase column chromatography (0.1% formic acid aqueous solution/0.1% formic acid acetonitrile solution) to obtain the compound aa004 ((2S)-2-[9H-fluoren-9-ylmethoxycarbonyl(methyl)amino]-3-methoxypropanoic acid, Fmoc-MeSer(Me)-OH) (782 mg, 53% through three steps).
LCMS (ESI) m/z = 356 (M + H)+
Retention time: 0.76 min (analysis condition SQDFA05)

### Synthesis of Compound aa005, (2S)-3-(4,4-difluorocyclohexyl)-2-[9H-fluoren-9-ylmethoxycarbonyl(methyl)amino] propanoic acid (Fmoc-MeCha(4-F2)-OH)

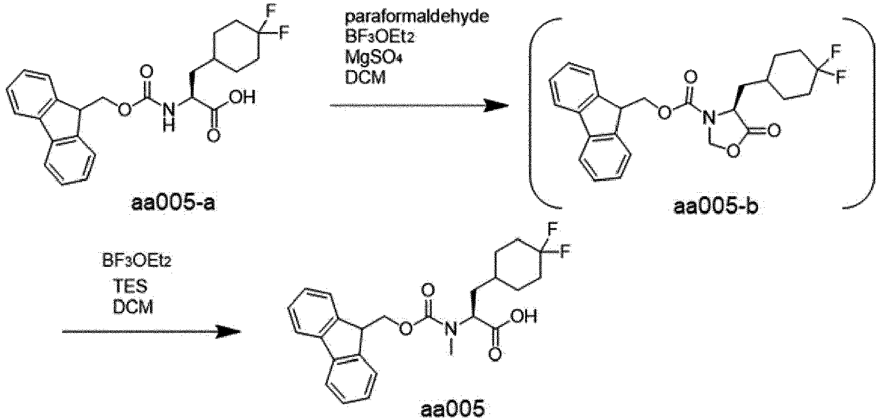

To a solution of a compound aa005-a (S)-2-((((9H-fluoren-9-yl)methoxy)carbonyl)amino)-3-(4,4-difluorocyclohexyl) propanoic acid, Fmoc-Cha(4-F2))-OH) (1.89 g, 4.40 mmol) in DCM (22 mL), paraformaldehyde (0.661 g, 22.00 mmol), magnesium sulfate (1.324 g, 11.00 mmol), and a boron trifluoride diethyl ether complex (BF₃·OEt₂) (0.558 mL, 4.40 mmol) were added under the nitrogen atmosphere, followed by stirring at room temperature for 2 hours. The resulting reaction solution was filtered through celite, and to the resultant filtrate, triethylsilane (2.103 mL, 13.20 mmol), water (0.079 mL, 4.40 mmol), and a boron trifluoride diethyl ether complex (BF₃·OEt₂) (0.837 mL, 6.60 mmol) were added under ice cooling in a nitrogen atmosphere, followed by stirring for 4 hours. To the resulting reaction solution, 50% Brine was added, followed by further stirring at room temperature for 30 minutes. The resultant was filtered with a phase separator, and the organic layer was collected and concentrated under reduced pressure to obtain a crude product. The obtained crude product was dissolved in acetonitrile, the resultant was washed with n-hexane, and the resultant acetonitrile layer was concentrated under reduced pressure to obtain the compound aa005 ((2S)-3-(4,4-difluorocyclohexyl)-2-[9H-fluoren-9-ylmethoxycarbonyl(methyl)amino] propanoic acid, Fmoc-MeCha(4-F2)-OH) (1.89 g, 97%).
LCMS (ESI) m/z = 444 (M + H)+
Retention time: 0.89 min (analysis condition SQDFA05)

### Synthesis of Compound aa006

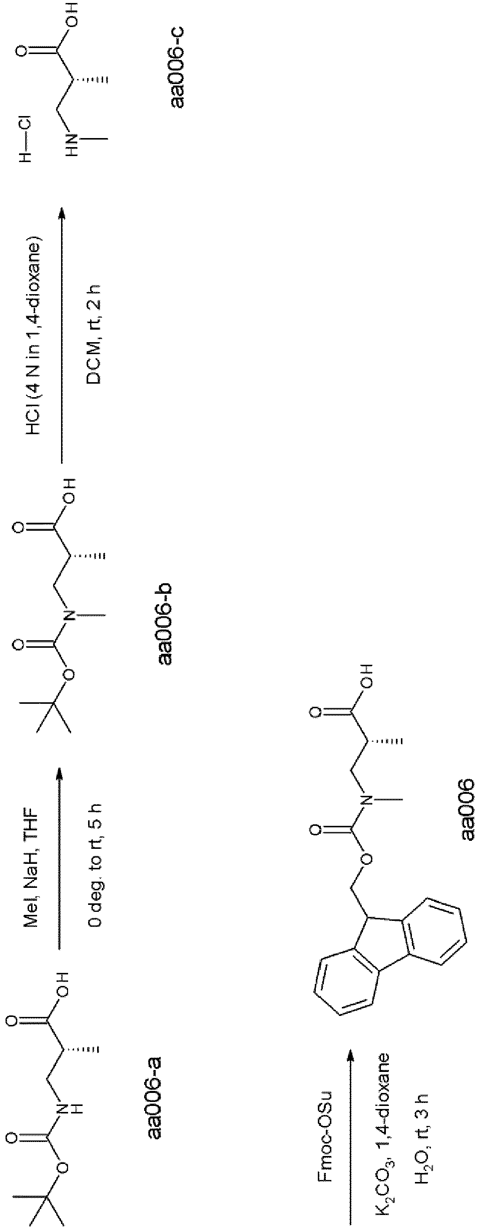

In a nitrogen atmosphere, aa06-a (90 g, 443 mmol) and methyl iodide (314 g, 2.21 mol) were dissolved in tetrahydrofuran (3.15 L). The resulting mixed solution was cooled to 0°C, and sodium hydride (60%, 77.56 g, 2.21 mol) was added thereto under stirring. The resulting reaction solution was stirred at room temperature for 5 hours, and then added to ice water. The resulting solution was washed with t-butylmethyl ether/n-hexane (1/3) three times. To the aqueous phase, 1N hydrochloric acid was added to adjust pH to 2 to 3, and the resultant was extracted with ethyl acetate three times. The resulting organic phase was washed with saturated saline, dried over anhydrous sodium sulfate, and concentrated under reduced pressure to obtain the aa006-b (94 g) as a crude product. The obtained crude product was directly used in the next reaction.
LCMS (ESI) m/z = 240 (M + Na)+
Retention time: 1.1 min (analysis condition SMDmethod_14)

The crude product (94 g) of aa006-b was dissolved in dichloromethane (600 mL). The resultant solution was cooled to 0°C, and 4NHCl/1,4-dioxane (660 mL) was added thereto. The resultant reaction solution was stirred at room temperature for 2 hours, and concentrated under reduced pressure to obtain a crude product (90 g) of aa006-c.
LCMS (ESI) m/z = 118 (M + H)+
Retention time: 0.25 min (analysis condition SMDmethod_14)

The crude product (90 g) of aa006-c was dissolved in water (560 mL), and potassium carbonate was added thereto to adjust pH to 7. Subsequently, potassium carbonate (149 g, 1.08 mol), Fmoc-OSu (131 g, 389 mmol), and 1,4-dioxane (560 mL) were added to the resulting reaction solution, followed by stirring at room temperature for 3 hours. Thereafter, the resultant reaction solution was washed with t-butylmethyl ether/n-hexane (1/3), 1 N hydrochloric acid was added thereto to adjust pH to 2 to 3, and a solid was filtered out. The obtained solid was washed with water, and dried under reduced pressure at 50°C for 16 hours to obtain aa006 (130 g, 86% (through 3 steps)).
LCMS (ESI) m/z = 362 (M + Na)+
Retention time: 2.0 min (analysis condition SMDmethod_15)

### 2. Synthesis of Amino Acid Carrying Resin for Use in Peptide Solid Phase Synthesis with Automatic Synthesizer

A compound aa011 was synthesized by the following method, and was caused to be carried on a resin for use in peptide synthesis with a peptide synthesizer. A compound aa012 was purchased from a commercial supplier, and was caused to be carried on a resin for use in peptide synthesis with a peptide synthesizer. Production of Fmoc amino acid, a carrying reaction of the produced Fmoc amino acid on a resin, and an analysis method were performed in accordance with methods described in WO2013/100132 or WO2018/225864. A 2-chlorotritylchloride resin (100-200 mesh, 1% DVB) was purchased from Watanabe Chemical Industries, Ltd. and Chem-Impex.

**[Table 4]**

| Compound No. | Abbreviation | Structural Formula | Name |
|---|---|---|---|
| aa011 | Fmoc-MeAsp-pip | 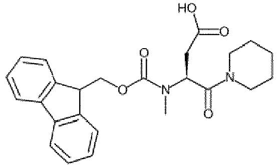 | (3S)-3-[9H-fluoren-9-ylmethoxycarbonyl(methyl)a mino]-4-oxo-4-(1-piperidyl)butanoic acid |
| aa012 | Fmoc-D-3-Abu | 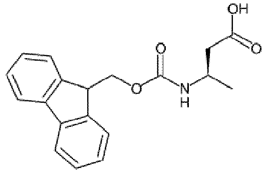 | (3R)-3-(9H-fluoren-9-ylmethoxycarbonylamino)bu tanoic acid |

### Synthesis of Compound aa011-resin, (3S)-3-[9H-fluoren-9-ylmethoxycarbonyl(methyl)amino]-4-oxo-4-piperidine-1-ylbutanoic acid-2-chlorotrityl resin (Fmoc-MeAsp(O-Trt(2-Cl)resin)-pip)

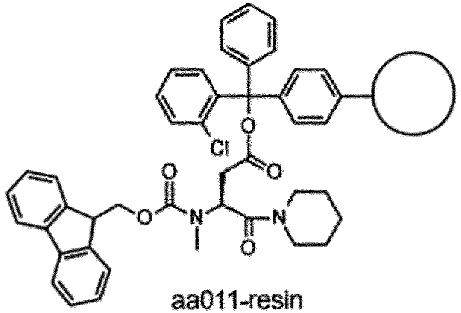

A compound aa011-resin ((3S)-3-[9H-fluoren-9-ylmethoxycarbonyl(methyl)amino]-4-oxo-4-piperidine-1-ylbutanoic acid-2-chlorotrityl resin, Fmoc-MeAsp(O-Trt(2-Cl)resin)-pip) was synthesized by a method described in WO2018/225864.

It is noted that a resin portion is herein shown with a circle in some cases when the resin is bound to a compound. Besides, for purposes of clarifying a reaction point in the resin portion, a chemical structure of the reaction site is shown to be connected to the circle in some cases. In the structure shown above, it is shown that a 2-chlorotrityl group on the resin is bound to MeAsp side chain carboxylic acid via an ester bond in the Fmoc-MeAsp(O-Trt(2-Cl)resin)-pip (compound aa011-resin).

### Synthesis of Compound aa012-resin, (3R)-3-(9H-fluoren-9-ylmethoxycarbonylamino)butanoic acid-2-chlorotrityl resin (Fmoc-D-3-Abu-O-Trt(2-Cl) resin)

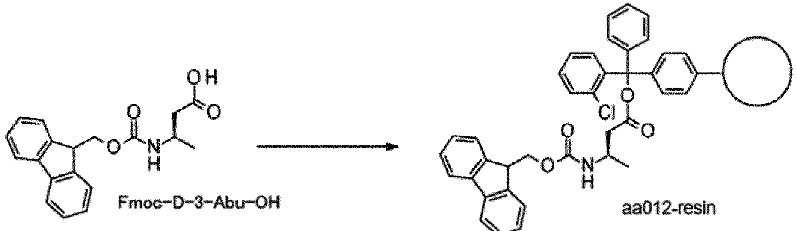

(3R)-3-(9H-fluoren-9-ylmethoxycarbonylamino)butanoic acid (Fmoc-D-3-Abu-OH) (7.1 g, 21.82 mmol) purchased from a commercial supplier, and a 2-chlorotritylchloride resin (1.6 mmol/g, 100-200 mesh, 1% DVB, 27.25 g, 43.6 mmol) were used to obtain a compound aa012-resin, (3R)-3-(9H-fluoren-9-ylmethoxycarbonylamino)butanoic acid-2-chlorotrityl resin (Fmoc-D-3-Abu-O-Trt(2-Cl) resin) in the same manner as in the synthesis of the compound aa011-resin (33.44 g, carried amount: 0.598 mmol/g).

It is noted that a different lot was similarly synthesized but different in the carried amount was also used in the peptide synthesis of this example.

### 3. Peptide Solid Phase Synthesis with Automatic Synthesizer

Cyclic peptide compounds (a compound 1, a compound 3, a compound 4, a compound 6, and a compound 7) (Table 5) were synthesized by a method described in WO2013/100132, WO2018/225864, or WO2021/090855. A peptide synthesizer (Multipep RS; manufactured by Intavis) was used to perform peptide synthesis by an Fmoc method described in detail below, and specific operation procedures were performed in accordance with a manual attached to the synthesizer.

### 1) Peptide Elongation Reaction from Amino Acid N Terminal by Fmoc Method

Each peptide compound was synthesized with a peptide synthesizer manufactured by Intavis (multipep RS or Multipep RSi) by a solid phase synthesis method using Fmoc-protected amino acid. Specific operation procedures were performed in accordance with a manual attached to the synthesizer.

Fmoc-protected amino acid (0.3 to 0.6 mol/L) contained in a target peptide, and HOAt, oxyma, or HOOBt (0.375 mol/L) as an activator for a carboxy group were dissolved in NMP or NMP/DMF (1/1) to prepare a solution 1. When the Fmoc-protected amino acid was difficult to be dissolved in the solvent, DMSO was added to be 20 to 30% (v/v) to prepare the solution 1. N,N'-Diisopropylcarbodiimide (DIC) (10% v/v) and N,N-dimethylformamide (DMF) were mixed to prepare a solution 2.

A 2-chlorotrityl resin (100 mg) having the N terminal bound to a side chain carboxylic acid portion of aspartic acid protected by Fmoc group, or the N terminal bound to a main chain carboxylic acid portion of an amino acid protected by Fmoc group was added to a solid phase reactor, and the resultant was set in the peptide synthesizer. The resin was swollen by adding dichloromethane (DCM) (0.8 mL) to the resin (100 mg), and allowing the resultant to stand still for 1 hour. Thereafter, the resultant solution was discharged from a frit. The solution 1 and the solution 2 were set in the peptide synthesizer, and automatic synthesis with the peptide synthesizer was started.

A solution (2% v/v, 0.7 mL) of diazabicycloundecene (DBU) in DMF was added to the solid phase reactor containing the resin, and deprotection of Fmoc group on the N terminal was performed at room temperature. For deprotection of the first residue, the reaction was performed for 4.5 minutes, and for deprotection of the second and subsequent residues, the reaction was performed for 10 minutes, and then the resultant solution was discharged from the frit. DMF (0.7 mL) was added thereto, the resultant was allowed to stand still for 5 minutes, and the resultant solution was discharged from the frit. This resin washing process was repeated another three times, and thus, an amino acid bound onto the resin, or the resin having the amino group with the Fmoc group of the peptide N terminal removed was obtained.

Subsequently, the solution 1 (0.3 mL) and the solution 2 (0.36 mL) were mixed in a mixing vial of the synthesizer, the resultant mixture was added to the resin resulting from the above-described deprotection treatment, and the solid phase reactor was heated to 40°C. If the sequence was difficult to elongate, the reactor was heated up to 60°C if necessary. A condensation reaction between the amino group on the resin and the Fmoc-protected amino acid was performed for 2.5 hours. If the sequence was difficult to elongate, the reaction was performed for 20 hours if necessary. After the reaction, the resulting solution was discharged from the frit. If elongation efficiency was low, the condensation reaction of the Fmoc-protected amino acid was performed repeatedly further once or twice. Subsequently, the resin was washed with DMF (0.7 mL) three times. Assuming that the condensation reaction of the Fmoc amino acid following the deprotection reaction of the Fmoc group was a single cycle, the cycle was repeated to elongate the peptide on the surface of the resin. After completing the peptide elongation, a solution (2% v/v, 0.7 mL) of diazabicycloundecene (DBU) in DMF was added to the resultant resin for performing a reaction for 15 minutes as Fmoc group deprotection reaction, and then, the resultant solution was discharged from the frit. The thus obtained resin was washed with DMF (0.7 mL) four times, and with DCM (0.7 mL) four times.

### 2) Cut-out of Elongated Peptide from Resin

To a resin obtained by a method described in WO2013/100132, or WO2018/225864, or obtained by the above-described method, 2,2,2-trifluoroethanol (TFE)/DCM (1/1, v/v, 2 mL) containing 0.75% (v/v) DIPEA was added, and a reaction of cutting a peptide chain out from the resin was performed by causing a reaction for 2 hours at room temperature. After the reaction, a solution remaining in the tube was collected from the frit. An operation of adding 2,2,2-trifluoroethanol (TFE)/DCM (1/1, v/v, 1 mL) to the remaining resin and collecting the solution from the frit was performed twice. All of the thus obtained cut solutions were mixed, and DMF (4 mL) or 1,2-dichloroethane (4 mL) was mixed therewith, and then the solvent was distilled off under reduced pressure with a high-throughput centrifugation evaporator (HT-12) manufactured by Genevac.

### 3) Cyclization of Cut-out Peptide

The residue obtained by the above-described method was dissolved in a mixed solution of DMF (4 mL) and DCM (4 mL), a solution (0.5 M, 1.5 equivalents) of (1-cyano-2-ethoxy-2-oxoethylidenaminooxy) dimethylamino-morpholino-carbenium hexafluorophosphate (COMU) in DMF, or a solution (0.5 M, 1.5 equivalents) of O-(7-aza-1H-benzotriazol-1-yl)-N,N,N,N-tetramethyluronium hexafluorophosphate (HATU) in DMF, and DIPEA (1.8 equivalents) were added thereto, and the resultant was stirred at room temperature for 2 hours to perform a condensation cyclization reaction between an N-terminal amino group and a C-terminal carboxy group. Each equivalent number was calculated based on a value obtained by multiplying a ratio of amino acid carried (mmol/g) in the resin used as a raw material by the amount of the used resin (usually 100 mg). Generation of a target cyclic peptide was checked by LC/MS measurement (SQ Detector 2 manufactured by Waters), and then the solvent was concentrated under reduced pressure from the resultant reaction solution with a high-throughput centrifugation evaporator (HT-12) manufactured by Genevac.

### 4) Method for Purifying Cyclic Peptide

To the residue obtained by the above-described method, DMF or DMSO was added, and after removing an insoluble matter by filtration, the resultant was purified by preparative-HPLC to obtain a target cyclic peptide. Waters Auto Purification System was used as the purification apparatus, YMC-Actus Triart C18 (20 mm in inner diameter, 100 mm in length) or YMC-Actus Triart Phenyl (20 mm in inner diameter, 100 mm in length) was used as the column, and a methanol-ammonium acetate aqueous solution (50 mmol/L) or acetonitrile-water containing 0.1% formic acid was used as the mobile phase.

### Example 1 Synthesis of Compound 1 ((3S,6S,9S,12S,18S,27S,30S,34S)-9-(cyclohexylmethyl)-12-[(3-iodophenyl)methyl]-3,30-diisobutyl-6-(methoxymethyl)-1,7,16,19,22,25,31-heptamethyl-27-[(1S)-1-methylpropyl]-34-(piperidine-1-carbonyl)-18-(p-tolylmethyl)-1,4,7,10,13,16,19,22,25,28,31-undecazacyclotetratriacontane-2,5,8,11,14,17,20,23,26,29,32-undecaone)

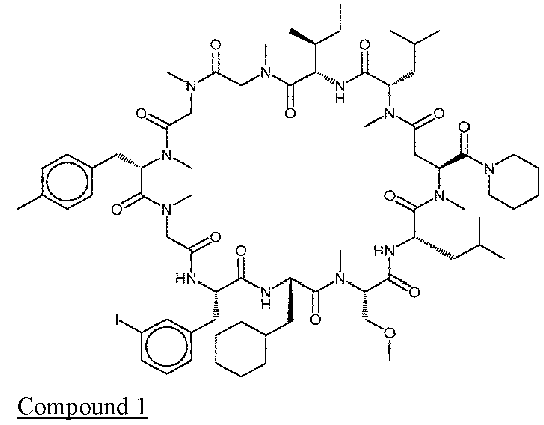

Using the compound aa011-resin ((3S)-3-[9H-fluoren-9-ylmethoxycarbonyl(methyl)amino]-4-oxo-4-piperidine-1-ylbutanoic acid-2-chlorotrityl resin, Fmoc-MeAsp(O-Trt-(2-Cl) resin)-pip) (0.462 mmol/g, 100 mg, 0.0462 mmol) as a raw material, and using Fmoc-Leu-OH, Fmoc-MeSer(Me)-OH, Fmoc-Cha-OH, Fmoc-Phe(3-I)-OH, Fmoc-MeGly-OH, Fmoc-MePhe(4-Me)-OH, Fmoc-MeGly-OH, Fmoc-MeGly-OH, Fmoc-Ile-OH, and Fmoc-MeLeu-OH, the peptide elongation reaction by the Fmoc method, the cut-out of an elongated peptide from the resin, the cyclization of a cut out peptide (using, as a cyclization reagent, O-(7-aza-1H-benzotriazol-1-yl)-N,N,N,N-tetramethyluronium hexafluorophosphate (HATU)), and the purification of a cyclic peptide were performed to obtain a target compound 1 (11.69 mg, 17.1%).

### Example 2 Synthesis of Compound 2 ((1S,6S,9S,15S,18S21S24S27S31S34S)-21-benzyl-34-cyclobutyl-9-[(4,4-difluorocyclohexyl)methyl]-15-[(3-iodophenyl)methyl]-24,31-diisobutyl-8,11,20,26,30-pentamethyl-18-(3-methylbut-2-enyl)-27-(piperidine-1-carbonyl)-3,8,11,14,17,20,23,26,30,33,36-undecazatricyclo[34.4.0.03,6]tetracontane-2,7,10,13,16,192225293235-undecaone)

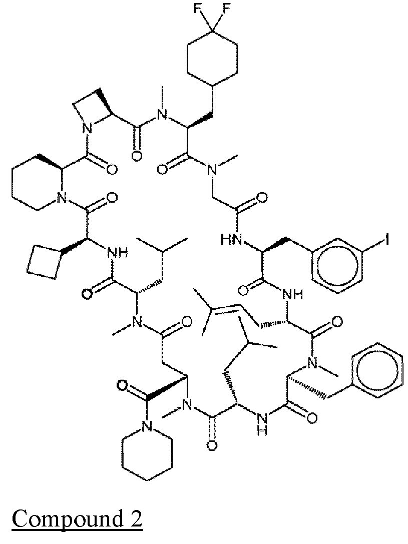

The compound aa011-resin ((3S)-3-[9H-fluoren-9-ylmethoxycarbonyl(methyl)amino]-4-oxo-4-piperidine-1-ylbutanoic acid-2-chlorotrityl resin, Fmoc-MeAsp(O-Trt(2-Cl) resin)-pip) was used as a raw material, and peptide chain was elongated by the general peptide elongation method described herein in the order of Fmoc-Leu-OH, Fmoc-MePhe-OH, Fmoc-Pregly-OH, Fmoc-Phe(3-I)-OH, Fmoc-MeGly-OH, Fmoc-MeCha(4-F2)-OH, Fmoc-Aze(2)-OH, Fmoc-Pic(2)-OH, and Fmoc-Gly(cBu)-OH. With the Fmoc group at the N terminal left, the next operation was performed.

The resulting resin was washed successively with DMF (0.7 mL × 4 times), DCM (0.7 mL × twice), and toluene (0.7 mL × twice). To the resultant, a solution (2% v/v, 0.7 mL) of diazabicycloundecene (DBU) in toluene was added, and a reaction was performed for 10 minutes for deprotection of the Fmoc group. The resulting solution was discharged from the frit, and then the resin was washed successively with toluene (0.7 mL × twice), and DCM (0.7 mL × twice).

To the resin obtained as described above, a solution, in DCM (0.7 mL), of Fmoc-MeLeu-OH (49 mg, 0.134 mmol, 4 equivalents), [ethylcyano(hydroxyimino)acetate-O2]tri-1-pyrrolidinylphosphonium hexafluorophosphate (PyOxim) (70.6 mg, 0.134 mmol, 4 equivalents), and DIPIEA (0.035 mL, 0.2 mmol, 6 equivalents) was added, and the resultant was allowed to stand still at room temperature for 3 hours. After discharging the resultant solution from the frit, the resin was washed with DCM (0.7 mL × three times), and further with DMF (0.7 mL × three times). To the resultant resin, a solution (2% v/v, 0.7 mL) of diazabicycloundecene (DBU) in DMF was added, a reaction was performed for 15 minutes for deprotection of the Fmoc group, and then, the resultant solution was discharged from the frit. The thus obtained resin was washed with DMF (0.7 mL × four times), and then with DCM (1.25 mL × four times).

Onto the resin obtained by the above-described method, 2,2,2-trifluoroethanol (TFE)/DCM (1/1, v/v, 2 mL) containing 0.75% DIPEA was added, and the resultant was shaken at room temperature for 2 hours for performing a reaction of cutting a peptide chain out of the resin. After the reaction, the solution remaining in the tube was collected from the frit. An operation of adding 2,2,2-trifluoroethanol (TFE)/DCM (1/1, v/v, 1 mL) to the remaining resin, and collecting the solution from the frit was performed twice. All of the thus obtained cut solutions were mixed, and DMF (4 mL) was added thereto, and then the solvent was concentrated under reduced pressure with a high-throughput centrifugation evaporator (HT-12) manufactured by Genevac.

The residue obtained by the above-described method was dissolved in a mixed solution of DMF (4 mL) and DCM (4 mL), a solution (0.5 M, 0.1 mL) of (1-cyano-2-ethoxy-2-oxoethylidenaminooxy) dimethylamino-morpholino-carbenium hexafluorophosphate (COMU) in DMF, and DIPEA (0.015 mL) were added thereto, and the resultant was stirred at room temperature for 2 hours to perform a condensation cyclization reaction between an N-terminal amino group and side chain carboxylic acid. Generation of a target cyclic peptide was checked by LC/MS measurement (SQ Detector 2 manufactured by Waters), and then the solvent was distilled off from the resultant reaction solution under reduced pressure with a high-throughput centrifugation evaporator (HT-12) manufactured by Genevac.

DMF was added to the residue obtained by the above-described method, an insoluble matter was removed by filtration, and the resultant was purified by preparative-HPLC (with Waters Auto Purification System, using YMC-Actus Triart Phenyl (20 mm in inner diameter, 100 mm in length) as a column, and a methanol-ammonium acetate aqueous solution (50 mmol/L) as a mobile phase) to obtain a target compound 2 (3.49 mg, 4.6%).

### Example 3 Synthesis of Compound 3 ((3S,6S,9S,12S,18S,27S,30S,34S)-9-butyl-12-[(3-iodol)henyl)methyl]-3-isobulyl-30-(2-methoxyethyl)-6-(methoxymethyl)-1,7,16,19,22,25,31-heptamethyl-27-[(1S)-1-methylpropyl]-34-(piperidine-1-carbonyl)-18-(p-tolylmethyl)-1,4,7,10,13,16,19,22,25,28,31-undecazacyclotetratriacontane-2,5,8,11,14,17,20,23,26,29,32-undecaone)

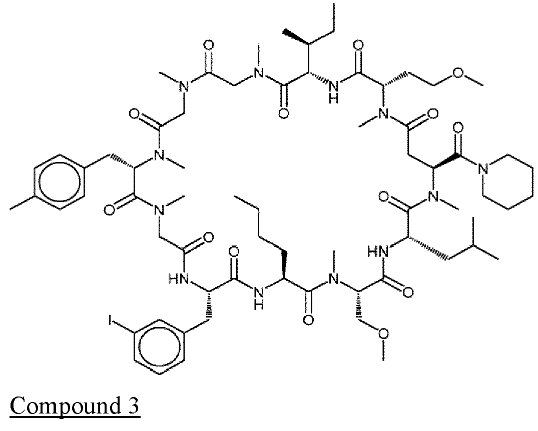

A compound 3 was produced in the same manner as in the method for producing the compound 1.

### Example 4 Synthesis of Compound 4 ((3S,9S,12S,18S,27S,30R,35S)-18-(cyclohexylmethyl)-12-[(3-ethynylphenyl)methyl]-3-isobutyl-9-[(4-methoxyphenyl)methyl]-1,6,6,7,16,19,22,25,30,32-decamethyl-27-[(1S)-1-methylpropyl]-35-(piperidine-1-carbonyl)-1,4,7,10,13,16,19,22,25,28,32-undecazacyclopentatriacontane-2,5,8,11,14,17,20,23,26,29,33-undecaone)

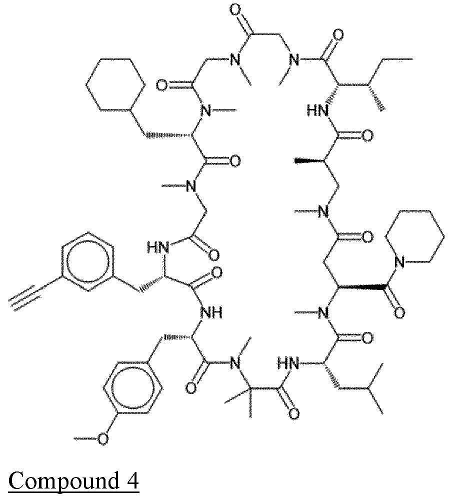

A compound 4 was produced in the same manner as in the method for producing the compound 1 to obtain the target compound 4 (14.72 mg, 26%).

### Comparative Example 1 Synthesis of Compound 5 ((1S,6S,9S,15S,18S,24S,27S,31S,34S)-18-butyl-34-cyclobutyl-15-[(2,3-difluorophenyl)methyl]-24,31-diisobutyl-9-[(4-methoxyphenyl)methyl]-8,11,20,21,21,26,30-heptamethyl-27-(piperidine-1-carbonyl)-3,8,11,14,17,20,23,26,30,33,36-undecazatricyclo[34.4.0.03,6]tetracontane-2,7,10,13,16,19,22,25,29,32,3 5-undecaone)

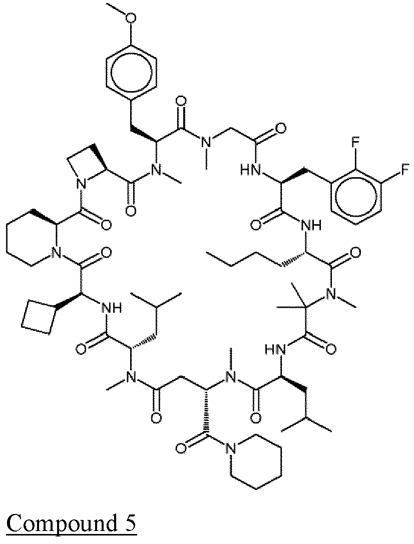

The compound aa011-resin ((3S)-3-[9H-fluoren-9-ylmethoxycarbonyl(methyl)amino]-4-oxo-4-piperidine-1-ylbutanoic acid-2-chlorotrityl resin, Fmoc-MeAsp(O-Trt(2-Cl) resin)-pip) was used as a raw material, and peptide chain was elongated by the general peptide elongation method described herein in the order of Fmoc-Leu-OH, Fmoc-MeAib-OH, Fmoc-Nle-OH, Fmoc-Phe(23-F2)-OH, Fmoc-MeGly-OH, Fmoc-MeTyr(Me)-OH, Fmoc-Aze(2)-OH, Fmoc-Pic(2)-OH, and Fmoc-Gly(cBu)-OH. With the Fmoc group at the N terminal left, the next operation was performed.

The resulting resin was washed successively with DMF (0.7 mL x 4 times), DCM (0.7 mL x twice), and toluene (0.7 mL x twice). To the resultant, a solution (2% v/v, 0.7 mL) of diazabicycloundecene (DBU) in toluene was added, and a reaction was performed for 10 minutes for deprotection of the Fmoc group. The resultant solution was discharged from the frit, and then the resin was washed successively with toluene (0.7 mL x twice), and DCM (0.7 mL x twice).

To the resin obtained as described above, a solution, in DCM (0.7 mL), of Fmoc-MeLeu-OH (49 mg, 0.134 mmol, 4 equivalents), [ethylcyano(hydroxyimino)acetate-O2]tri-1-pyrrolidinylphosphonium hexafluorophosphate (PyOxim) (70.6 mg, 0.134 mmol, 4 equivalents), and DIPIEA (0.035 mL, 0.2 mmol, 6 equivalents) was added, and the resultant was allowed to stand still at room temperature for 3 hours. After discharging the resultant solution from the frit, the resin was washed with DCM (0.7 mL x three times), and further with DMF (0.7 mL x three times). To the resultant resin, a solution (2% v/v, 0.7 mL) of diazabicycloundecene (DBU) in DMF was added, a reaction was performed for 15 minutes for deprotection of the Fmoc group, and then, the resultant solution was discharged from the frit. The thus obtained resin was washed with DMF (0.7 mL x four times), and then with DCM (1.25 mL x four times).

Onto the resin obtained by the above-described method, 2,2,2-trifluoroethanol (TFE)/DCM (1/1, v/v, 2 mL) containing 0.75% DIPEA was added, and the resultant was shaken at room temperature for 2 hours for performing a reaction of cutting a peptide chain out of the resin. After the reaction, the solution remaining in the tube was collected from the frit. An operation of adding 2,2,2-trifluoroethanol (TFE)/DCM (1/1, v/v, 1 mL) to the remaining resin, and collecting the solution from the frit was performed twice. All of the thus obtained cut solutions were mixed, and DMF (4 mL) was added thereto, and then the solvent was concentrated under reduced pressure with a high-throughput centrifugation evaporator (HT-12) manufactured by Genevac.

The residue obtained by the above-described method was dissolved in a mixed solution of DMF (4 mL) and DCM (4 mL), a solution (0.5 M, 0.1 mL) of (1-cyano-2-ethoxy-2-oxoethylidenaminooxy) dimethylamino-morpholino-carbenium hexafluorophosphate (COMU) in DMF, and DIPEA (0.015 mL) were added thereto, and the resultant was stirred at room temperature for 2 hours to perform a condensation cyclization reaction between an N-terminal amino group and side chain carboxylic acid. Generation of the target cyclic peptide was checked by LC/MS measurement (SQ Detector 2 manufactured by Waters), and then the solvent was concentrated from the resultant reaction solution under reduced pressure with a high-throughput centrifugation evaporator (HT-12) manufactured by Genevac.

DMF was added to the residue obtained by the above-described method, an insoluble matter was removed by filtration, and the resultant was purified by preparative-HPLC (with Waters Auto Purification System, using YMC-Actus Triart Phenyl (20 mm in inner diameter, 100 mm in length) as a column, and a methanol-ammonium acetate aqueous solution (50 mmol/L) as a mobile phase) to obtain a target compound 5 (16.92 mg, 29%).

### Comparative Example 2 Synthesis of Compound 6 ((3S,9S,12S,17S,20S,23S,27R,30S,36S)-3-[2-[3-chloro-4-(trifluoromethyl)phenyl]ethyl]-9-(cyclohexylmethyl)-23-isobutyl-30-isopropyl-7,10,17,18,24,27,31-heptamethyl-20-[(S)-1-methylpropyl]spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecaone)

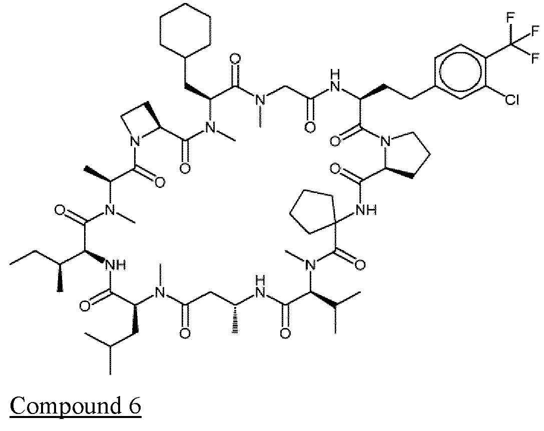

A compound 6 was produced in the same manner as in the method for producing the compound 1. Thus, the target compound 6 (4.41 mg, 11%) was obtained.

### Comparative Example 3 Synthesis of Compound 7 ((11S,17S,26S,29S,33S,36S)-11-[2-[3-chloro-4-(trifluoromethyl)phenyl]ethyl]-36-cyclohexyl-17-(cyclohexylmethyl)-29-isobutyl-15,18,21,24,30,34,37-heptamethyl-26-[(1S)-1-methylpropyl]-9-(2-piperazine-1-ylethyl)-33-(piperidine-1-carbonyl)-6,9,12,15,18,21,24,27,30,34,37-undecazaspiro[4.33]octatriacontane-7,10,13,16,19,22,25,28,31,35,38-undecaone)

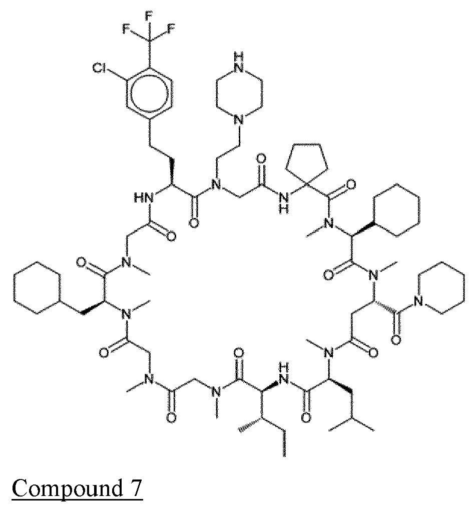

A compound 7 was produced in the same manner as in the production method of WO2021090855 (compound 2184).

**[Table 5]**

| Compound No. | Structural Formula | Compound No. | Structural Formula |
|---|---|---|---|
| 1 | 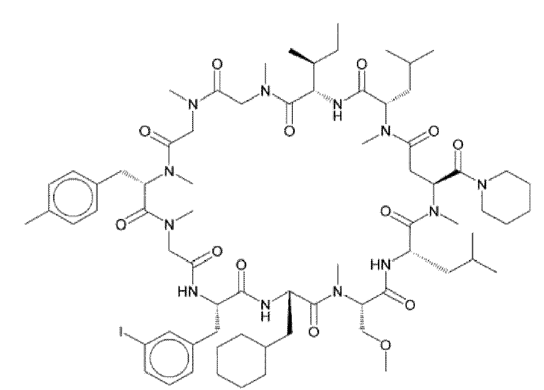 | 3 | 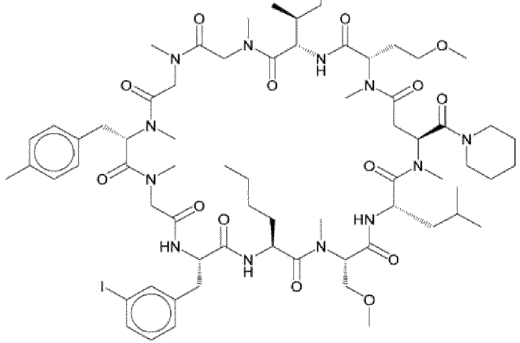 |
| 2 | 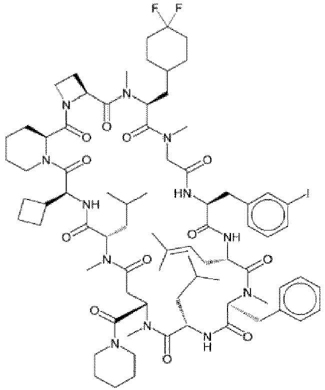 | 4 | 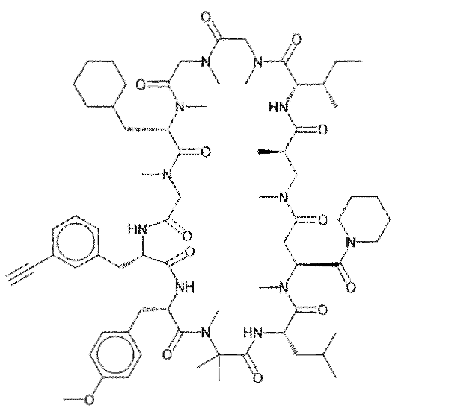 |
| 5 | 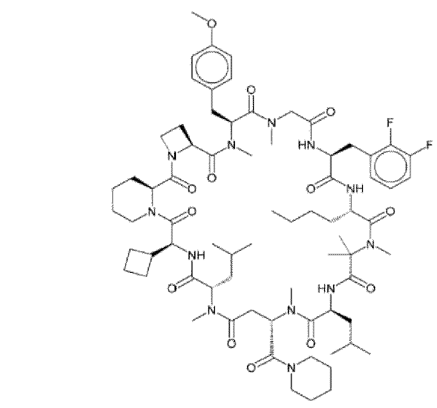 | 7 | 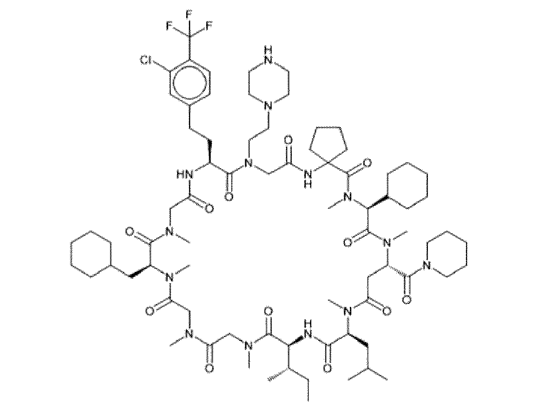 |
| 6 | 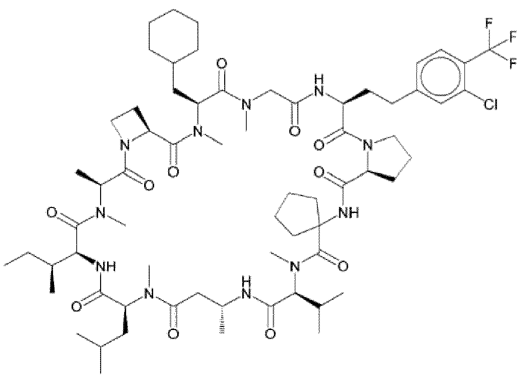 | | |

Compound 1: (3S,6S,9S,12S,18S,27S,30S,34S)-9-(cyclohexylmethyl)-12-[(3-iodophenyl)methyl]-3,30-diisobutyl-6-(methoxymethyl)-1,7,16,19,22,25,31-heptamethyl-27-[(1S)-1-methylpropyl]-34-(piperidine-1-carbonyl)-18-(p-tolylmethyl)- 1,4,7,10,13, 16, 19,22,25,28,31-undecazacyclotetratriacontane-2,5,8, 11, 14, 17,20,23,26,29,32-undecaone (molecular weight: 1479.6, CLogP: 15.1)
Compound 2: (1S,6S,9S,15S,18S,21S,24S,27S,31S,34S)-21-benzyl-34-cyclobutyl-9-[(4,4-difluorocyclohexyl)methyl]-15-[(3-iodophenyl)methyl]-24,31-diisobutyl-8,11,20,26,30-pentamethyl-18-(3-methylbut-2-enyl)-27-(piperidine-1-carbonyl)-3,8,11,14,17,20,23,26,30,33,36-undecazatricyclo[34.4.0.03,6]tetracontane-2,7,10,13,16,19,22,25,29,32,35-undecaone (molecular weight: 1575.7, CLogP: 15.8)
Compound 3: (3S,6S,9S,12S,18S,27S,30S,34S)-9-butyl-12-[(3-iodophenyl)methyl]-3-isobutyl-30-(2-methoxyethyl)-6-(methoxymethyl)-1,7,16,19,22,25,31-heptamethyl-27-[(1S)-1-methylpropyl]-34-(piperidine-1 -carbonyl)-18-(p-tolylmethyl)-1,4,7,10,13,16,19,22,25,28,31-undecazacyclotetratriacontane-2,5,8,11,14,17,20,23,26,29,32-undecaone (molecular weight: 1441.5, CLogP: 11.8)
Compound 4: (3S,9S,12S,18S,27S,30R,35S)-18-(cyclohexylmethyl)-12-[(3-ethynylphenyl)methyl]-3-isobutyl-9-[(4-methoxyphenyl)methyl]-1,6,6,7,16,19,22,25,30.32-decamethyl-27-[(1S)-1-methylpropyl]-35-(piperidine-1-carbonyl)-1,4,7,10,13,16,19,22,25,28,32-undecazacyclopentatriacontane-2,5,8,11,14,17,20,23,26,29,33-undecaone (molecular weight: 1349.7, CLogP: 12.5)
Compound 5: (1S,6S,9S,15S,18S,24S,27S,31S,34S)-18-butyl-34-cyclobutyl-15-[(2,3-difluorophenyl)methyl]-24,31-diisobutyl-9-[(4-methoxyphenyl)methyl]-8,11,20,21,21,26,30-heptamethyl-27-(piperidine-1-carbonyl)-3,8, 11, 14, 17,20,23,26,30,33,36-undecazatricyclo[34.4.0.03,6]tetracontane-2,7,10,13,16,19,22,25,29,32,35-undecaone (molecular weight: 1399.7, CLogP: 13.4)
Compound 6: (3S,9S,12S,17S,20S,23S,27R,30S,36S)-3-[2-[3-chloro-4-(trifluoromethyl)phenyl]ethyl]-9-(cyclohexylmethyl)-23-isobutyl-30-isopropyl-7,10,17,18,24,27,31-heptamethyl-20-[(1S)-1-methylpropyl]spiro[1,4,7,10,15,18,21,24,28,31,34-undecazatricyclo[34.3.0.012,15]nonatriacontane-33,1'-cyclopentane]-2,5,8,11,16,19,22,25,29,32,35-undecaone (molecular weight: 1317.0, CLogP: 14.2)
Compound 7: (11S,17S,26S,29S,33S,36S)-11-[2-[3-chloro-4-(trifluoromethyl)phenyl]ethyl]-36-cyclohexyl-17-(cyclohexylmethyl)-29-isobutyl-15,18,21,24,30,34,37-heptamethyl-26-[(1S)-1-methylpropyl]-9-(2-piperazine-1-ylethyl)-33-(piperidine-1-carbonyl)-6,9,12,15,18,21,24,27,30,34,37-undecazaspiro[4.33]octatriacontane-7,10,13,16,19,22,25,28,31,35,38-undecaone (molecular weight: 1514.3, CLogP: 15.7)

**[Table 6]**

| **Compound No.** | **Measurement Conditions** | **Retention Time (min)** | **MS Found(m/z)** | **MS polarity** |
|---|---|---|---|---|
| 1 | SSC-A-FA-01 | 6.685 | 1478.1 | (M-H)- |
| 2 | SSC-A-FA-01 | 6.745 | 1574.3 | (M-H)- |
| 3 | SSC-A-FA-01 | 5.564 | 1439.6 | (M-H)- |
| 4 | SSC-A-AF-01 | 7.212 | 1347.7 | (M-H)- |
| 5 | SSC-A-FA-01 | 5.425 | 1397.8 | (M-H)- |
| 6 | SSC-A-FA-01 | 6.063 | 1316.6 | (M+H)+ |
| 7 | SSC-A-AF-01 | 7.564 | 1513.9 | (M+H)+ |

### Evaluation Example 1 X-ray Crystal Structure Analysis of Complex of KRASG12D and Peptide Compound

### 1. Production of Human KRASG12D Mutant Protein

Regarding human KRASG12D mutant protein [created based on Uniprot ID: P01116, Isoform 2B], a construct HATnorTh-KRasG12D in which HAT (histidine affinity tag) sequence and thrombin recognition sequence were added to the N terminal of an expression region 2-174 was designed. The amino acid sequence is shown in SEQ ID NO: 1. The designed gene was incorporated into pETBlue-1 vector to be transformed into *E.coli* strain Tuner(DE3)pLacI [Novagen] for using *E.coli* expression system.

The recombinant *E. coli* having the target vector was subjected to shaking culture in LB (lysogeny broth) medium containing Ampicillin at 37°C until OD (optical density) of 0.6 was obtained. Thereafter, the culture temperature was lowered to 30°C, IPTG (isopropyl β-D-1-thiogalactopyranoside) was added to 0.1 mM for each of the compounds 3, 4, and 7 to be cultured for 16 hours, and to 1.0 mM for each of the compounds 1, 2, 5, and 6 to be cultured for 24 hours, and thus, expression of the target protein was induced.

The cultured *E.coli* was collected by centrifugation. The collected bacterial cells were suspended in an extraction buffer of 50 mM Tris pH 8.0, 150 mM NaCl, 1% CHAPS (3-[(3-cholamidopropyl)-dimethylammonio]-1-propanesulfate)), 5% glycerol, Complete Protease Inhibitor (EDTA (ethylenediaminetetraacetic acid)-Free) for each of the compounds 1 to 5 and 7, and of 50 mM Tris pH 8.0, 400 mM NaCl, 1% CHAPS, 5% glycerol, 1 mM DTT (dithiothreitol), Complete Protease Inhibitor (EDTA-Free) for the compound 6. To each of the bacterial cell suspensions, lysozyme was added, the resultant was incubated at room temperature for 20 minutes, and the resultant bacterial cells were disrupted by ultrasonic disruption. For each of the compounds 1 to 5 and 7, MgCl₂ (magnesium chloride hexahydrate) and benzonase were added to the liquid resulting from the disruption. The resultant was allowed to stand still at room temperature for 20 minutes, and then a supernatant was collected by centrifugation, and was filtered.

The supernatant of the bacterial cell disrupted solution thus obtained was purified under the following conditions:

### [Compound 1]

The supernatant of the bacterial cell disrupted solution was purified using cOmplete His-Tag Purification Column [Roche 6781543001] and BioLogic DuoFlow [Bio-Rad]. After equilibrating a column with Buffer A (50 mM Tris-HCl, 400 mM NaCl, 1% CHAPS, 5% glycerol, 1 mM DTT, pH 8.0), the supernatant of the bacterial cell disrupted solution was loaded into the column, and human KRasG12D mutant protein was adsorbed onto the column. After washing the column with Buffer A, Buffer A and Buffer B (50 mM Tris-HCl, 400 mM NaCl, 1% CHAPS, 5% glycerol, 1 M imidazole, 1 mM DTT, pH 8.0) were caused to flow in the form of a mixture with concentration gradient, and thus the human KRasG12D mutant protein was eluted. An elution fraction containing the human KRasG12D mutant protein was concentrated, thrombin [Sigma T6634] was added thereto, and the resultant was dialyzed with a dialysis buffer (50 mM Tris-HCl, 400 mM NaCl, 1 mM DTT, pH 8.0) at 15°C, and thus, a HAT (histidine affinity tag) tag added for purification was cleaved. A ratio between an outer liquid and an inner liquid of the dialysis was set to 100:1. Next, to a sample resulting from the dialysis, NaCl was added to a final concentration of 0.8 M, and Benzamidine Sepharose 4 Fast Flow [GE Healthcare 17-5123-10] equilibrated with Buffer C (50 mM Tris-HCl, 800 mM NaCl, 1 mM DTT, pH 8.0) was further added thereto. After 2 hours, Benzamide Sepharose 4 Fast Flow was removed by centrifugation, Buffer D (150 mM Tris-HCl, pH 7.6, 1.5 M arginine-HCl) was added to the resultant to an arginine concentration of 0.5 M, and the resultant was concentrated.

Finally, SEC (size exclusion chromatography) purification was performed using cOmplete His-Tag-Purification Column connected to HiLoad 26/600 Superdex 75 pg [GE Healthcare 28-9893-34], and BioLogic DuoFlow.

### [Compound 2]

The supernatant of the bacterial cell disrupted solution was purified using cOmplete His-Tag Purification Column [Roche 6781535001] and BioLogic DuoFlow [Bio-Rad]. After equilibrating a column with Buffer A (50 mM Tris-HCl, 400 mM NaCl, 1% CHAPS, 5% glycerol, 1 mM DTT, pH 8.0), the supernatant of the bacterial cell disrupted solution was loaded into the column, and human KRasG12D mutant protein was adsorbed onto the column. After washing the column with Buffer A, Buffer A and Buffer B (50 mM Tris-HCl, 400 mM NaCl, 1% CHAPS, 5% glycerol, 1 M imidazole, 1 mM DTT, pH 8.0) were caused to flow in the form of a mixture with concentration gradient, and thus the human KRasG12D mutant protein was eluted. An elution fraction containing the human KRasG12D mutant protein was concentrated, thrombin [Sigma T6634] was added thereto, and the resultant was dialyzed with a dialysis buffer (50 mM Tris-HCl, 400 mM NaCl, 1 mM DTT, pH 8.0) at 15°C, and thus, a HAT (histidine affinity tag) tag added for purification was cleaved. A ratio between an outer liquid and an inner liquid of the dialysis was set to 100:1. Next, to a sample resulting from the dialysis, NaCl was added to a final concentration of 0.8 M, and Benzamidine Sepharose 4 Fast Flow [GE Healthcare 17-5123-10] equilibrated with Buffer C (50 mM Tris-HCl, 800 mM NaCl, 1 mM DTT, pH 8.0) was further added thereto. After 2 hours, Benzamidine Sepharose 4 Fast Flow was removed by centrifugation, Buffer D (150 mM Tris-HCl, pH 7.6, 1.5 M Arginine-HCl) was added to the resultant to an arginine concentration of 0.5 M, and the resultant was concentrated.

Finally, SEC (size exclusion chromatography) purification was performed using cOmplete His-Tag-Purification Column connected to HiLoad 26/600 Superdex 75 pg [GE Healthcare 28-9893-34] and BioLogic DuoFlow.

### [Compound 3]

The supernatant of the bacterial cell disrupted solution was purified with HisTrap excel 5 mL [GE Healthcare 17-3712-06]. After adsorbing human KRasG12D mutant protein onto a column, thrombin was injected into the column. A HAT tag for purification added to the human KRasG12D mutant protein was cleaved with thrombin, and the human KRasG12D mutant protein alone was eluted. Finally, SEC (size exclusion chromatography) purification using HiLoad 26/600 Superdex 75 pg [GE Healthcare 28-9893-34] was performed.

### [Compound 4]

The supernatant of the bacterial cell disrupted solution was purified with Ni Sepharose excel [GE Healthcare 17-3712-01]. After adsorbing human KRasG12D mutant protein onto a column, thrombin was injected into the column. A HAT tag for purification added to the human KRasG12D mutant protein was cleaved with thrombin, and the human KRasG12D mutant protein alone was eluted. Finally, SEC (size exclusion chromatography) purification using HiLoad 26/600 Superdex 75 pg [GE Healthcare 28-9893-34] was performed.

### [Compound 5]

The supernatant of the bacterial cell disrupted solution was purified using a nickel column and NGC chromatography system [Bio-Rad]. After equilibrating a column with Buffer A (50 mM Tris-HCl, 400 mM NaCl, 1% CHAPS, 5% glycerol, 1 mM DTT, pH 8.0), the supernatant of the bacterial cell disrupted solution was loaded into the column, and human KRasG12D mutant protein was adsorbed onto the column. After washing the column with Buffer A, Buffer A and Buffer B (50 mM Tris-HCl, 400 mM NaCl, 1% CHAPS, 5% glycerol, 1 M imidazole, 1 mM DTT, pH 8.0) were caused to flow in the form of a mixture with concentration gradient, and thus the human KRasG12D mutant protein was eluted. An elution fraction containing the human KRasG12D mutant protein was concentrated, thrombin [GE Healthcare 27-0846-01] was added thereto, and the resultant was dialyzed with a dialysis buffer (50 mM Tris-HCl, 400 mM NaCl, 1 mM DTT, pH 8.0) at 20°C, and thus, a HAT tag added for purification was cleaved. A ratio between an outer liquid and an inner liquid of the dialysis was set to 100:1. Next, to a sample resulting from the dialysis, NaCl was added to a final concentration of 0.8 M, and Benzamidine Sepharose 4 Fast Flow [GE Healthcare 17-5123-10] equilibrated with Buffer C (50 mM Tris-HCl, 800 mM NaCl, 1 mM DTT, pH 8.0) was further added thereto. After 2 hours, Benzamidine Sepharose 4 Fast Flow was removed by centrifugation, and the resultant was concentrated.

Finally, SEC (size exclusion chromatography) purification was performed using a gel filtration column and the NGC chromatography system.

### [Compound 6]

The supernatant of the bacterial cell disrupted solution was purified using a nickel column and the NGC chromatography system. After equilibrating a column with Buffer A (50 mM Tris-HCl, 400 mM NaCl, 1% CHAPS, 5% glycerol, 1 mM DTT, pH 8.0), the supernatant of the bacterial cell disrupted solution was loaded into the column, and human KRasG12D mutant protein was adsorbed onto the column. After washing the column with Buffer A, Buffer A and Buffer B (50 mM Tris-HCl, 400 mM NaCl, 1% CHAPS, 5% glycerol, 1 M imidazole, 1 mM DTT, pH 8.0) were caused to flow in the form of a mixture with concentration gradient, and thus the human KRasG12D mutant protein was eluted. An elution fraction containing the human KRasG12D mutant protein was concentrated, thrombin [GE Healthcare 27-0846-01] was added thereto, and the resultant was dialyzed with a dialysis buffer (50 mM Tris-HCl, 400 mM NaCl, 1 mM DTT, pH 8.0) at 20°C, and thus, a HAT tag added for purification was cleaved. A ratio between an outer liquid and an inner liquid of the dialysis was set to 100:1. Next, to a sample resulting from the dialysis, NaCl was added to increase a salt concentration, and Benzamidine Sepharose 4 Fast Flow [GE Healthcare 17-5123-10] equilibrated with Buffer C (one obtained by adding NaCl to 50 mM Tris-HCl, 800 mM NaCl, 1 mM DTT, pH 8.0 to obtain a NaCl concentration of approximately 1 M) was further added thereto. After 2 hours, Benzamidine Sepharose 4 Fast Flow was removed by centrifugation, and the resultant was concentrated.

Finally, SEC (size exclusion chromatography) purification was performed using a gel filtration column HiLoad 16/600 Superdex 75 pg [GE Healthcare 28-9893-33], cOmplete His-Tag Purification Column 1 mL [Roche 06781543001], and a chromatography system.

### [Compound 7]

The supernatant of the bacterial cell disrupted solution was purified with HisTrap excel 5 mL [GE Healthcare 17-3712-06]. After adsorbing human KRasG12D mutant protein onto a column, thrombin was injected into the column. A HAT tag for purification added to the human KRasG12D mutant protein was cleaved with thrombin, and the human KRasG12D mutant protein alone was eluted. Finally, SEC (size exclusion chromatography) purification using HiLoad 26/600 Superdex 75 pg [GE Healthcare 28-9893-34] was performed.

It is noted, in all the compounds 1 to 7, that conditions of the buffer of the final SEC employed were 20 mM HEPES (4-(2-hydroxyethyl)-1-piperazineethanesulfonic acid), 100 mM NaCl, 1 mM DTT, 5 mM MgCl₂, pH 8.0.

### 2. Preparation of Complex

For the compounds 2 and 5, the human KRasG12D mutant protein was diluted to 1 mg/mL with KRAS buffer (20 mM HEPES, 100 mM NaCl, 5 mM MgCl₂, 1 mM DTT, pH 7.1). Each of the compounds 1 to 7 in a concentration of 10 mM dissolved in 100% DMSO (dimethyl sulfoxide) was diluted to 1 mM with KRAS buffer (20 mM HEPES (pH 7.1), 100 mM NaCl, 5 mM MgCl₂, 1 mM DTT, pH 7.1) for the compounds 1, 2, 3, 4, and 7, with KRAS buffer (20 mM HEPES, 100 mM NaCl, 5 mM MgCl₂, 1 mM DTT, pH 7.1) for the compound 5, and with pure water for the compound 6. The human KRasG12D mutant protein was mixed with each peptide compound so as to attain a molar ratio to the peptide compound of 3-fold or more as follows:
Compound 1: 8.03-fold
Compound 2: 5.73-fold
Compound 3: 5.73-fold
Compound 4: 5.68-fold
Compound 5: 3.34-fold
Compound 6: 5.69-fold
Compound 7: 5.73-fold

Thereafter, the compound 1 was allowed to stand on ice for 2 hours, the compounds 2, 3, 4 and 7 were allowed to stand on ice for 1 hour, the compound 5 was allowed to stand at 4°C for 1 hour, and the compound 6 was allowed to stand at room temperature for 1 hour, and thus, human KRasG12D mutant protein-peptide compound complexes were prepared.

### 3. Crystallization

A human KRasG12D mutant protein-peptide compound complex solution was centrifuged, and a supernatant was collected. The human KRasG12D mutant protein-peptide compound complex was concentrated until each compound attained an absorbance A₂₈₀ (Absorbance 280) at a wavelength of 280 nm of the following numerical value:
Compound 1: 11.2
Compound 2: 23.9
Compound 3: 17.6
Compound 4: 18.18
Compound 5: 8.352
Compound 6: 10
Compound 7: 17.6

The sample resulting from the concentration was used to perform screening for crystallization conditions using ATS-100 [EDC Biosystems] for the compounds 1, 2, 5, and 6, and with NT-8 [Formulatrix] for the compounds 3, 4, and 7. Crystals were obtained by a sitting drop vapor diffusion method under the following conditions:
Compound 1: 90 mM Morpheus Halogens, 0.1 M Morpheus buffer 1 (pH 6.5), 30.0%w/v P550MME_P20K, 20°C
Compound 2: 0.1 M Tris pH 8.5, 25.0%w/v polyethylene glycol 3,350, 20°C
Compound 3: 0.2 M MgCl₂, 0.1 M Tris pH 8.5, 20.0%w/v polyethylene glycol 8,000, 20°C
Compound 4: 0.2 M lithium sulfate monohydrate, 0.1 M 2,2-bis(hydroxymethyl)-2,2',2"-nitrilotriethanol [BIS-TRIS] (pH 6.5), 25.0 %w/v polyethylene glycol 3,350, 20°C
Compound 5: 0.2 M lithium sulfate monohydrate, 0.1 M Tris pH 8.5, 30.0%w/v polyethylene glycol 4,000, 20°C
Compound 6: 30.0 %w/v P550MME_P20K [550M_20K], 0.1 M Morpheus buffer 3 [MB3] (pH 8.5), 60 mM Morpheus Divalents [divalent], 21°
Compound 7: 0.2 M lithium sulfate monohydrate, 0.1 M Tris pH 8.5, 30.0%w/v polyethylene glycol 4,000, 20°C

### 4. Acquisition of X-ray Diffraction Data and Structural Analysis

X-ray diffraction data was acquired under the following conditions:
Compound 1:
   [Large synchrotron radiation facility]
      Swiss Light Source X10SA (Paul Scherrer Institute)
   [Sample treatment conditions]
      The crystal was frozen with liquid nitrogen in 90 mM Morpheus Halogens, 0.1 M Morpheus buffer 1 (pH 6.5), 30.0% w/v P550MME_P20K used as a cryoprotectant solution.
   [Measurement conditions]
      Temperature: 100 K
      X-ray wavelength: 1.96810 Å
Compound 2:
   [Large synchrotron radiation facility]
      SPring-8 BL45XU (National Research and Development Agency, Institute of Physical and Chemical Research, Harima Safety Center)
   [Sample treatment conditions]
      The crystal was frozen with liquid nitrogen in 0.1 M Tris pH 8.5, 25.0% w/v polyethylene glycol, 3,350, 25% ethylene glycol used as a cryoprotection solution.
   [Measurement conditions]
      Temperature: 100 K
      X-ray wavelength: 1.0 Å
Compound 3:
   [Large synchrotron radiation facility]
      SPring-8 BL45XU (National Research and Development Agency, Institute of Physical and Chemical Research, Harima Safety Center)
   [Sample treatment conditions]
      The crystal was frozen with liquid nitrogen in 0.2 M MgCl₂, 0.1 M Tris pH 8.5, 20.0%w/v polyethylene glycol 8,000, 25% ethylene glycol used as a cryoprotection solution.
   [Measurement conditions]
      Temperature: 100 K
      X-ray wavelength: 1.0 Å
Compound 4:
   [Large synchrotron radiation facility]
      SPring-8 BL45XU (National Research and Development Agency, Institute of Physical and Chemical Research, Harima Safety Center)
   [Sample treatment conditions]
      The crystal was frozen with liquid nitrogen in 0.2 M lithium sulfate monohydrate, 0.1 M 2,2-bis(hydroxymethyl)-2,2',2"-nitrilotriethanol [BIS-TRIS] (pH 6.5), 25.0 %w/v polyethylene glycol 3,350, 30% ethylene glycol used as a cryoprotection solution.
   [Measurement conditions]
      Temperature: 100 K
      **X-ray wavelength: 1.0 Å**
Compound 5:
   [Large synchrotron radiation facility]
      Photon Factory BL17A (Inter-University Research Institute Corporation, High Energy Accelerator Research Organization)
   [Sample treatment conditions]
      The crystal was frozen with liquid nitrogen in 0.2 M lithium sulfate monohydrate, 0.1 M Tris pH 8.5, 30.0% w/v polyethylene glycol 4,000, 25% glycerol used as a cryoprotection solution.
   [Measurement conditions]
      Temperature: 95 K
      **X-ray wavelength: 1.9 Å**
Compound 6:
   [Large synchrotron radiation facility]
      Photon Factory BL17A (Inter-University Research Institute Corporation, High Energy Accelerator Research Organization)
   [Sample treatment conditions]
      The crystal was frozen with liquid nitrogen in 30.0% w/v P550MME_P20K [550M_20K], 0.1 M Morpheus buffer 3 [MB3] (pH 8.5, 60 mM Morpheus Divalents [divalent] used as a cryoprotection solution.
   [Measurement conditions]
      Temperature: 95 K
      **X-ray wavelength: 0.98 Å**
Compound 7:
   [Large synchrotron radiation facility]
      SPring-8 BL45XU (National Research and Development Agency, Institute of Physical and Chemical Research, Harima Safety Center)
   [Sample treatment conditions]
      The crystal was frozen with liquid nitrogen in 0.2 M lithium sulfate monohydrate, 0.1 M 2,2-bis(hydroxymethyl)-2,2',2"-nitrilotriethanol [BIS-TRIS] (pH 6.5), 25.0% w/v polyethylene glycol 3,350, 30% ethylene glycol used as a cryoprotection solution.
   [Measurement conditions]
      Temperature: 100 K
      **X-ray wavelength: 1.0 Å**

Regarding the compounds 1 to 6, the X-ray diffraction data was analyzed with autoPROC (XDS (Kabsch, W., 2010), AIMLESS (Evans, P. R., 2006), STARANISO (Tickle, I. J. et al., 2019), CCP4 (Winn, M. D. et al., 2011)) (Global Phasing Ltd.) (Vonrhein, C. et al., 2001). Regarding the compound 7, the X-ray diffraction data were analyzed with XDS (Kabsch, W., 2010), CCP4 (Winn, M. D. et al., 2011). The crystal structures were determined using Phaser (McCoy, A. J. et al., 2007) by a molecular replacement method using the known structure of KRAS as a search model. The thus obtained structures were refined using Coot (Emsley, P. et al, 2010), Phenix (Adams, P. D. et al., 2010) and the like.

### 5. Determination of Crystal Structure

The crystal structures of the human KRasG12D mutant protein-peptide compound complexes were determined with the following degradative capacities:
Compound 1: 2.31 angstroms
Compound 2: 2.61 angstroms
Compound 3: 1.85 angstroms
Compound 4: 1.07 angstroms
Compound 5: 1.872 angstroms
Compound 6: 1.43 angstroms
Compound 7: 1.58 angstroms

Based on the thus determined structures, binding form of the peptide compounds 1 to 7 to the human KRasG12D mutant protein were revealed. Various statistical values obtained as a result of the structural analysis are shown in a table below. Besides, the entire structure of the complex of the compound 1 and the human KRasG12D mutant protein is illustrated in Figure 1, the entire structure of the complex of the compound 2 and the human KRasG12D mutant protein is illustrated in Figure 2, the entire structure of the complex of the compound 3 and the human KRasG12D mutant protein is illustrated in Figure 3, and the entire structure of the complex of the compound 4 and the human KRasG12D mutant protein is illustrated in Figure 4.

**[Table 7]**

| | | Compound 1 | Compound 2 | Compound 3 | Compound 4 |
|---|---|---|---|---|---|
| Space Group | | *P*3121 | *P*212121 | P3121 | *P*212121 |
| Unit Cell | a, b, c (Å) | 72.839 72.839 89.313 | 120.888 41.955 83.119 | 66.579 66.579 90.083 | 45.770 47.182 95.385 |
| | α, β, γ(°) | 90.00 90.00 120.00 | 90.00 90.00 90.00 | 90.00 90.00 120.00 | 90.00 90.00 90.00 |
| Degradative Capacity (Å) | | 63.080 - 2.317 (2.603 - 2.317)∗ | 83.118 - 2.618 (2.920 - 2.618)∗ | 57.659 - 1.856 (2.143 - 1.856)∗ | 47.693 - 1.066 (1.156 - 1.066)∗ |
| *R*_{merge} | | 0.904 (4.258)∗ | 0.461 (3.798)∗ | 0.228 (1.906)∗ | 0.094 (1.425)∗ |
| *CC* (1/2) | | 0.986 (0.599)∗ | 0.984 (0.385)∗ | 0.997 (0.682)∗ | 0.999 (0.577)∗ |
| mean *I*/σ (*I*) | | 7.6 (1.5)∗ | 6.3 (1.3)∗ | 9.8 (1.8)∗ | 12.5 (1.5)∗ |
| Completeness, sphere (%) | | 67.9 (11.7)∗ | 57.1 (10.5)∗ | 52.4 (7.6)∗ | 72.2 (16.9)∗ |
| Completeness, ellipsoid (%) | | 91.8 (57.6)∗ | 90.8 (54.3)∗ | 93.2 (66.2)∗ | 91.2 (57.8)∗ |
| Total Reflection | | 313867 (12030)∗ | 94262 (4415)∗ | 198440 (8814)∗ | 584708 (31190)∗ |
| Independent Reflection | | 8416 (422)∗ | 7632 (383)∗ | 10504 (527)∗ | 67124 (3357)∗ |
| Redundancy | | 37.3 (28.5)∗ | 12.4 (11.5)∗ | 18.9 (16.7)∗ | 8.7 (9.3)∗ |
| Refinement | Degradative Capacity (Å) | 63.08 - 2.31 | 68.49 - 2.61 | 57.66 - 1.85 | 47.69 - 1.07 |
| | Reflection | 8391 | 7615 | 10503 | 67122 |
| | *R*_{work}/*R*_{free}^{a} | 0.2019 / 0.3685 | 0.2187 / 0.3364 | 0.1938 / 0. 2360 | 0.1860 / 0.1957 |
| A value in parentheses was obtained in outermost shell. | | | | | |
| R_{free} was calculated using 5% of randomly selected reflections. | | | | | |

| | | Compound 5 | Compound 6 | Compound 7 | |
|---|---|---|---|---|---|
| Space Group | | *P*212121 | P1211 | P31 21 | |
| Unit Cell | **a,** b, c (A) | 41.2185 77.9646 124.4455 | 45.768. 41. 168. 56.732 | 65.619 65. 619 81. J66 | |
| | α, β, γ (°) | 90.00 90.00 90.00 | 90.000, 101.434. 90.000 | 90. 00 90.00 120. 00 | |
| Degradative Capacity (Å) | | 124.446 - 1.886 (2.058 - 1.886)∗ | 44.860 - 1.238 (1.34 - 1.238)∗ | 47.734 - 1.584 | |
| *R*_{merge} | | 0.270 (1.739)∗ | 0.081 (1.207)∗ | - | |
| *CC* (1/2) | | 0.995 (0.173)∗ | 0.999 (0.433)∗ | 0.999 | |
| mean *I*/*σ (I)* | | 10.1 (1.2)∗ | 13.3 (1.3)∗ | 10.45 | |
| Completeness, sphere (%) | | 64.9 (14.3)∗ | 77.8 (18.5)∗ | 99. 9 | |
| Completeness, ellipsoid (%) | | 91.7 (46.9)∗ | 92.3 (47.5)∗ | - | |
| Total Reflection | | 472612 (8788)∗ | 298789 (11410)∗ | 601933 | |
| Independent Reflection | | 21566 (1078)∗ | 46057 (2305)∗ | 57609 | |
| Redundancy | | 21.9 (8.2)∗ | 6.5 (5.0)∗ | 10.4 | |
| Refinement | Degradative Capacity (A) | 66.188 - 1.872 | 55.61 - 1.43 | 47.73 - 1.58 | |
| | Reflection | 20218 | 36732 | 3025d | |
| | *R*_{work}/*R*_{free}^{a} | 0.2190 / 0.2625 | 0.16931 / 0.19554 | 0.2073 / 0.2362 | |
| A value in parentheses was obtained in outermost shell. | | | | | |
| R_{free} was calculated using 5% of randomly selected reflections. | | | | | |

### Evaluation Example 2 Analysis of Interaction between KRASG12D and Peptide Compound

### 1. Definition of Amino Acids Interacting with Peptide Compound

Amino acids contained in the mutant RAS interacting with a peptide compound were identified by using Display receptor-ligand interactions function of Discovery studio 2020 Client.

**[Table 8]**

| Compound No. | Amino Acid Residues of Mutant RAS Interacting with Peptide Compound | |
|---|---|---|
| | Switch 2 Region (ALA59-GLU76) | α3 Helix Region (THR87-LYS104) |
| 1 | GLN61, GLU62, ARG68, MET72 | HIS95, TYR96. GLN99, ARG102, VAL103 |
| 2 | GLN61, ARG68, MET72 | HtS95. TYR96, GLN99, VAL103 |
| 3 | GLN61, GLU62, ARG68, ASP69, MET72 | HIS95, TYR96, GLN99, ARG102. VAL103 |
| 4 | GLN61, GLU62, ARG68, ASP69, MET72, ARG73 | HIS95, TYR96, GLN99, ARG102, VAL103 |
| 5 | GLN61, ARG68, ASP69, MET72, ARG73 | TYR96, GLN99, AR0102, VAL103 |
| 6 | ARG68. TYR71 | TYR96. GLN99. ARG102, VAL103 |
| 7 | TYR64, ARG68, MET72 | ASP92, HIS95, TYR96. GLN99, ARG102, VAL103 |

### 2. Calculation of Interatomic Distance between Peptide Compound and ASP 12

Discovery studio 2020 Client was used to specify an atom (direct interaction atom) excluding a hydrogen atom located within a distance of 4.50 angstroms or less from either of two oxygen atoms of a carboxy group present in ASP12. In the compounds 1 to 3, iodine substituted in the 3-position of a phenyl group present in amino acid residues derived from Fmoc-Phe(3-I)-OH corresponds to, in the compound 4, carbon on hydrogen side of end alkyne of an acetylene group substituted in the 3-position of a phenyl group present in amino acid residues derived from Fmoc-Phe(3-C#C)-OH corresponds to, and in the compound 7, nitrogen located in the 4-position of a piperazine group corresponds to the direct interaction atom. Next, with each of these direct interaction atoms and one of the two oxygen atoms of the carboxy group present in ASP12 of KRASG12D were specified to measure an interatomic distance therebetween. Next, similar measurement was performed on the other oxygen atom, and a shorter distance was defined as the interatomic distance between the peptide compound and ASP12. It is noted that the direct interaction atom was not found in the compounds 5 and 6, and hence the interatomic distance was not measured.

Results are shown in the following table:

**[Table 9]**

| Compound No. | **Interatomic Distance (Å)** |
|---|---|
| 1 | 3.59 |
| 2 | 3.58 |
| 3 | 3.70 |
| 4 | 3.49 |
| 5 | - |
| 6 | - |
| 7 | 2.89 |

It is noted that the direct interaction atom and the oxygen atom (at a shorter distance from the direct interaction atom) of the carboxy group present in ASP12 of KRASG12D are interacting with each other not via another molecule in all of the compounds 1 to 4 and 7.

It is understood, from the results shown in Table 9, that the direct interaction atom and the two oxygen atoms (at a shorter distance from the direct interaction atom) of the carboxy group present in ASP12 of KRASG12D are directly interacting with each other in all of the compounds 1 to 4.

Besides, in the compound 7, although the direct interaction with the two oxygen atoms (at a shorter distance from the direct interaction atom) of the carboxy group present in ASP12 was found, none of strong interaction with GLN61 in the Switch 2 region, a phenomenon of distance reduction between the Switch 2 region and the α3 helix region, and strong interaction between an atom of an amino acid residue present in the Switch 2 region and an atom of ASP12 described below were found.

### 3-1. Calculation of Interaction Energy between Peptide Compound and GLN61

Calculation was performed using Calculate Interaction Energy function of Discovery studio 2020 Client.

Before the calculation, Apply Force Field function was used to allocate a force field in the entire system. As the force field, CHARMm corresponding to the default was used.

Calculate Interaction Energy panel was opened, a peptide compound was set as Atom Selection 1, and the mutant RAS was specified as Residue Selection 2. Implicit Distance-Dependent Dielectrics was specified as a Dielectric Model parameter, and a Dielectric Constant parameter was set to 1. A Nonbond List Radius parameter was set to 14.

A value of the Interaction Energy item between the peptide compound and GLN61 obtained as a calculation result was used to obtain a sum of interaction energies between all the atoms contained in the peptide compound and all the atoms contained in GLN61.

### 3-2. Calculation of Interaction Energy between Peptide Compound and GLN61 after Optimization of Hydrogen Atom

Minimization function of Discovery studio 2020 Client was used to optimize hydrogen atoms through the following procedures.

First, heavy atoms of the entire system were selected, and Create Fixed Atom Constraint function was used to apply conformational constraints to the heavy atoms. Thereafter, Apply Force Field function was used to allocate the force field. As the force field, CHARMm was used.

Then, Minimization function was used to optimize hydrogen atoms. At this point, Distance-Dependent Dielectrics was specified as Implicit Solvent Model.

After the optimization of hydrogen atoms, a sum of interaction energies between all the atoms contained in the peptide compound and all the atoms contained in GLN61 was obtained in the same manner as in 3-1.

Calculation results are shown in the following table:

**[Table 10]**

| Compound No. | Interaction Energy (kcal/mol) | Interaction Energy (kcal/mol) after Hydrogen Optimization |
|---|---|---|
| 1 | -16.6 | -16. 7 |
| 2 | -11.4 | -16.6 |
| 3 | -18.4 | -19.1 |
| 4 | -20.9 | -20.24 |
| 5 | -9.5 | -9.8 |
| 6 | -1.8 | -1.8 |
| 7 | -2.5 | -2.6 |

It is understood, from the results shown in Table 10, that all the compounds 1 to 4 is interacting strongly with GLN61 of the Switch 2 region. On the other hand, the interaction with GLN61 of the Switch 2 region was weaker in either case in the compounds 6 and 7 than in the compounds 1 to 4. The interaction with GLN61 of the Switch 2 region was stronger in the compound 5 than in the compounds 6 and 7, but was weaker than in the compounds 1 to 4, and no direct interaction atom was found therein as described above.

### 4. Calculation of Distance between Switch 2 and α3 Helix

Distance Monitor function of Discovery studio 2020 Client commercially available from Dassault Systèmes was used to calculate an interatomic distance between Cα present ASP69 and Cα present GLN99 of the mutant RAS.

Calculation results are shown in the following table:

**[Table 11]**

| Compound No. | Interatomic Distance between Cα of ASP69 and Cα of GLN99 (Å) |
|---|---|
| 1 | 12.35 |
| 2 | 11.77 |
| 3 | 12.21 |
| 4 | 12.48 |
| 5 | 12.67 |
| 6 | 14.63 |
| 7 | 14.78 |

It is understood, from the results shown in Table 11, that when the compounds 1 to 4 were bound to KRAS (G12D), the distance between the Switch 2 region and the α3 helix region was smaller than when the compounds 6 and 7 were bound to KRAS (G12D).

Besides, the distance between the Switch 2 region and the α3 helix region was found to be small in the compound 5 equivalently to that in the compounds 1 to 4, but no direct interaction atom was found therein as described above.

### 5-1. Calculation of Interaction Energy of Pre-orientation (target: main chain atom)

A sum of interaction energies between all atoms contained in a main chain of an amino acid residue, out of the ALA59, GLY60, GLN61 and GLU62, having one or more non-hydrogen atoms located within a distance of 4.5 angstroms or less from either of two oxygen atoms of a carboxy group of a side chain of the ASP12, and all atoms of ASP12 was calculated.

It is noted that the amino acid residue having one or more non-hydrogen atoms located within a distance of 4.5 angstroms or less from either of two oxygen atoms of a carboxy group of a side chain of ASP12 was ALA59, GLY60, and GLN61 in the compounds 1 to 4, and was GLN61 in the compound 5. In the compounds 6 and 7, no amino acid residues satisfied the above-described conditions, and hence the interaction energy was not calculated.

The calculation was performed using Calculate Interaction Energy function of Discovery studio 2020 Client.

Before the calculation, the force field was allocated in the entire system using Apply Force Field function. As the force field, CHARMm was used. Calculate Interaction Energy panel was opened, and all the atoms present in ASP12 of the mutant RAS were specified as Atom Selection 1. As Atom Selection 2, all the main chain atoms of amino acids, out of the amino acids of ALA59, GLY60, GLN61, and GLU62 of the mutant RAS, located within a distance of 4.5 angstroms or less from the two O atoms of a carboxy group of ASP12 were specified. As Dielectric Model parameter, Implicit Distance-Dependent Dielectrics was specified, and a Dielectric Constant parameter was set to 1. A Nonbond List Radius parameter was set to 14. Values in Interaction Energy item of calculation results were used.

### 5-2. Calculation of Interaction Energy of Pre-orientation after Hydrogen Atom Optimization (target: main chain atom)

Minimization function of Discovery studio 2020 Client was used to optimize hydrogen atoms through the following procedures. First, heavy atoms of the entire system were selected, and Create Fixed Atom Constraint function was used to apply conformational constraints to the heavy atoms. Thereafter, Apply Force Field function was used to allocate the force field. As the force field, CHARMm was used.

Then, Minimization function was used to optimize hydrogen atoms. At this point, Distance-Dependent Dielectrics was specified as Implicit Solvent Model.

After the optimization of hydrogen atoms, interaction energy of pre-orientation was calculated in the same manner as in 5-1.

### 5-3. Calculation of Interaction Energy of Pre-orientation (target: all atoms)

Interaction energy of pre-orientation was calculated in the same manner as in 5-1 except that the calculation was performed on all the atoms of amino acid residues, out of the amino acids of ALA59, GLY60, GLN61, and GLU62, located within a distance of 4.5 angstroms or less from either of the two O atoms of a carboxy group on a side chain of ASP12.

### 5-4. Calculation of Interaction Energy of Pre-orientation after Hydrogen Atom Optimization (target: all atoms)

Interaction energy of pre-orientation was calculated in the same manner as in 5-2 except that the calculation was performed on all the atoms of amino acid residues, out of the amino acids of ALA59, GLY60, GLN61, and GLU62, located within a distance of 4.5 angstroms or less from either of the two O atoms of a carboxy group on a side chain of ASP12.

Calculation results are shown in the following table:

**[Table 12]**

| Compound No. | Interaction Energy (kcal/mol) (main chain atoms) | Interaction Energy (kcal/mol) after Hydrogen Optimization (main chain atoms) | Interaction Energy (kcal/mol) (all atoms) | Interaction Energy (kcal/mol) after Hydrogen Optimization (all atoms) |
|---|---|---|---|---|
| 1 | -16.7 | -16.6 | -17.6 | -17.5 |
| 2 | -17.5 | -17.2 | -17.1 | -16.8 |
| 3 | -16.8 | -17.3 | -16.5 | -16.9 |
| 4 | -17.7 | -17.4 | -17.4 | -17.1 |
| 5 | -1.8 | -1.7 | -14.2 | -13.6 |
| 6 | - | - | - | - |
| 7 | - | - | - | - |

It is noted that atoms of amino acid residues present in the Switch 2 region and atoms of ASP 12 are interacting with each other not via another molecule in all of the compounds 1 to 4 and 5.

It is understood, from the results shown in Table 12, that atoms of amino acid residues present in the Switch 2 region of KRAS-G12D and atoms of ASP12 are strongly interacting with each other in all of the compounds 1 to 4.

Also in the compound 5, interaction between atoms of amino acid residues present in the Switch 2 region of KARS-G12D and atoms of ASP 12 was found, but the interaction was not as strong as that found in the compounds 1 to 4. Besides, no direct interaction atoms were found as described above.

In the compound 6, pre-orientation was not found, and only interaction weaker than that in the compound 5 was found in the compound 7.

### Evaluation Example 3 Evaluation of Bond of Compound to KRAS by Surface Plasmon Resonance (SPR)

Biacore 8K+ (GE Healthcare) was used as an apparatus, and HBS (10 mM HEPES-NaOH, 150 mM NaCl, pH 7.4) containing 1 mM DTT, 10 mM MgCl₂, 0.01% Tween 20, 10 µM GDP, and 4% DMSO was used as a running buffer. Each compound solution was prepared, with the running buffer used as a solvent, as dilution series of 4 concentrations in a common ratio of 3 with a concentration shown in Table 13 used as the maximum concentration. In creating the dilution series, a dilution series of a compound solution 100 times thicker than the final concentration was first prepared using DMSO as a solvent, and then the solution was diluted 100 times with a dilution buffer (HBS containing 1 mM DTT, 10 mM MgCl₂, 0.01% Tween 20, 10 µM GDP, 3.03% DMSO) for the preparation.

Each of biotinylated Avi-tag fused wild type KRAS(WT) and the mutant KRAS (G12D) was immobilized in an immobilization amount of about 150 to 300 RU on a surface of Sensor Chip CAP (Cytiva) coated with Biotin CAPture Reagent. Binding of each compound was evaluated by Single Cycle Kinetics method, and a running buffer or a compound solution was added onto a no-KRAS immobilized surface and immobilized surface to obtain binding response. A flow rate was set to 100 µL/min, and in a compound addition cycle, compound solutions having different 4 concentrations were added intermittently from the lower concentration for 75 seconds each. A dissociation phase was observed for 3,500 seconds. In a blank cycle, the running buffer was intermittently added 4 times instead of the compound solution. Biotin CAPture Reagent and KRAS were immobilized in each cycle, and a sensor chip was reproduced with Regeneration solution attached to Biotin CAPture Kit (Cytiva) after completing each cycle. The measurement was performed at 30°C.

**[Table 13]**

| | KRAS (G1 2D)-GDP | KRAS (WT)-GDP |
|---|---|---|
| Compound No. | Top dose ( *µ*M) | Top dose ( *µ*M) |
| 1 | 1 | 1 |
| 2 | 5 | 5 |
| 3 | 0.5 | 5 |
| 4 | 0.5 | 0.5 |
| 5 | 0.5 | 1 |
| 6 | 1 | 1 |
| 7 | 0.1 | 0.5 |

The thus obtained sensorgram was subjected to Double Reference treatment and solvent correction with Biacore Insight Evaluation Software, and then was subjected to curve fitting based on 1:1 binding model, and thus, dissociation constants K_{D} for KRAS of the compounds 1 to 7 prepared in Example 1 were determined.

The following table shows selectivity for the mutant KRAS (G12D) over wild type KRAS calculated based on the dissociation constants K_{D} of the respective peptide compounds.

**[Table 14]**

| | | Dissociation Constant K_{D} (nM) for Mutant KRAS (G12D) | Dissociation Constant K_{D} (nM) for Wild Type KRAS | Selectivity (times) for Mutant KRAS (G12D) over Wild Type KRAS |
|---|---|---|---|---|
| | Example 1 | 6. 6 | 91 | 14 |
| | Example 2 | 4.4 | 44 | 10 |
| | Example 3 | 2.9 | 43 | 15 |
| | Example 4 | 1.5 | 23 | 15 |
| Comparative Example 1 | | 3.6 | 19 | 5.3 |
| Comparative Example 2 | | 21 | 16 | 0.76 |
| Comparative Example 3 | | 0.18 | 0.94 | 5.2 |

It is understood, from the results shown in Table 14, that all of the compounds 1 to 4 exhibited higher selectivity for KRAS (G12D) as compared with the compounds 5 to 7.

The reason why such high selectivity for KRAS (G12D) is exhibited is not exactly cleared, but the following can be a tentative theory.

First, the compounds of Examples 1 to 4 of the present disclosure are directly interacting with ASP12 of KRAS (G12D) (Table 9). Besides, the compounds of Examples 1 to 4 interact with the Switch 2 region and the α3 helix of KRAS (G12D) (Table 8), and hence the distance between the Switch 2 region and the α3 helix region is smaller (Table 11). This is probably a cause of the pre-orientation (Table 12) corresponding to the interaction between the Switch 2 region and ASP12 of KRAS (G12D).

In consideration of all of these, it is presumed that the compounds of Examples 1 to 4 directly interact with ASP12 of KRAS (G12D) as well as interact with the Switch 2 region and the α3 helix region of KRAS (G12D), and hence causes a change in the intramolecular structure more dominantly stabilized in KRAS (G12D), resulting in exhibiting high selectivity for KRAS (G12D).

Besides, in some compounds, the selectivity for KRAS (G12D) is higher than a sum of selectivities of the compound 5 in which no direct interaction could be found, and the compound 7 in which pre-orientation was not found. This suggests that the direct interaction with ASP12 of KRAS (G12D) described above (direct interaction) and the action of causing the change in the intramolecular structure more dominantly stabilized in KRAS (G12D) (pre-orientation) together exhibit synergistic effect to contribute to high selectivity for KRAS (G12D).

## Claims

1. A binding molecule having the following features (1) and (2), and having binding selectivity for mutant RAS (G12D) 5 times or more over wild type RAS:
(1) the binding molecule interacts with Asp12 of the mutant RAS (G12D); and
(2) the binding molecule interacts with a Switch 2 region of the mutant RAS (G12D), and an amino acid residue present in the Switch 2 region intermolecularly interacts with Asp12 in the mutant RAS (G12D).

2. The binding molecule according to claim 1, wherein the binding molecule further interacts with an α3 helix region of the mutant RAS (G12D).

3. The binding molecule according to claim 1 or 2, wherein when the amino acid residue present in the Switch 2 region is intermolecularly interacting with Asp12, a distance between an α carbon atom of Asp69 present in the Switch 2 region and an α carbon atom of Gln99 present in the α3 helix region is 15.0 angstroms (Å) or less in the mutant RAS (G12D).

4. The binding molecule according to any one of claims 1 to 3, wherein the binding selectivity is 10 times or more.

5. The binding molecule according to any one of claims 1 to 4, wherein a dissociation constant (K_{D}) for the mutant RAS (G12D) is 10 nM or less.

6. The binding molecule according to any one of claims 1 to 5, wherein the amino acid residue that is present in the Switch 2 region and interacts with the Asp12 is any one or more selected from the group consisting of Ala59, Gly60, Gln61, and Glu62.

7. The binding molecule according to claim 6, wherein a sum of interaction energies between all atoms contained in a main chain of an amino acid residue, out of the Ala59, Gly60, Gln61, and Glu62, having one or more non-hydrogen atoms present within a distance of 4.5 angstroms (Å) or less from either of two oxygen atoms of a carboxy group of a side chain of the Asp12, and all atoms of Asp12 is -0.5 kcal/mol or less.

8. The binding molecule according to claim 6, wherein a sum of interaction energies between all atoms contained in an amino acid residue, out of the Ala59, Gly60, Gln61, and Glu62, having one or more non-hydrogen atoms present within a distance of 4.5 angstroms (Å) or less from either of two oxygen atoms of a carboxy group of a side chain of the Asp12, and all atoms of Asp12 is -0.5 kcal/mol or less.

9. The binding molecule according to any one of claims 1 to 8, wherein the binding molecule interacts with Gln61 of the Switch 2 region.

10. The binding molecule according to any one of claims 1 to 9, comprising an amino acid residue that interacts with Gln61, wherein a sum of interaction energies between all atoms contained in the amino acid residue that interacts with the Gln61 and all atoms of the Gln61 is - 7.0 kcal/mol or less.

11. The binding molecule according to any one of claims 1 to 10, comprising a structure (1) of *-(linear or branched C₁-C₈ alkylene)-X, or *-(linear or branched C₁-C₈ alkylene)-(C₆-C₁₀ arylene)-X, wherein X is hydroxy, Cl, Br, I, -NR_{A}R_{B} (wherein R_{A} and R_{B} are both hydrogen, or each independently C₁-C₃ alkyl, or R_{A} and R_{B} form, together with nitrogen atoms binding thereto, a 4- to 8-membered heterocycle containing 1 to 3 hetero atoms selected from the group consisting of N, O, and S), C₁-C₃ alkyl, C₁-C₃ haloalkyl, -C---CH, or optionally substituted C₃-C₈ cycloalkyl, * means a binding point, and if the binding molecule comprises *-(linear or branched C₁-C₈ alkylene)-X, X is not C₁-C₃ alkyl.

12. The binding molecule according to any one of claims 1 to 11, comprising a structure (2) of *-(linear or branched C₁-C₈ alkylene)-Y, wherein Y is optionally substituted C₁-C₁₀ alkyl, optionally substituted C₂-C₁₀ alkenyl, optionally substituted C₂-C₁₀ alkynyl, optionally substituted C₁-C₆ alkoxy C₁-C₆ alkyl, optionally substituted C₆-C₁₀ aryl, or optionally substituted C₃-C₈ cycloalkyl, and * means a binding point.

13. The binding molecule according to any one of claims 1 to 12, comprising the following structure (3): 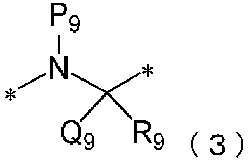
wherein R₉ is C₁-C₆ alkyl, optionally substituted C₁-C₆ alkoxy C₁-C₆ alkyl, or optionally substituted C₆-C₁₀ aryl, or
R₉ forms a 4- to 7-membered saturated heterocycle together with carbon atoms to which P₉ and R₉ are bound, and a nitrogen atom to which P₉ is bound,
P₉ is hydrogen or C₁-C₃ alkyl unless R₉ and P₉ form a 4- to 7-membered saturated heterocycle,
Q₉ is hydrogen or C₁-C₆ alkyl, and
* means a binding point.

14. The binding molecule according to claim 13, wherein an atom farthest from the binding point of the structure (1) among atoms contained in X of the structure (1), and an atom farthest from the binding point of the structure (2) among atoms contained in Y of the structure (2) are bound to each other via 20 or less atoms.

15. The binding molecule according to claim 13, wherein an atom farthest from the binding point of the structure (1) among atoms contained in X of the structure (1), and an atom farthest from a carbon atom binding to Q₉ and R₉ in the structure (3) among atoms contained in R₉ of the structure (3) are bound to each other via 25 or less atoms unless R₉ and P₉ form a 4- to 7-membered saturated heterocycle.

16. The binding molecule according to any one of claims 1 to 15, wherein a molecular weight of the binding molecule is 5,000 or less.
